(19) Europäisches Patentamt
European Patent Office
Office européen des brevets

(11) EP 4 144 820 A1

(12) **EUROPEAN PATENT APPLICATION**
published in accordance with Art. 153(4) EPC

(43) Date of publication:
**08.03.2023 Bulletin 2023/10**

(21) Application number: **21797628.1**

(22) Date of filing: **13.04.2021**

(51) International Patent Classification (IPC):
***C09K 19/12*** (2006.01) ***C09K 19/14*** (2006.01)
***C09K 19/16*** (2006.01) ***C09K 19/18*** (2006.01)
***C09K 19/20*** (2006.01) ***C09K 19/22*** (2006.01)
***C09K 19/24*** (2006.01) ***C09K 19/28*** (2006.01)
***C09K 19/30*** (2006.01) ***C09K 19/32*** (2006.01)
***C09K 19/34*** (2006.01) ***C09K 19/38*** (2006.01)
***C09K 19/42*** (2006.01) ***C09K 19/54*** (2006.01)
***G02B 5/26*** (2006.01) ***G02B 5/30*** (2006.01)
***C07C 69/92*** (2006.01)

(52) Cooperative Patent Classification (CPC):
**C07C 69/92; C09K 19/12; C09K 19/14; C09K 19/16; C09K 19/18; C09K 19/20; C09K 19/22; C09K 19/24; C09K 19/28; C09K 19/30; C09K 19/32; C09K 19/34; C09K 19/38; C09K 19/42; C09K 19/54;** (Cont.)

(86) International application number:
**PCT/JP2021/015240**

(87) International publication number:
**WO 2021/220794 (04.11.2021 Gazette 2021/44)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.04.2020 JP 2020079609**
**17.03.2021 JP 2021043343**

(71) Applicant: **FUJIFILM Corporation**
**Tokyo 106-8620 (JP)**

(72) Inventors:
- **KATOH, Shunya**
  **Minamiashigara-shi, Kanagawa 250-0193 (JP)**
- **YAMAMOTO, Aiko**
  **Minamiashigara-shi, Kanagawa 250-0193 (JP)**

(74) Representative: **Klunker IP**
**Patentanwälte PartG mbB**
**Destouchesstraße 68**
**80796 München (DE)**

(54) **COMPOUND, LIQUID CRYSTAL COMPOSITION, CURED PRODUCT AND FILM**

(57) A compound includes, in a molecule, a structure A represented by the following A and a structure B represented by the following B, in which the compound includes two or more of the structures B in the molecule. A: A structure in which at least one or more aromatic ring structures are included and a total number of $\pi$ electrons of the aromatic ring structure in the molecule is 8 or more. However, an aryl amine structure represented by a predetermined structure is excluded. B: A fluorine-containing terminal structure represented by a predetermined structural formula.

EP 4 144 820 A1

(52) Cooperative Patent Classification (CPC): (Cont.)
**G02B 5/26; G02B 5/30**

## Description

## BACKGROUND OF THE INVENTION

### 1. Field of the Invention

The present invention relates to a compound (particularly, a liquid crystal alignment accelerator) useful for various use applications, including materials of various optical members such as an optically anisotropic film, a visible light reflecting film, and a heat shield film, a liquid crystal composition and a cured substance containing the compound, and a film using these.

### 2. Description of the Related Art

The liquid crystal is regularly aligned in a case of being applied onto a film (an alignment film) that has been subjected to alignment treatment. In addition, the alignment state of the liquid crystal can be controlled by sandwiching the liquid crystal between the two alignment films. Accordingly, in a liquid crystal display device consisting of a liquid crystal cell consisting of a rod-like liquid crystalline molecule and two substrates for encapsulating the rod-like liquid crystalline molecule, and an electrode layer for applying a voltage to the rod-like liquid crystalline molecule, the rod-like liquid crystalline molecule is in a state of being injected into the gap between the alignment films respectively formed on the two substrates, and thus the alignment state of the rod-like liquid crystalline molecule can be controlled relatively easily.

On the other hand, for the intended purpose of expanding the viewing angle of the liquid crystal display device or eliminating coloration, an optical compensation sheet (a phase difference plate) is disposed between the liquid crystal cell and the polarizing plate. In this case, an optically anisotropic element having an optically anisotropic layer formed from the liquid crystalline molecule on a transparent support is used as the optical compensation sheet. The optically anisotropic layer is formed by aligning liquid crystalline molecules and subsequently fixing the alignment state thereof. At this time, the liquid crystalline molecules are aligned by a single alignment film provided between the transparent support and the optically anisotropic layer. However, liquid crystalline molecules are difficult to be subjected to uniform alignment (monodomain alignment) from the alignment film interface to the air interface in the single alignment film. This is because there is no alignment restricting force on the interface (air interface) side where the alignment treatment is not applied, and thus the alignment of the liquid crystal is disturbed. In a case where the liquid crystalline molecules are not uniformly aligned, light scattering due to disclination occurs, and an opaque film is formed. Such a film is not preferable from the viewpoint of improving visibility of the liquid crystal display device.

In consideration of such a necessity, development has been made for a technique in which on the interface (air interface) side where the alignment treatment is not carried out, an alignment restricting force is applied to the liquid crystal even without an alignment film, thereby uniformly aligning the liquid crystal (for example, see JP2002-129162A and JP2013-47204A). Here, the alignment of the liquid crystalline molecules is controlled by adding a liquid crystal alignment accelerator. Further, by using a liquid crystal alignment accelerator, this technique provides a liquid crystal composition with which liquid crystalline molecules are easily and uniformly aligned.

## SUMMARY OF THE INVENTION

The inventors of the present invention revealed that the usable concentration range and the solubility of the liquid crystal alignment accelerator described in JP2002-129162A are not always sufficient, and there is still room for improvement.

In addition, the inventors of the present invention revealed that the liquid crystal alignment accelerator described in JP2013-47204A may also include perfluorooctanoic acid (PFOA), which has been pointed out to be harmful to the environment, and an analog substance thereof, and thus there is room for improvement.

Accordingly, an object of the present invention is to provide a compound that solves the above problem in the related art, exhibits sufficient solubility, has a wide usable concentration range, has environmental suitability, and exhibits an excellent liquid crystal alignment promoting property.

In addition, an object of the present invention is also to provide a liquid crystal composition with which liquid crystalline molecules can be easily and uniformly aligned by the compound, and a cured substance thereof.

Further, another object of the present invention is also to provide a film (for example, an optically anisotropic film, a visible light reflecting film, or a heat shield film) using the liquid crystal composition or the cured substance.

As a result of diligent research to achieve the above-described objects, the inventors of the present invention found that a compound having a predetermined ring structure and a predetermined fluorine-containing terminal structure in the molecule and having two or more predetermined fluorine-containing terminal structures in the molecule exhibits

sufficient solubility, has a wide usable concentration range, has environmental suitability, and exhibits an excellent liquid crystal alignment promoting property, thereby completing the present invention.

That is, the inventors of the present invention have found that the above objects can be achieved by the following configurations.

[1] A compound comprising, in a molecule:

a structure A represented by the following A; and
a structure B represented by the following B,
in which the compound includes two or more of the structures B in the molecule,

A: a structure in which at least one or more aromatic ring structures are included and a total number of $\pi$ electrons of the aromatic ring structure in the molecule is 8 or more, provided that aryl amine structures represented by Formulae (I) to (IV) described later are excluded,
B: a fluorine-containing terminal structure represented by any one of Formulae (B-1), ..., or (B-5) described later.

[2] The compound according to [1], in which the structure A is a structure represented by Formula (1) or (2) described later.
[3] The compound according to [1] or [2], in which the structure A is a structure represented by any one of Formulae (1-1) to (1-10) described later.
[4] The compound according to [3], in which in Formulae (1-1) to (1-10) described later, $L^1$, $L^2$, $L^3$, and $L^4$ are each independently a single bond or a divalent linking group of any one of -C(=O)O-, -OC(=O)-, -O-, -NH-C(=O)-, or -NH-.
[5] The compound according to [1] or [2], in which the structure A is a structure represented by Formula (2-1) or (2-2) described later.
[6] The compound according to any one of [1] to [5], in which the compound is used in a liquid crystal alignment accelerator.
[7] A liquid crystal composition comprising:

the compound according to any one of [1] to [6]; and
a polymerizable liquid crystal compound.

[8] The liquid crystal composition according to [7], in which the polymerizable liquid crystal compound is a rod-like liquid crystal compound or a disk-like liquid crystal compound.
[9] The liquid crystal composition according to [7] or [8], further comprising at least one chiral compound.
[10] A cured substance obtained by polymerizing the liquid crystal composition according to any one of [7] to [9].
[11] A film comprising at least one kind of the cured substance according to [10].
[12] A film obtained by immobilizing a cholesteric liquid crystalline phase of the liquid crystal composition according to any one of [7] to [9].
[13] The film according to [11] or [12], in which the film exhibits optical anisotropy.
[14] The film according to any one of [11] to [13], in which the film exhibits selective reflection characteristics.
[15] The film according to [14], in which the film exhibits selective reflection characteristics in an infrared wavelength range.
[16] The film according to [14], in which the film exhibits selective reflection characteristics in a visible light wavelength range.

According to the present invention, it is possible to provide a compound that exhibits sufficient solubility, a wide usable concentration range, has environmental suitability, and exhibits an excellent liquid crystal alignment promoting property.

In addition, according to the present invention, it is also possible to provide a liquid crystal composition with which liquid crystalline molecules can be easily and uniformly aligned by the compound, and a cured substance thereof.

Further, according to the present invention, it is also possible to provide a film (for example, an optically anisotropic film, a visible light reflecting film, or a heat shield film) using the liquid crystal composition or the cured substance.

## DESCRIPTION OF THE PREFERRED EMBODIMENTS

Hereinafter, the present invention will be described in detail.

The following description of constituent requirements is based on representative embodiments of the invention

and specific examples; however, the present invention is not limited to such embodiments and specific examples.

A numerical value range represented using "to" in the present specification means a range including the numerical values described before and after "to" as the lower limit and the upper limit respectively.

Further, in the present specification, as each component, a substance corresponding to each component may be used alone, or two or more kinds of substances may be used in a combination. Here, in a case where two or more kinds of substances are used in combination for each component, the content of the component refers to the total content of the substances used that is used in combination unless otherwise particularly specified.

Further, in the present specification, "(meth)acrylate" is a notation representing "acrylate" or "methacrylate", "(meth)acryl" is a notation representing "acryl" or "methacryl", and "(meth)acryloyl" is a notation representing "acryloyl" or "methacryloyl".

[Compound]

The compound according to the embodiment of the present invention is a compound including, in a molecule, a structure A represented by the following A and a structure B represented by the following B, in which the compound includes two or more of the structures B in the molecule.

A: A structure in which at least one or more aromatic ring structures are included and a total number of $\pi$ electrons of the aromatic ring structure in the molecule is 8 or more. However, aryl amine structures represented by Formulae (I) to (IV) are excluded.

B: a fluorine-containing terminal structure represented by any one of Formulae (B-1), ..., or (B-5).

(I) 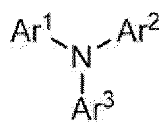

(II) 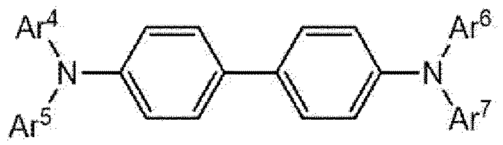

(III) 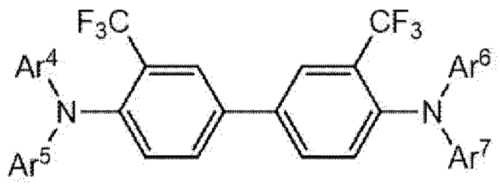

(IV) 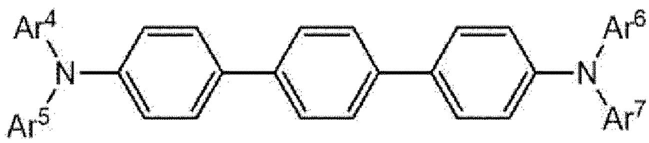

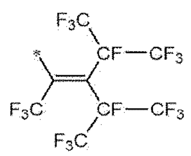
(B-1)

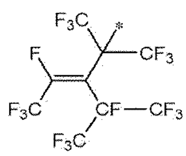
(B-2)

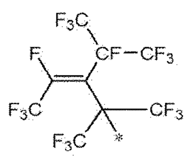
(B-3)

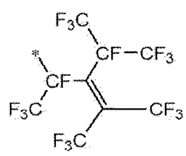
(B-4)

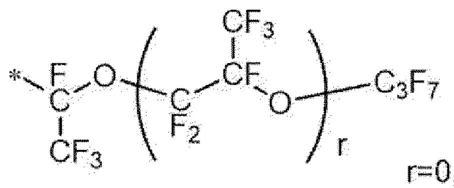

r=0,1,2

(B-5)

In Formulae (I) to (IV), $Ar^1$, $Ar^2$, $Ar^3$, $Ar^4$, $Ar^5$, $Ar^6$, and $Ar^7$ each independently represent a substituted or unsubstituted aryl group having 6 to 20 carbon atoms.

In addition, in Formulae (B-1) to (B-5), * represents a bonding position.

Since the compound according to the embodiment of the present invention has two or more structures B (fluorine-containing terminal structures) in the molecule, the compound has a function as a liquid crystal alignment accelerator.

Here, although a compound having a fluorinated alkyl group at the terminal is effective as the alignment accelerator, the use application of the alignment accelerator known in the related art has been restricted due to the fact that the range of the concentration to be used is narrow, the solubility is low, and a structure having concern about environmental harm is used.

On the other hand, the compound according to the embodiment of the present invention exhibits equal or higher alignment performance in a wider concentration range and has good solubility but does not have a long-chain perfluoroalkyl structure having concern about environmental harm, and thus a composition containing it has an advantage that it is easy to be used in production.

In addition, due to having alignment performance with respect to a polymerizable liquid crystal composition capable of being cured, it is useful in various use applications such as an optical member.

[Structure A]

As described above, the compound according to the embodiment of the present invention has the structure A represented by the following A.

A: A structure in which at least one or more aromatic ring structures are included and a total number of $\pi$ electrons of the aromatic ring structure in the molecule is 8 or more. However, aryl amine structures represented by Formulae (I) to (IV) are excluded.

Due to the reason that a more excellent liquid crystal alignment promoting property is exhibited, the structure A is preferably a structure represented by Formula (1) or (2).

$$Cy^1 \left[ \left( L^A - Cy^2 \right)_m \left( L^B - Cy^3 \right)_n \right]_p \quad (1)$$

$$L^C \left[ Cy^4 \right]_q \quad (2)$$

In Formula (1), m represents 0 or 1, n represents 1 or 2, and p represents an integer of 0 to 3. In a case where p is 2 or 3, a plurality of $L^A$'s, $Cy^2$'s, m's, $L^B$'s, $Cy^3$'s, and n's may be respectively the same or different from each other. In a case where n is 2, a plurality of $L^B$'s and $Cy^3$'s may be respectively the same or different from each other.

In addition, in Formula (1), $L^A$ and $L^B$ each independently represent a single bond or a divalent linking group.

In addition, in Formula (1), $Cy^1$, $Cy^2$, and $Cy^3$ each independently represent a ring structure represented by any one of Formulae (CY-1), ..., or (CY-23). However, at least one of $Cy^1$, $Cy^2$, or $Cy^3$ has a ring structure having aromaticity, and among $Cy^1$, $Cy^2$, and $Cy^3$, a total number of $\pi$ electrons of the ring structure exhibiting the aromaticity is 8 or more.

In Formula (2), q represents 3 or 4, where a plurality of $Cy^4$'s may be the same or different from each other.

In addition, in Formula (2), $L^C$ represents a q-valent linking group.

In Formula (2), $Cy^4$ represents a ring structure represented by any one of Formulae (CY-1), ..., or (CY-23). However, at least one of a plurality of $Cy^4$'s has a ring structure having aromaticity, and among the plurality of $Cy^4$'s, a total number of $\pi$ electrons of the ring structure exhibiting aromaticity is 8 or more.

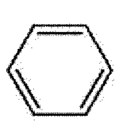
(CY-1)
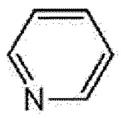
(CY-2)
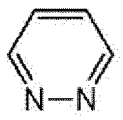
(CY-3)
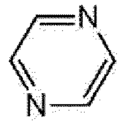
(CY-4)
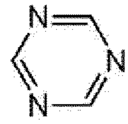
(CY-5)

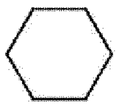
(CY-6)
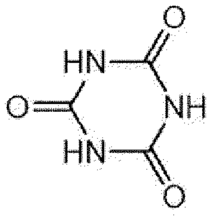
(CY-7)
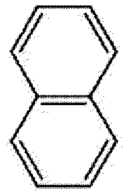
(CY-8)
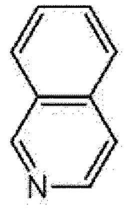
(CY-9)
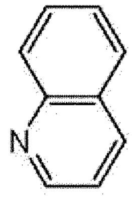
(CY-10)

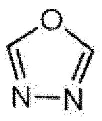
(CY-11)
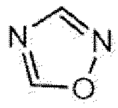
(CY-12)
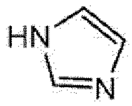
(CY-13)
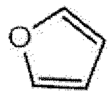
(CY-14)
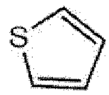
(CY-15)
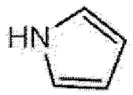
(CY-16)

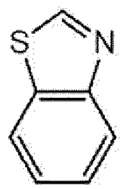
(CY-17)
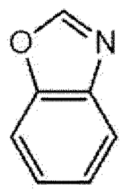
(CY-18)
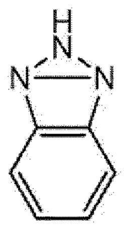
(CY-19)
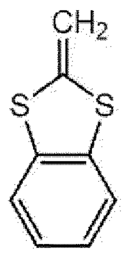
(CY-20)

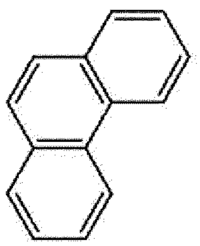
(CY-21)
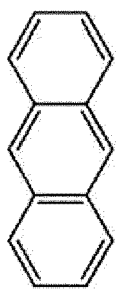
(CY-22)
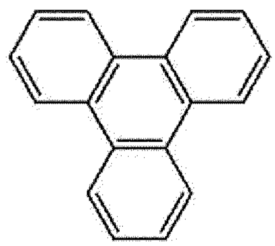
(CY-23)

The hydrogen atom in Formulae (CY-1) to (CY-23), that is, the hydrogen atom possessed by the carbon atom constituting the ring structure, the hydrogen atom of -NH-, or the hydrogen atom of $=CH_2$ may be substituted with a substituent or a group including the structure B.

Examples of the substituent include a halogen atom, a cyano group, a hydroxy group, an amino group, and a linear or branched alkyl group having 1 to 30 carbon atoms.

Here, any methylene group in the alkyl group may be substituted -O-, -S-, -NR-, -C=C-, -C=N-, -C=C-, or -C(=O)-, where R represents an alkyl group having 1 to 3 carbon atoms.

In addition, the hydrogen atom in the alkyl group may be substituted with a cyano group or a halogen atom, or it may be substituted with the structure B.

Among these substituents, a halogen atom, a cyano group, or an alkyl group having 1 to 10 carbon atoms is preferable, a group in which any methylene group in the alkyl group having 1 to 10 carbon atoms is substituted with -O-. -NR-, -C=C-, or -C(=O)- is more preferable, and a group in which the hydrogen atom in the alkyl group having 1 to 10 carbon atoms is substituted with the structure B is still more preferable.

In Formula (1), at least one of $Cy^1$, $Cy^2$, or $Cy^3$ has a ring structure having aromaticity, and among $Cy^1$, $Cy^2$, and $Cy^3$, a total number of $\pi$ electrons of the ring structure exhibiting the aromaticity is 8 or more.

In addition, in Formula (2), at least one of a plurality of $Cy^4$'s has a ring structure having aromaticity, and among the plurality of $Cy^4$'s, a total number of $\pi$ electrons of the ring structure exhibiting aromaticity is 8 or more.

Here, the ring structure having aromaticity refers to a ring structure in which the number of $\pi$ electrons is 4n + 2 (n is an integer of 0 or more), and suitable examples thereof include a phenyl group, a naphthyl group, a triphenylene group, and a fused-ring structure thereof.

It is noted that among Formulae (CY-1) to (CY-23), in Formulae (CY-1) to (CY-5) and Formulae (CY-8) to (CY-23), which are ring structures having aromaticity, the number of $\pi$ electrons is as follows.

Ring having 6 $\pi$ electrons: Formulae (CY-1) to (CY-5), (CY-11) to (CY-16), (CY-19), and (CY-20)
Ring having 10 $\pi$ electrons: Formulae (CY-8) to (CY-10), (CY-17), and (CY-18)
Ring having 14 $\pi$ electrons: Formulae (CY-21) and (CY-22)
Ring having 18 $\pi$ electrons: Formula (CY-23)

The aromatic ring structure contained in the structures represented by Formulae (1) and (2) preferably has two or more, and more preferably three or more, benzene rings (that is, those represented by Formula (CY-1)), for the intended purpose of enhancing liquid crystal alignment properties.

In Formula (1), $L^A$ and $L^B$ each independently represent a single bond or a divalent linking group.

Here, examples of the divalent linking group include a divalent group consisting of a combination of a carbon atom, an oxygen atom, a nitrogen atom, a sulfur atom, C=C, C=O, C=N, N=N, and C=C, where the carbon atom and the nitrogen atom may be optionally substituted with a hydrogen atom.

In addition, the divalent linking group is preferably a divalent linking group obtained by combining, for example, a linear alkylene group having 1 to 18 carbon atoms, a branched or cyclic alkylene group having 3 to 18 carbon atoms, an arylene group having 6 to 12 carbon atoms, which may have a substituent, an ether group (-O-), a carbonyl group (-C(=O)-), and at least two or more groups selected from the group consisting of imino groups (-NH-) which may have a substituent.

In the present invention, $L^A$ and $L^B$ are preferably a divalent linking group including a single bond or -O-.

In Formula (2), $L^C$ represents a q-valent (that is, trivalent or tetravalent) linking group.

Here, examples of the trivalent or tetravalent linking group include a trivalent or tetravalent group consisting of a combination of a carbon atom, an oxygen atom, a nitrogen atom, a sulfur atom, C=C, C=O, C=N, N=N, and C=C, where the carbon atom and the nitrogen atom may be optionally substituted with a hydrogen atom.

In addition, examples of the trivalent linking group include a group obtained by combining at least selected from a group consisting of an aliphatic hydrocarbon group, an aromatic hydrocarbon group, a heterocyclic group, and a combination thereof, an aliphatic hydrocarbon group, an aromatic hydrocarbon group, or a heterocyclic group; and at least one selected from -O-, -CO-, -COO-, -OCO-, or -NH-. Here, an aliphatic hydrocarbon group is preferable. The aliphatic hydrocarbon group preferably has 1 to 30 carbon atoms, more preferably 1 to 20 carbon atoms, and still more preferably 1 to 15 carbon atoms.

In addition, examples of the tetravalent linking group include a tetravalent arylene group and a group represented by the following formula. In the following formula, * represents a bonding position.

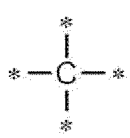
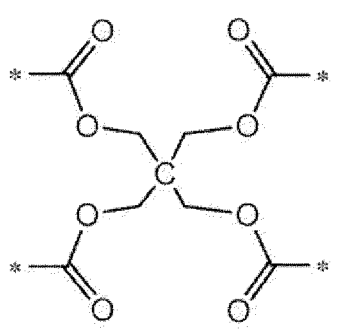

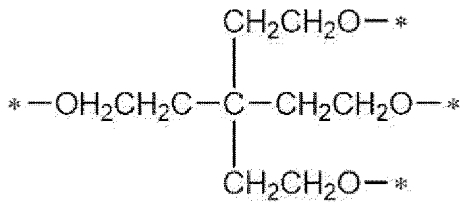
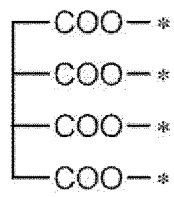

In Formula (1), m represents 0 or 1, n represents 1 or 2, and p represents an integer of 0 to 3. p is preferably an integer of 1 to 3 and more preferably 2 or 3.

In Formula (2), q represents 3 or 4.

In the present invention, due to the reason that a more excellent liquid crystal alignment promoting property is exhibited, the structure A, particularly the structure represented by Formula (1) is preferably a structure represented by any one of Formulae (1-1), ..., or (1-10).

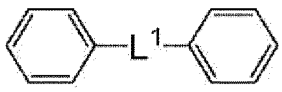
(1-1)

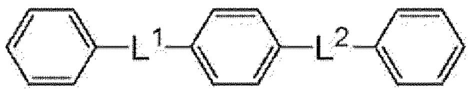
(1-2)

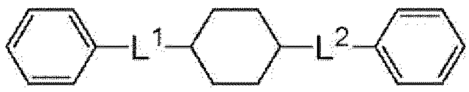
(1-3)

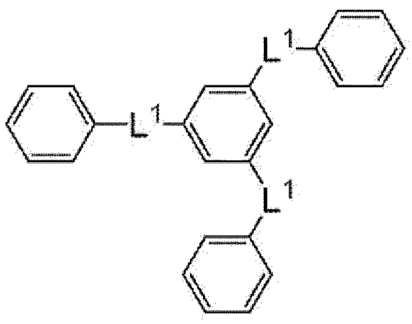
(1-4)

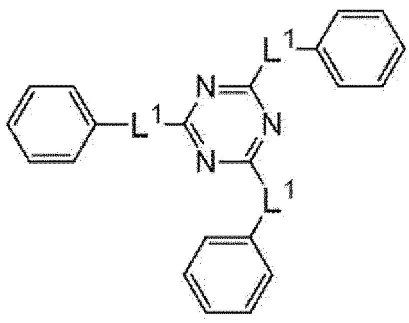
(1-5)

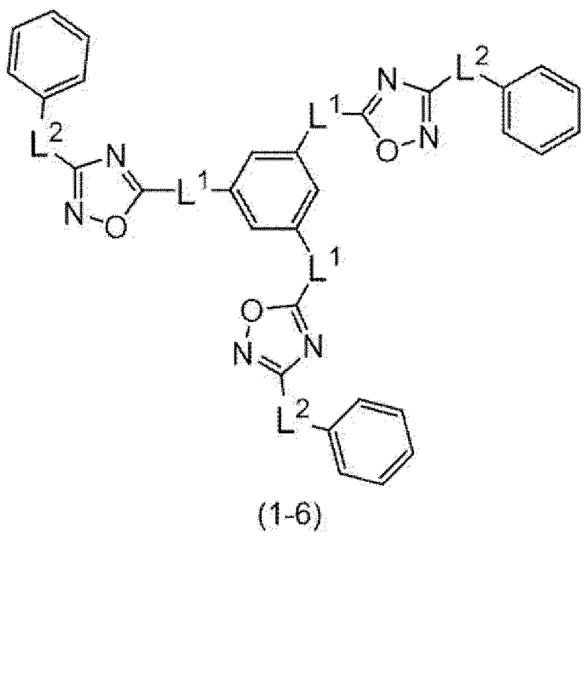
(1-6)

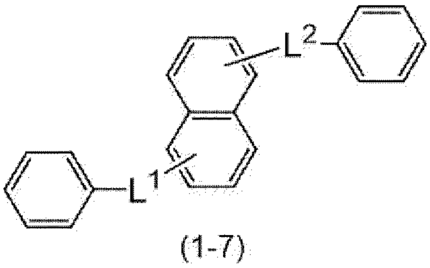

(1-7)

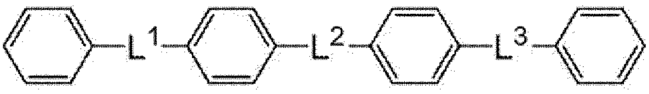

(1-8)

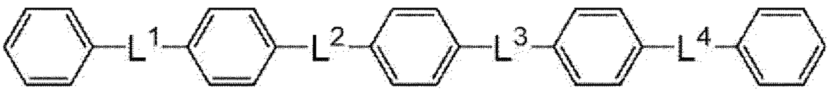

(1-9)

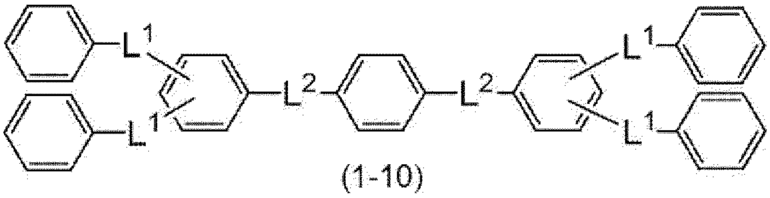

(1-10)

In Formulae (1-1) to (1 to 10), the hydrogen atom possessed by the carbon atom constituting the ring structure may be substituted with a substituent or a group including the structure B. It is noted that examples of the substituent include the same as the substituent with which the hydrogen atom in Formulae (CY-1) to (CY-23) may be substituted, and the same is applied to the preferred range.

In addition, in Formulae (1-1) to (1 to 10), $L^1$, $L^2$, $L^3$, and $L^4$ each independently represent a single bond or a divalent linking group.

Examples of the divalent linking group represented by one aspect of $L^1$, $L^2$, $L^3$, and $L^4$ include a divalent group consisting of a combination of a carbon atom, an oxygen atom, a nitrogen atom, a sulfur atom, C=C, C=O, C=N, N=N, and C=C, where the carbon atom and the nitrogen atom may be optionally substituted with a hydrogen atom.

In addition, the divalent linking group is preferably a divalent linking group obtained by combining, for example, a linear alkylene group having 1 to 18 carbon atoms, a branched or cyclic alkylene group having 3 to 18 carbon atoms, an arylene group having 6 to 12 carbon atoms, which may have a substituent, an ether group (-O-), a carbonyl group (-C(=O)-), and at least two or more groups selected from the group consisting of imino groups (-NH-) which may have a substituent.

In the present invention, $L^1$, $L^2$, $L^3$, and $L^4$ are each independently preferably a single bond or a divalent linking group containing an oxygen atom or a nitrogen atom, and it is more preferably a single bond or a divalent linking group of any one of -C(=O)O-, -OC(=O)-, -O-, -NH-C(=O)-, or -NH-.

In the present invention, due to the reason that a more excellent liquid crystal alignment promoting property is exhibited, it is preferable that the structure A, particularly the structure represented by Formula (2) is a structure represented by Formula (2-1) or (2-2).

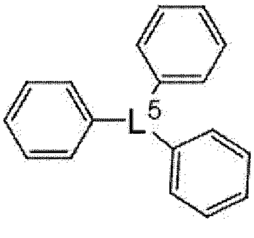

(2-1)

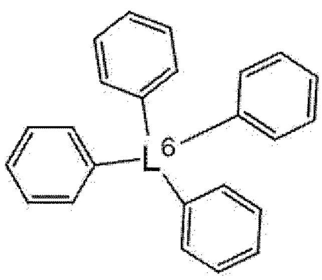

(2-2)

In Formulae (2-1) to (2 to 2), the hydrogen atom possessed by the carbon atom constituting the ring structure may be substituted with a substituent or a group including the structure B. It is noted that examples of the substituent

include the same as the substituent with which the hydrogen atom in Formulae (CY-1) to (CY-23) may be substituted, and the same is applied to the preferred range.

In addition, in Formula (2-1), $L^5$ represents a trivalent linking group, and in Formula (2-2), $L^6$ represents a tetravalent linking group.

Here, examples of the trivalent linking group represented by $L^5$ include a linear or branched trivalent structure having three or more atoms of the methylene carbon, and any methylene carbon may be substituted with an oxygen atom, a nitrogen atom, or a sulfur atom, C=C, or C=O, where the carbon atom and the nitrogen atom may be optionally substituted with a hydrogen atom. In addition, the number of methylene carbon atoms is more preferably 5 or more and still more preferably 7 or more.

Here, examples of the tetravalent linking group represented by $L^6$ include a linear or branched tetravalent structure having three or more atoms of the methylene carbon, and any methylene carbon may be substituted with an oxygen atom, a nitrogen atom, or a sulfur atom, C=C, or C=O, where the carbon atom and the nitrogen atom may be optionally substituted with a hydrogen atom. In addition, the number of methylene carbon atoms is more preferably 5 or more and still more preferably 7 or more.

[Structure B]

As described above, the compound according to the embodiment of the present invention has two or more structures B represented by the following B.

B: A fluorine-containing terminal structure represented by any one of Formulae (B-1), ..., or (B-5).

In Formulae (B-1) to (B-5), * represents a bonding position.

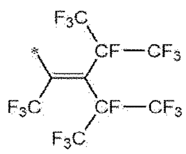
(B-1)

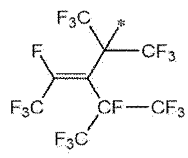
(B-2)

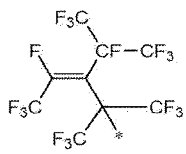
(B-3)

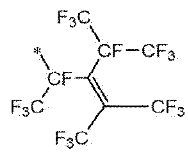
(B-4)

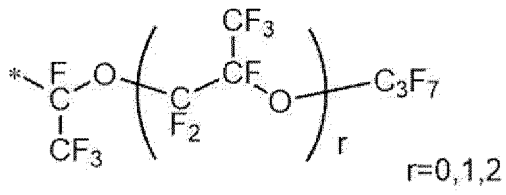
(B-5)

The compound according to the embodiment of the present invention has two or more of structures B in the molecule and preferably has four or more structures B.

In addition, the compound according to the embodiment of the present invention preferably has 3 or more and 10 or less of structures B in the molecule and more preferably 4 or more and 6 or less of structures B.

In addition, in order to further enhance the effect of enhancing liquid crystal alignment properties, it is preferable that at least two or more of structures B are substituted to have steric symmetry.

Due to the reason that the usable concentration range is further widened, the compound according to the embodiment of the present invention has, as the structure B, preferably two or more fluorine-containing terminal structures represented by Formula (B-5) and more preferably has 4 to 6 fluorine-containing terminal structures represented by Formula (B-5).

Further, due to the reason that the usable concentration range is still further widened, it is preferable that the compound according to the embodiment of the present invention has 4 to 6 fluorine-containing terminal structures represented by Formula (B-5), as the structure B, and a ring structure represented by Formula (CY-1) or (CY-8), as a ring structure located at the center of the molecule among the structures A.

The linking group that links the structure A and the structure B (hereinafter, abbreviated as a "linking group X") is not limited; however, examples thereof include a single bond and a linear or branched alkylene group having 1 to 30 carbon atoms.

Here, any methylene group in the alkylene group may be substituted -O-, -S-, -NR-, -C=C-, -C=N-, -C=C-, or -C(=O)-. However, R represents an alkyl group having 1 to 3 carbon atoms.

Among the above, the linking group X is preferably a linear alkylene group having 1 to 10 carbon atoms. It is

more preferably an alkylene group having 2 to 7 carbon atoms. In particular, it preferably has a structure in which any methylene group in the alkylene group is substituted with -C(=O)- or -O-.

Specific examples of the compound suitable for the present invention will be shown below. However, the compound that can be adopted in the present invention should not be construed as being limited by the following specific examples.

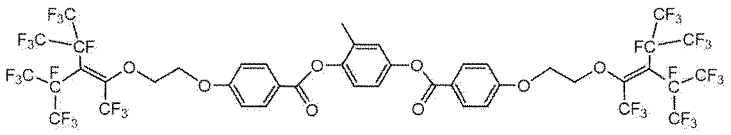
A-101

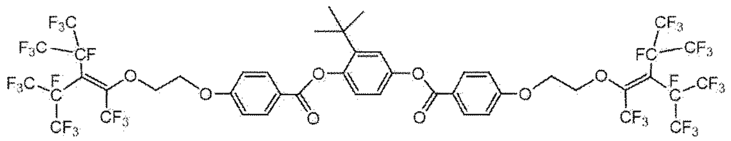
A-102

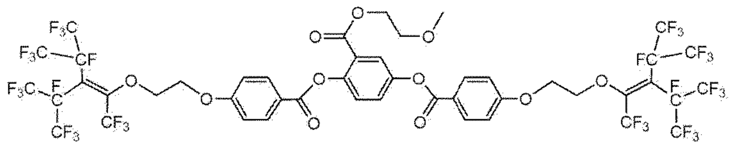
A-103

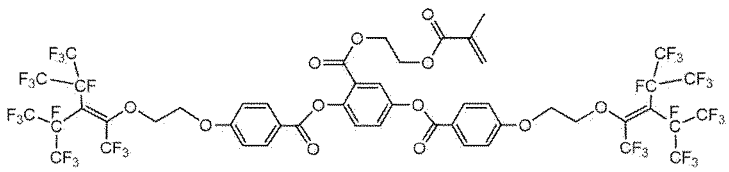
A-104

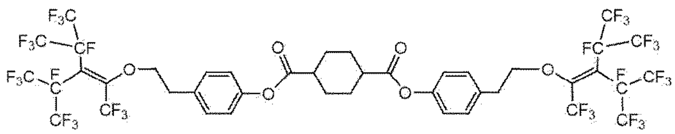
A-105

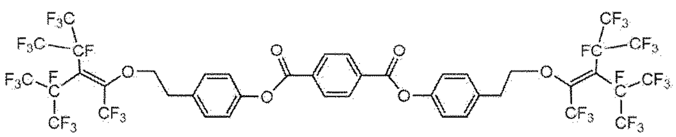
A-106

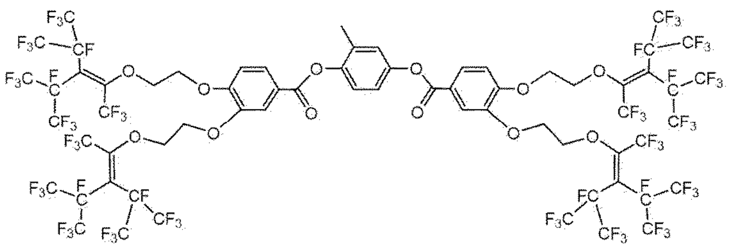
A-107

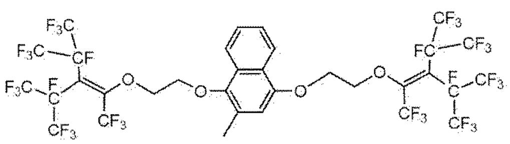
A-108

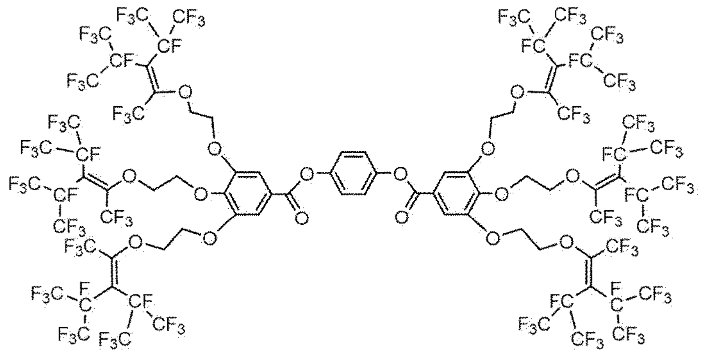

A-109

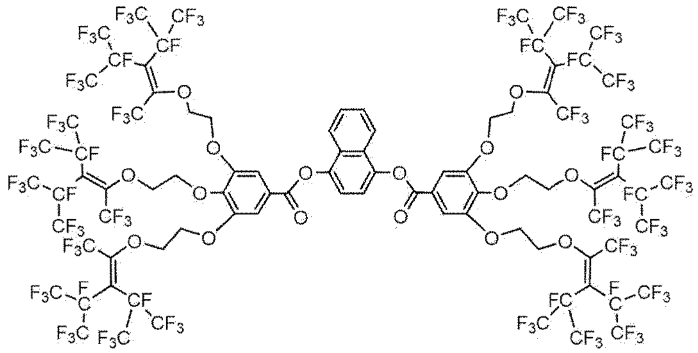

A-110

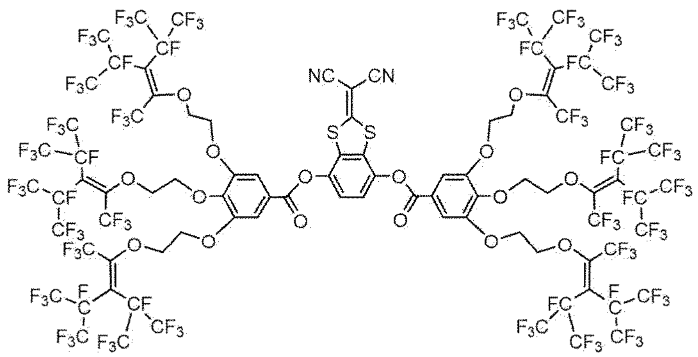

A-111

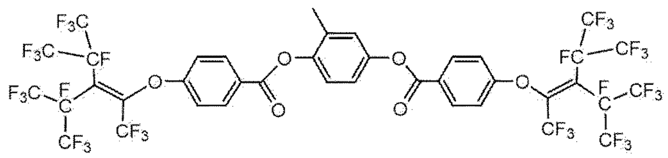

A-112

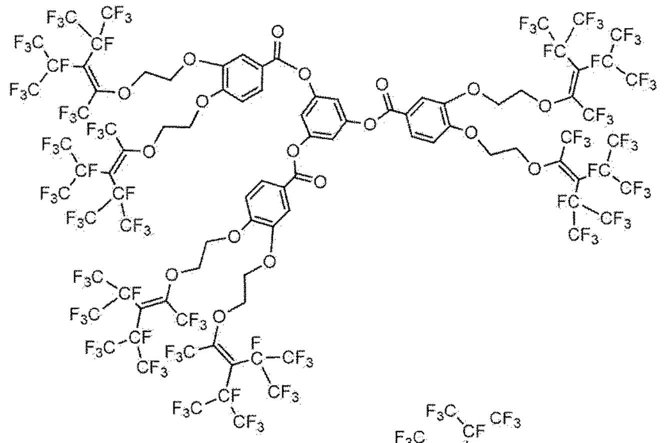

A-201

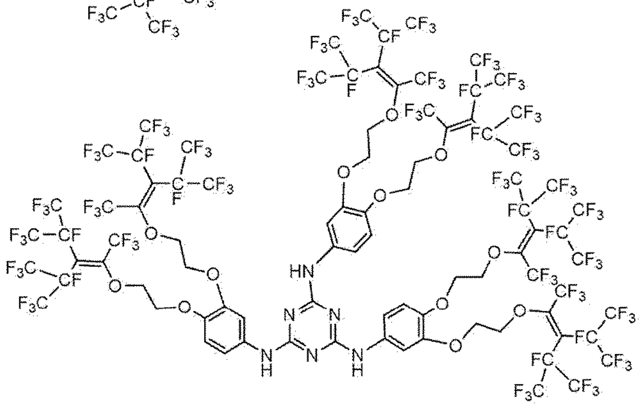

A-202

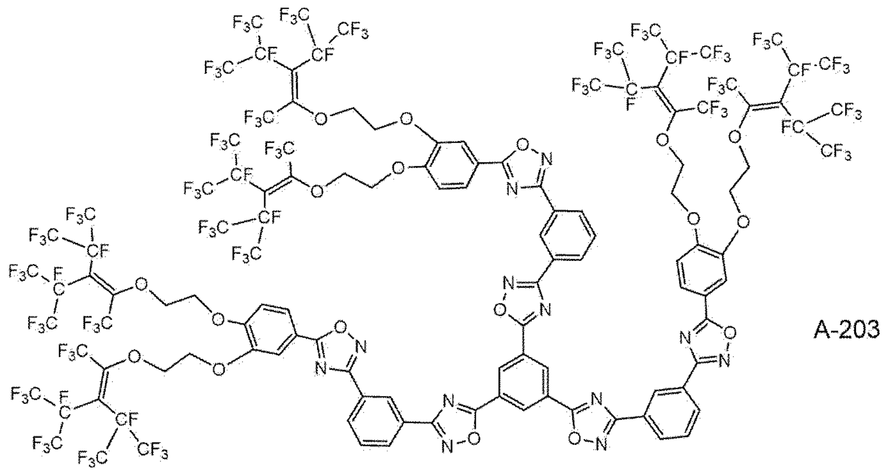

A-203

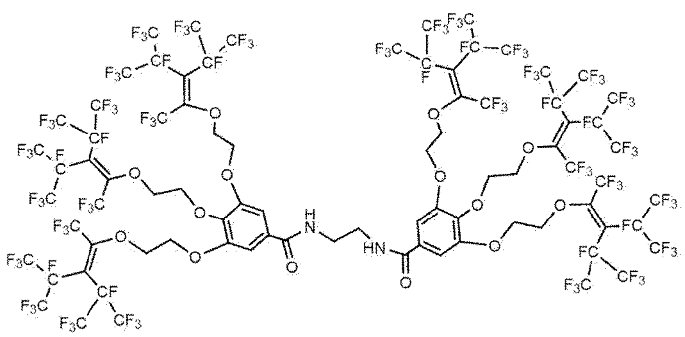

A-301

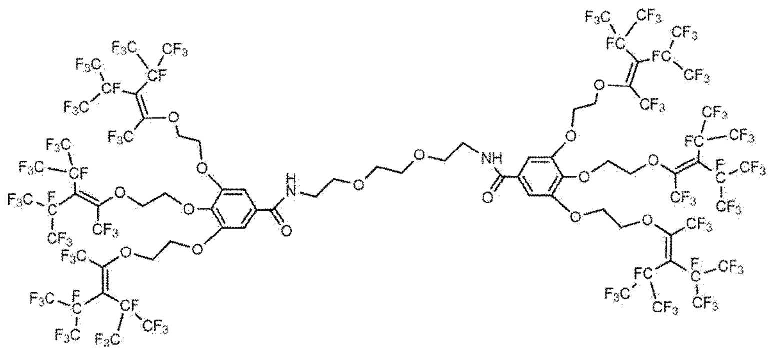

A-302

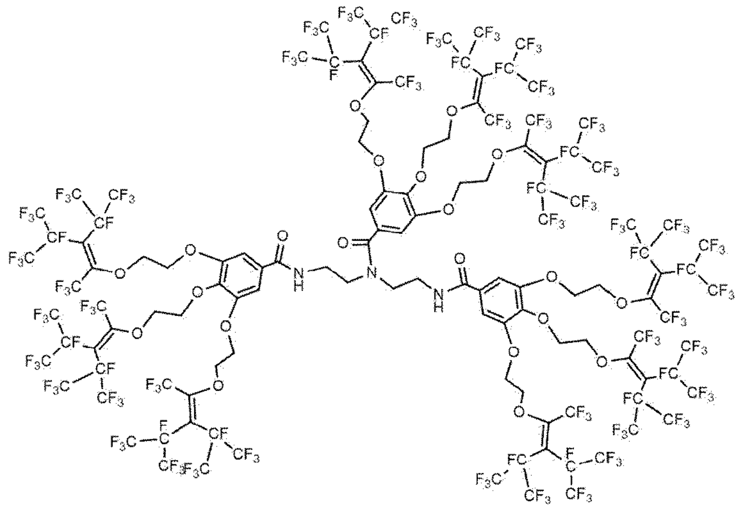

A-303

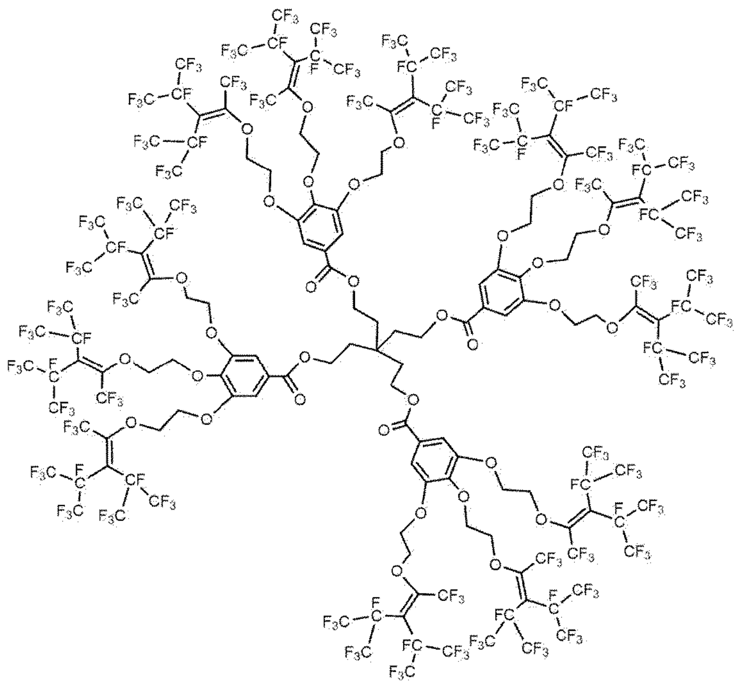

A-304

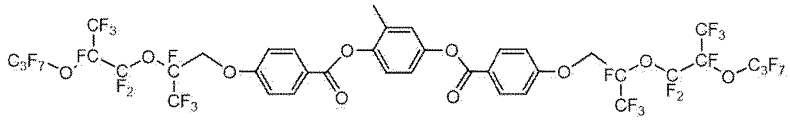

A-401

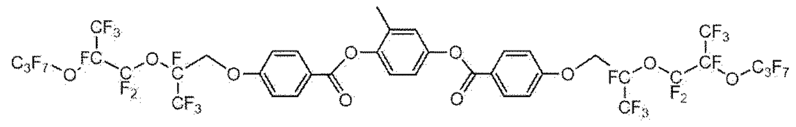

A-402

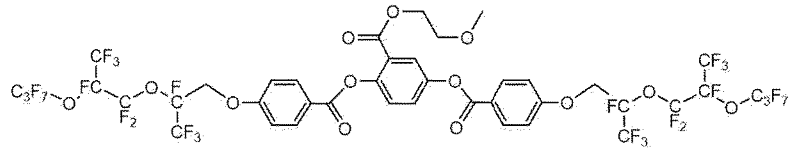

A-403

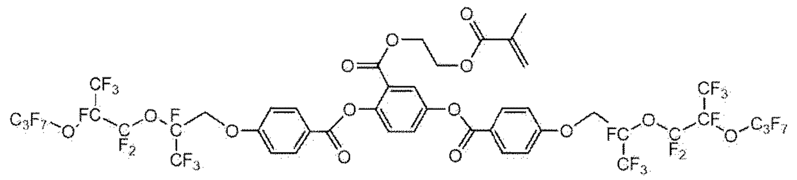

A-404

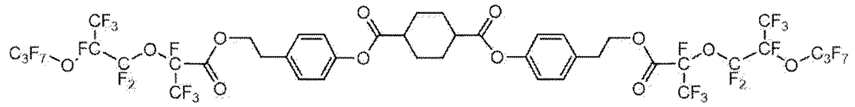

A-405

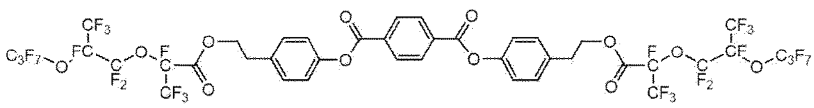

A-406

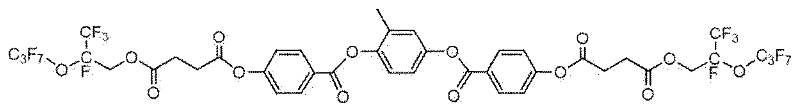

A-407

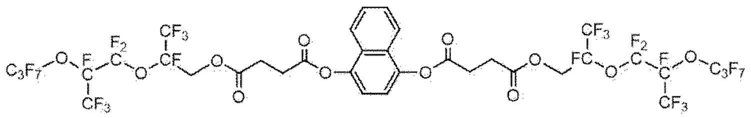

A-408

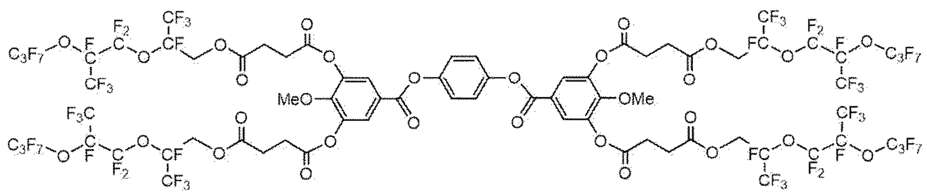

A-409

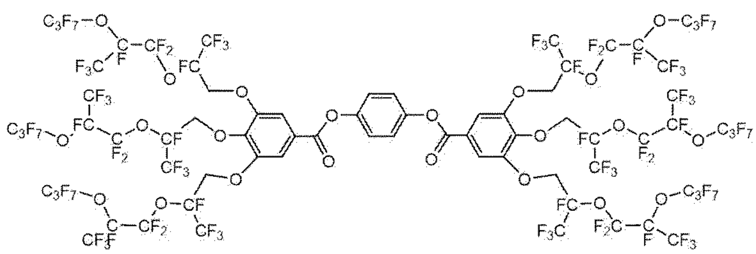

A-410

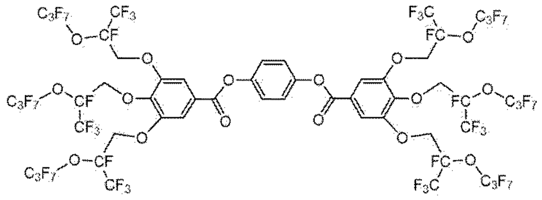

A-411

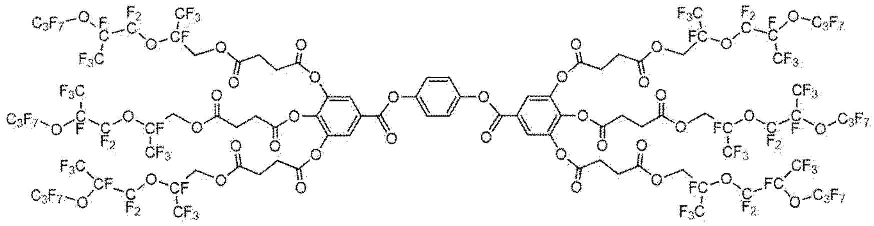

A-412

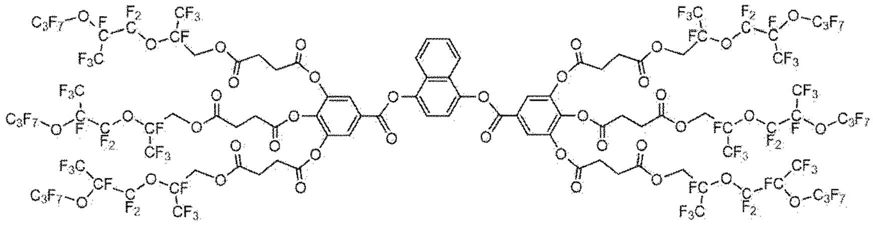

A-413

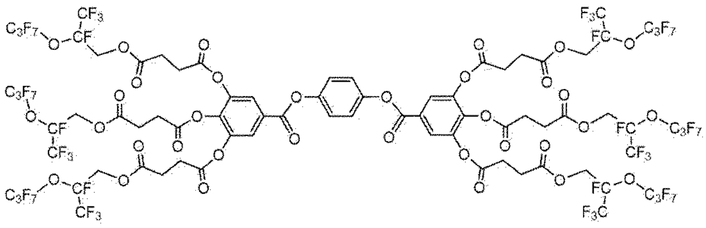

A-414

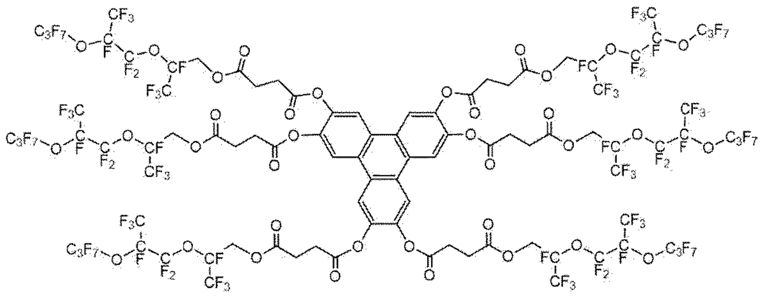

A-415

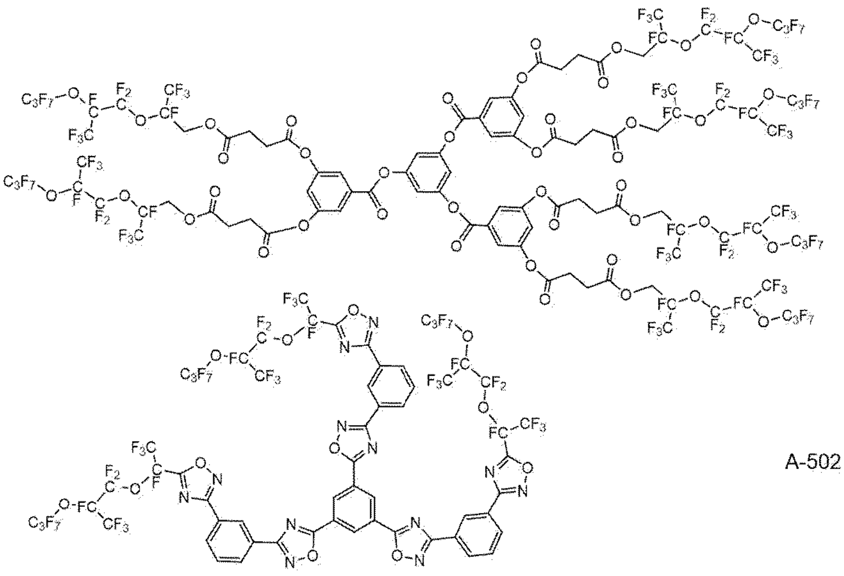

A-501

A-502

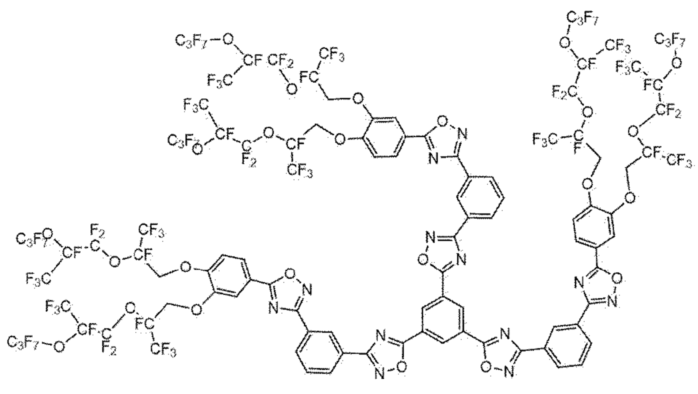

A-503

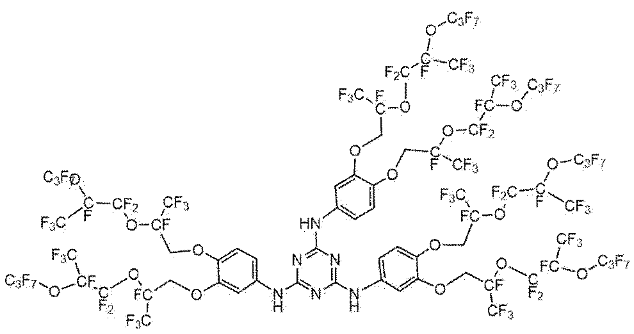

A-504

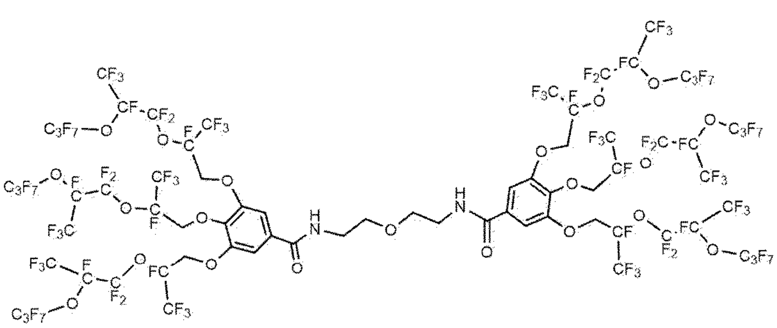

A-601

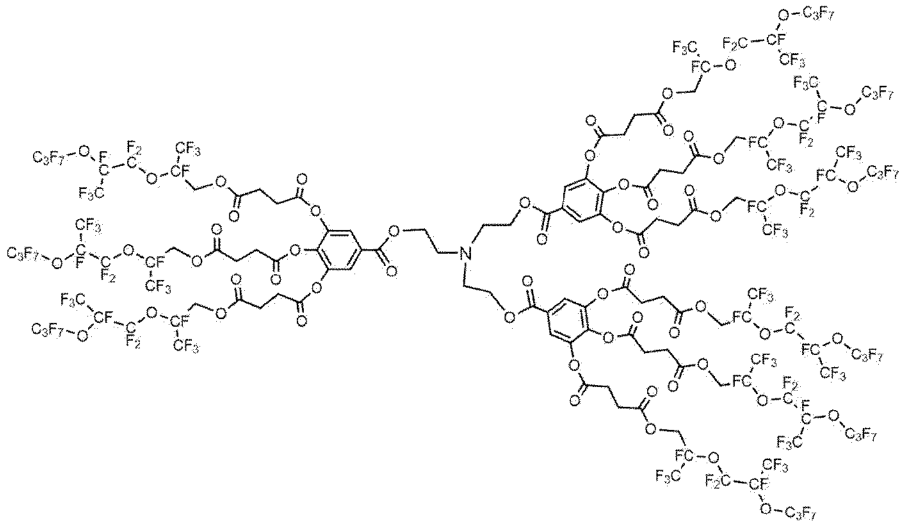

A-602

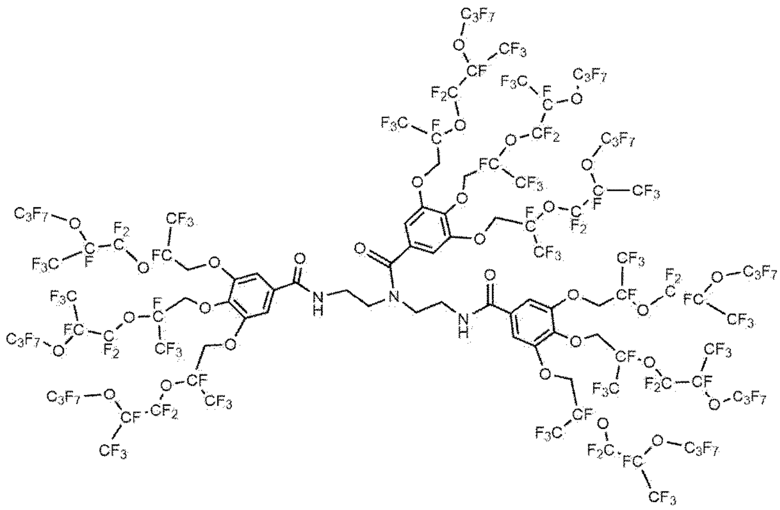

A-603

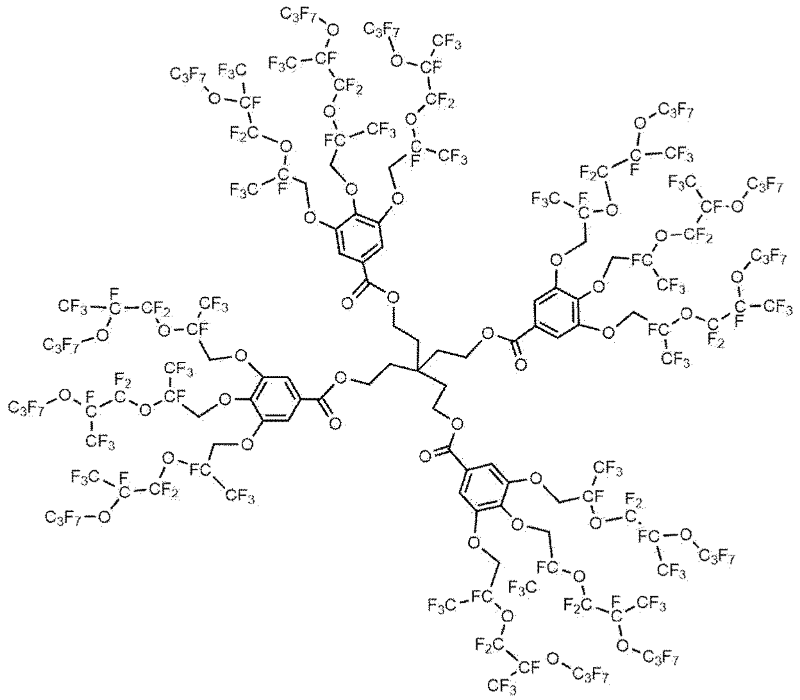

A-604

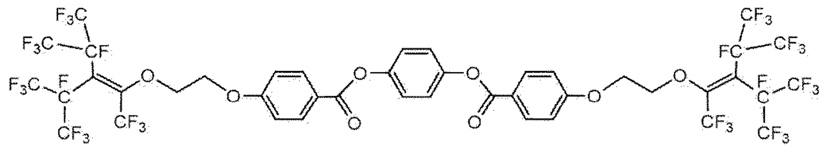

A-701

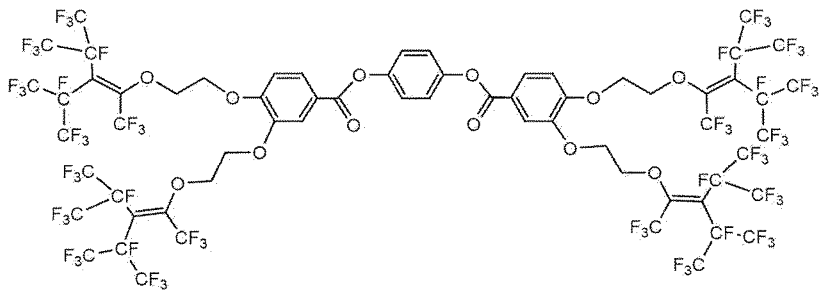

A-702

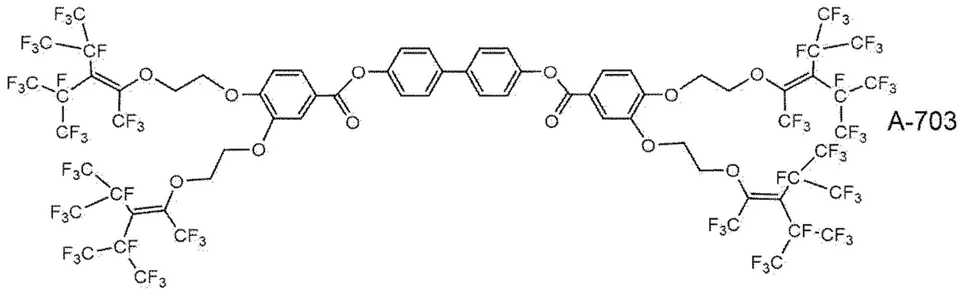

A-703

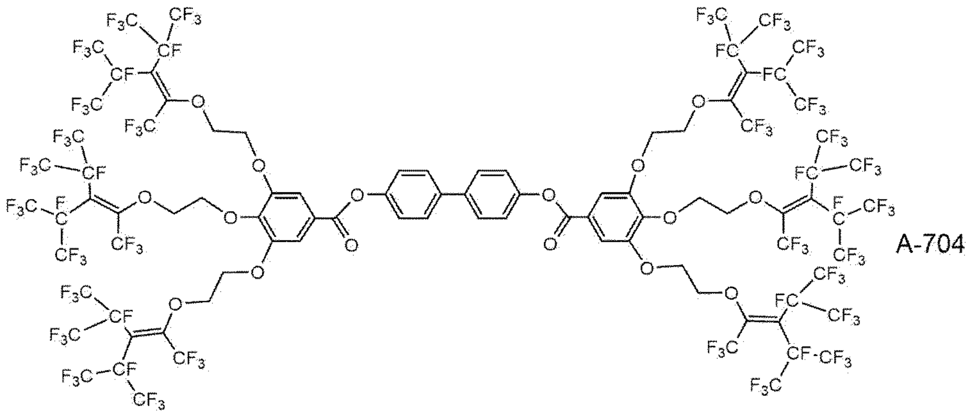

A-704

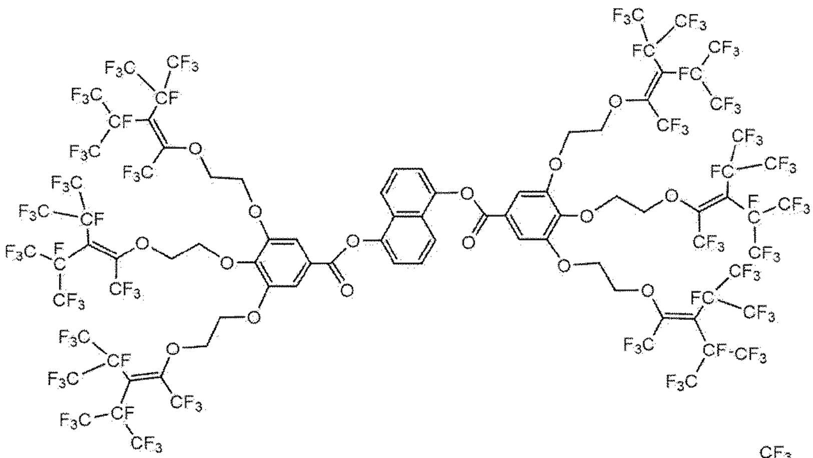

A-705

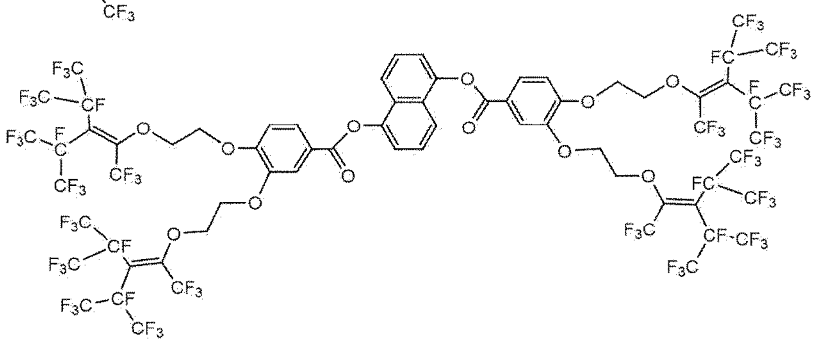

A-706

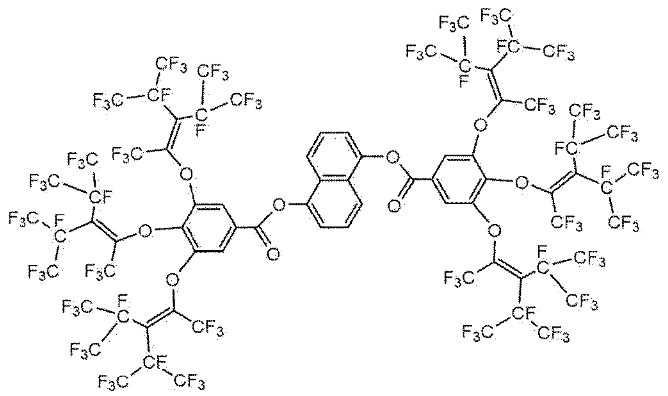

A-707

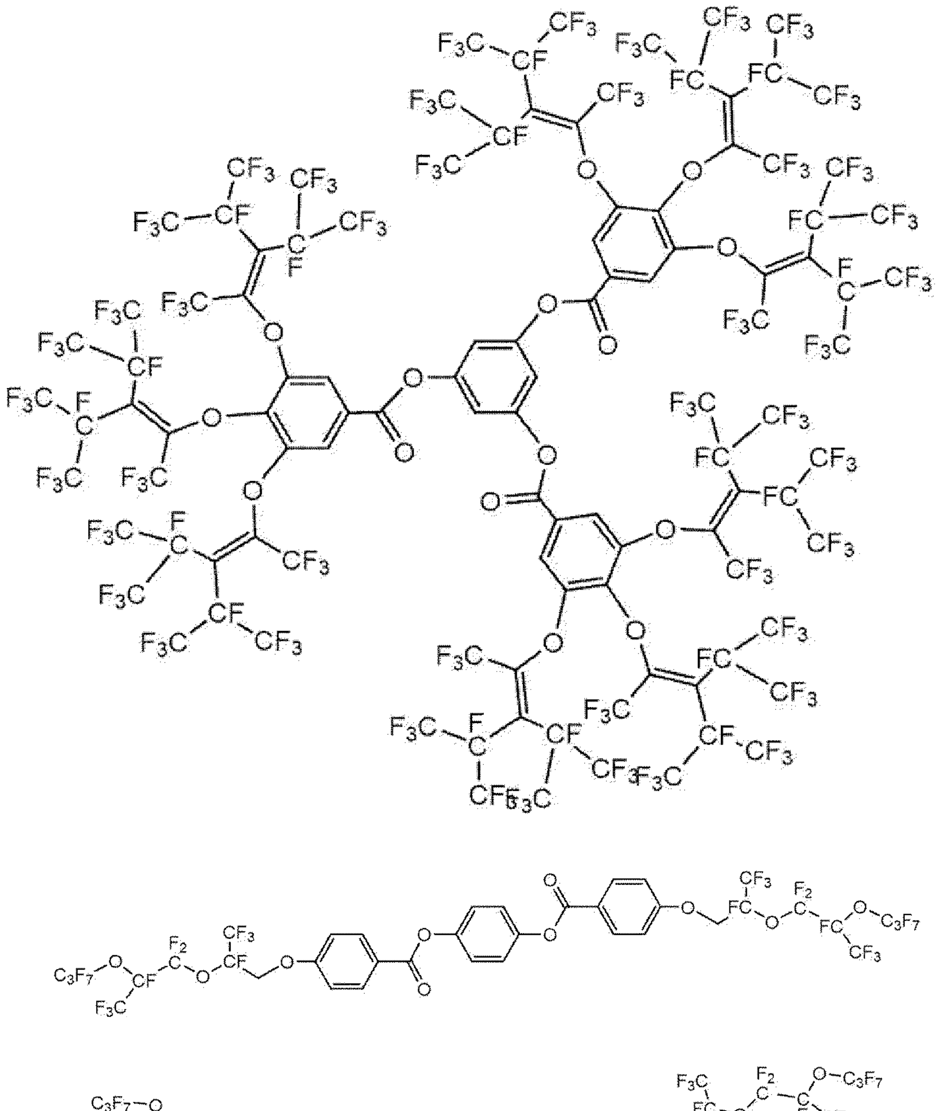

A-708

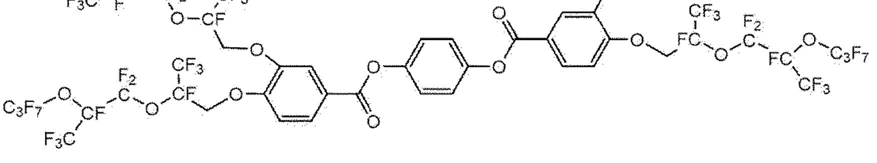

A-801

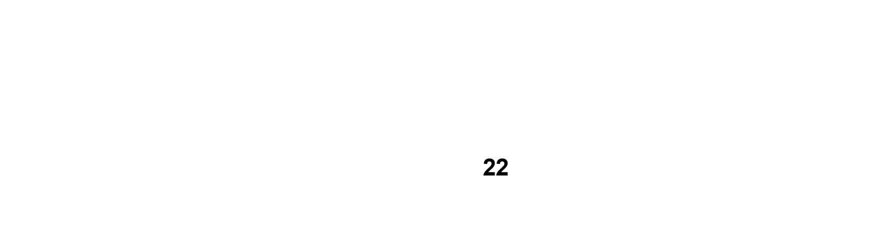

A-802

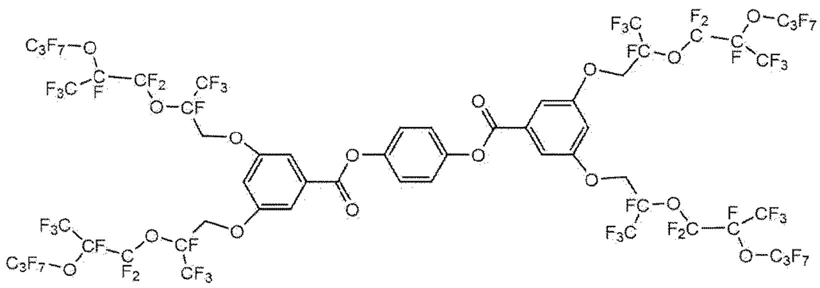

A-803

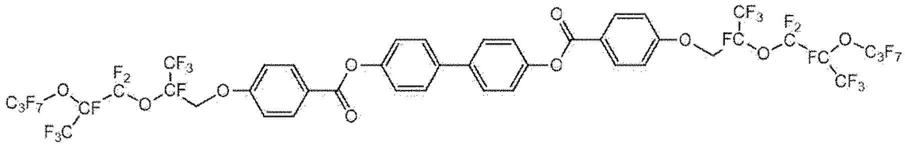

A-804

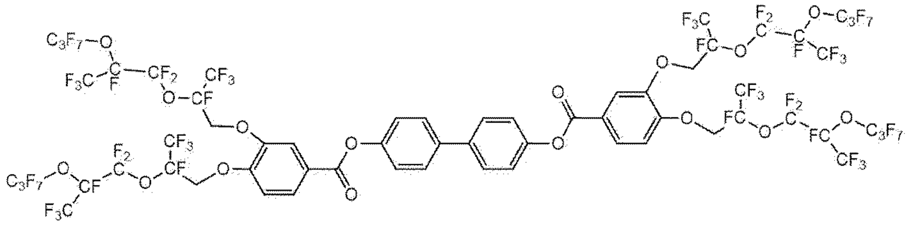

A-805

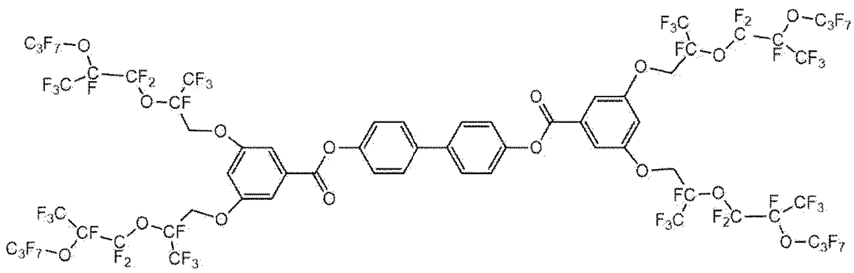

A-806

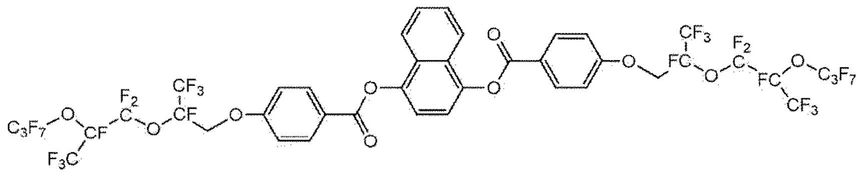

A-807

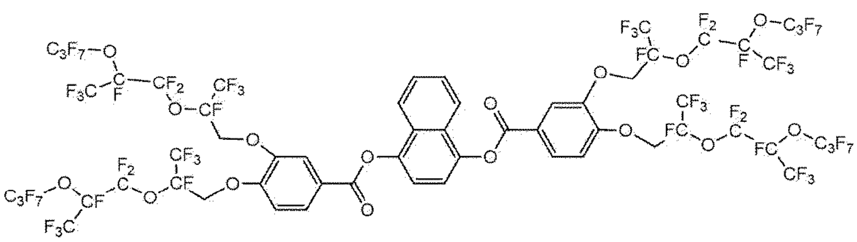

A-808

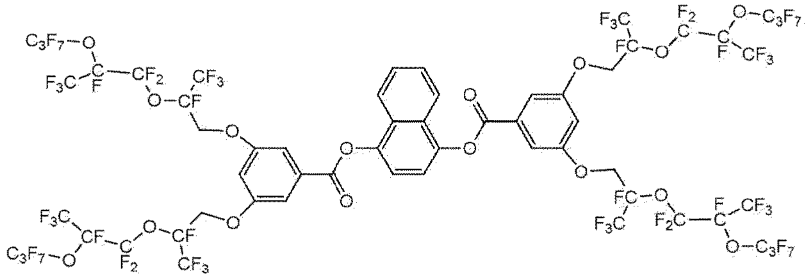

A-809

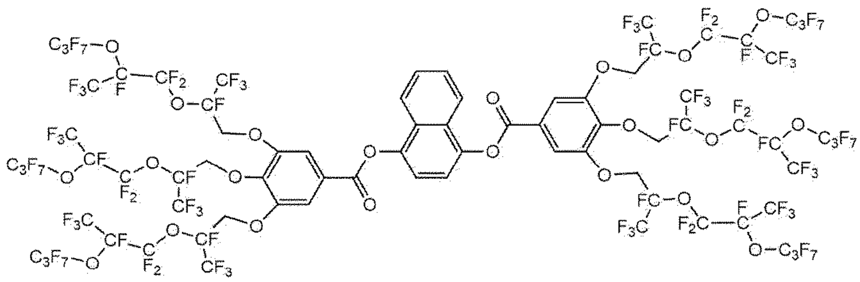

A-810

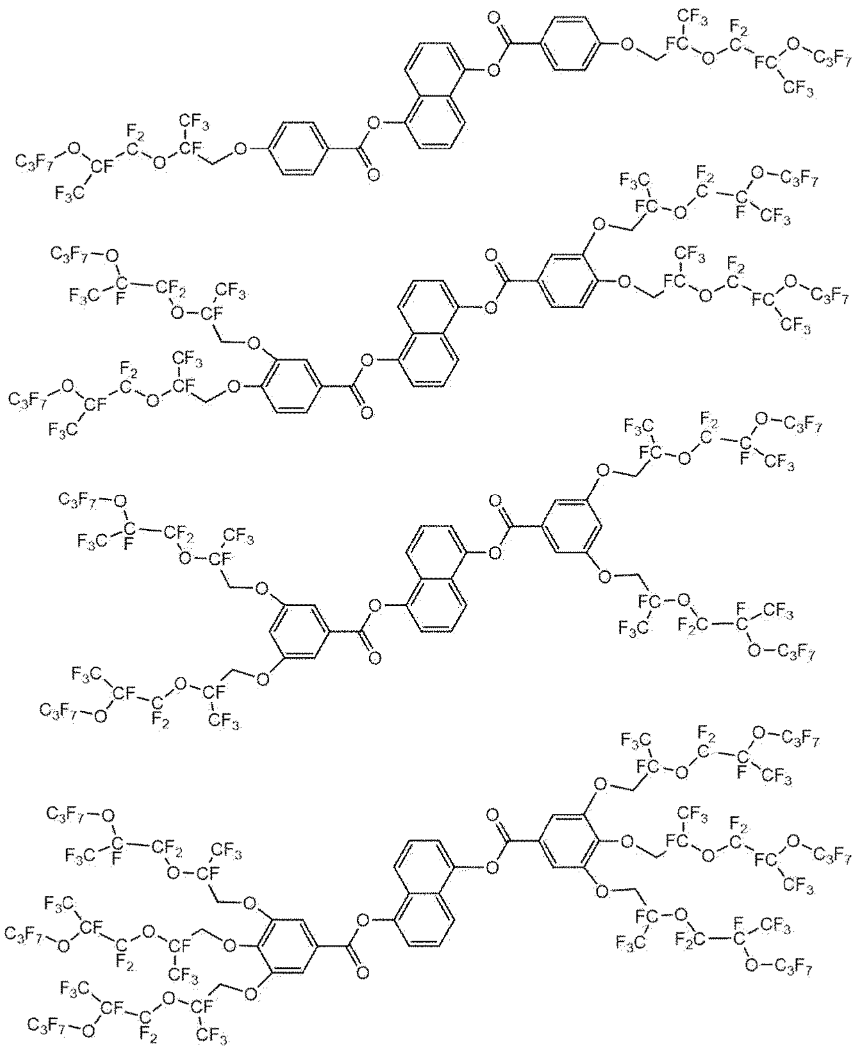

A-811

A-812

A-813

A-814

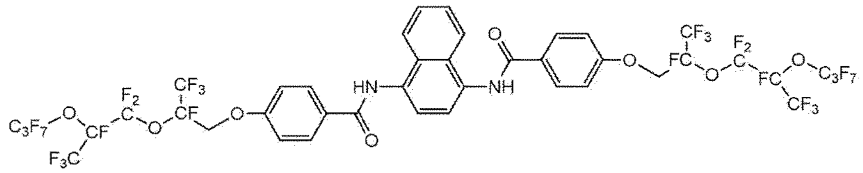

A-815

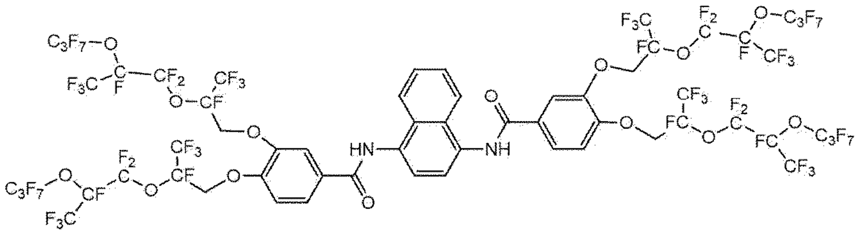

A-816

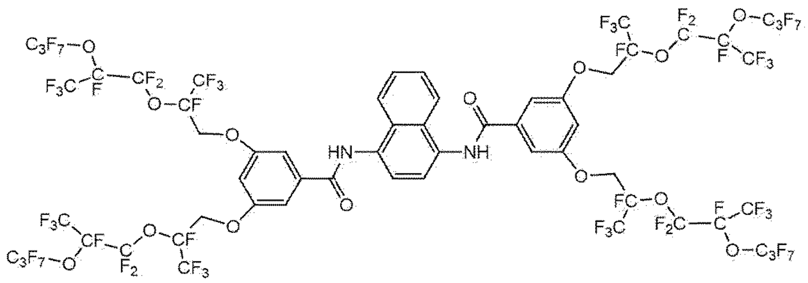

A-817

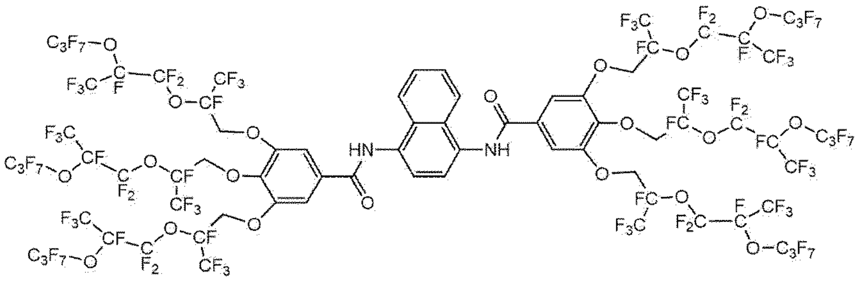

A-818

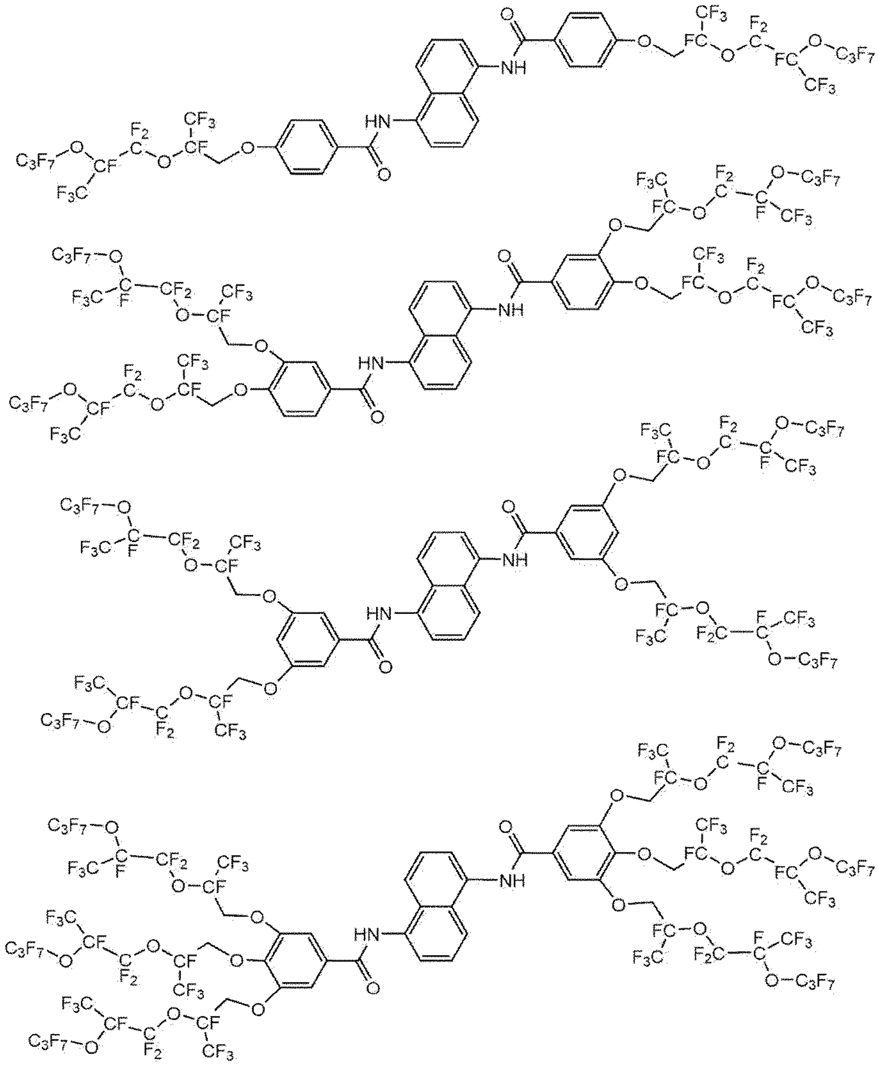

A-819

A-820

A-821

A-822

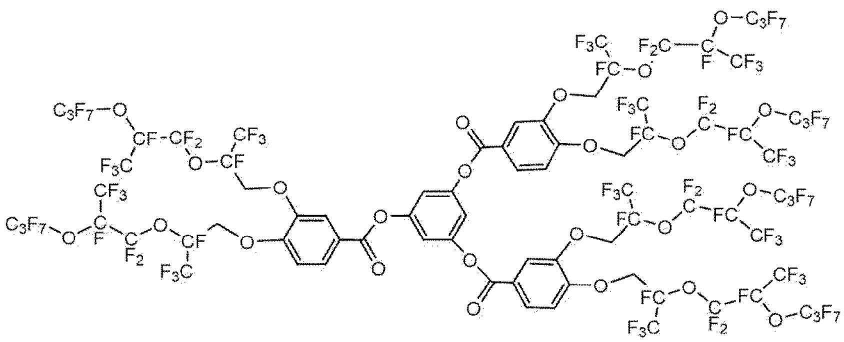

A-823

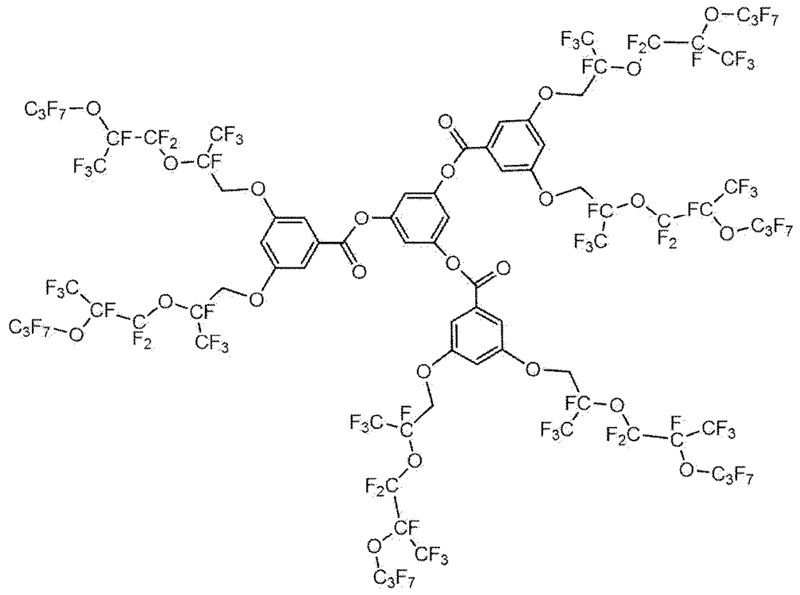

A-824

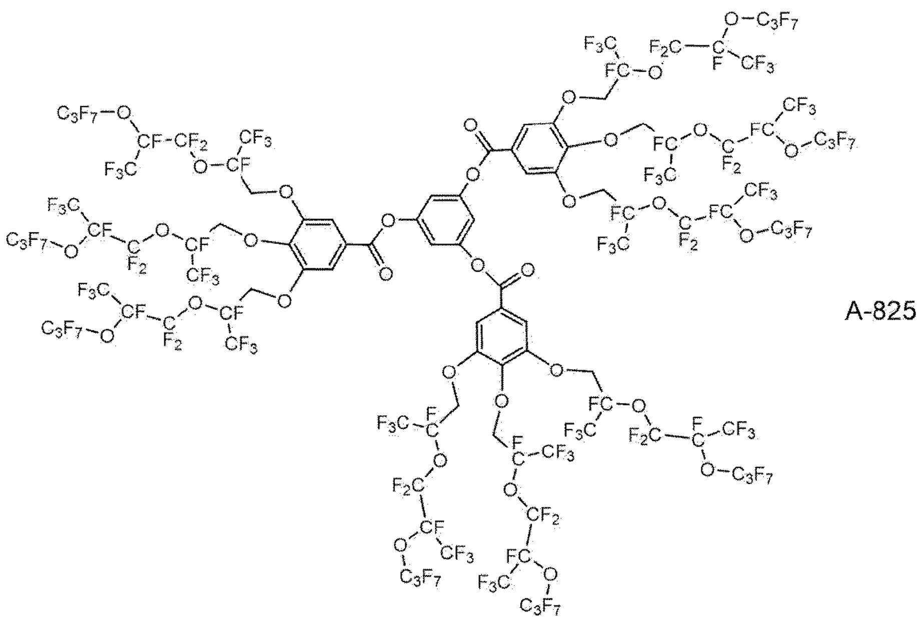

A-825

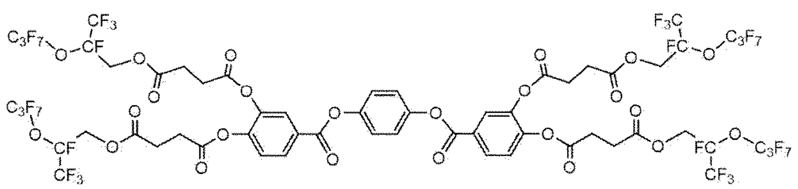

A-901

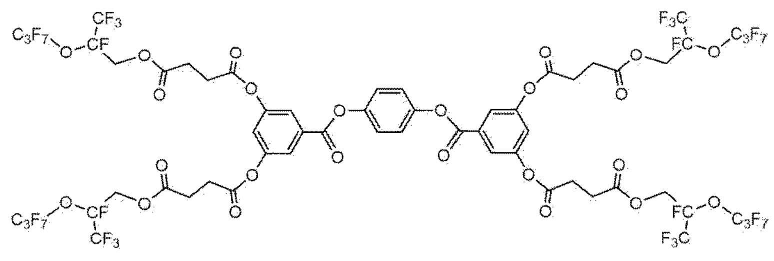

A-902

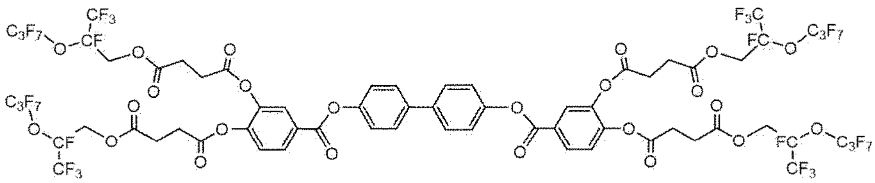

A-903

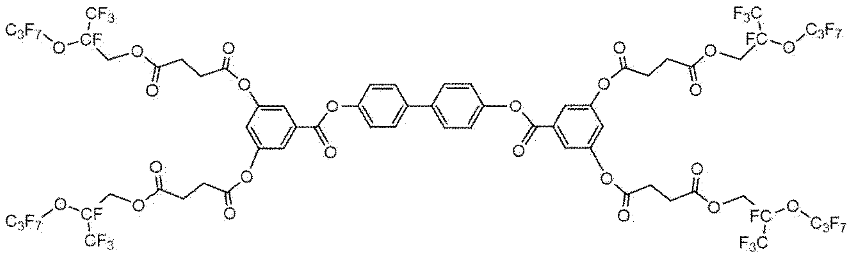

A-904

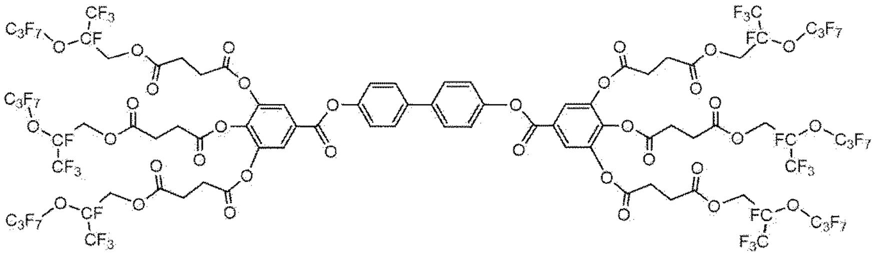

A-905

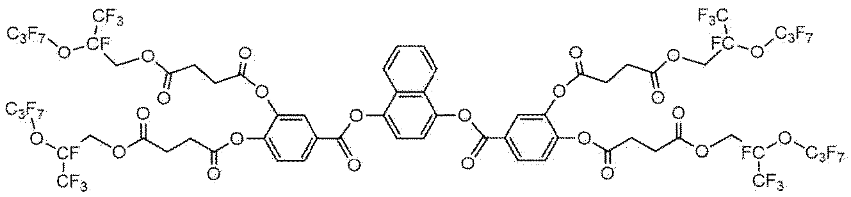

A-906

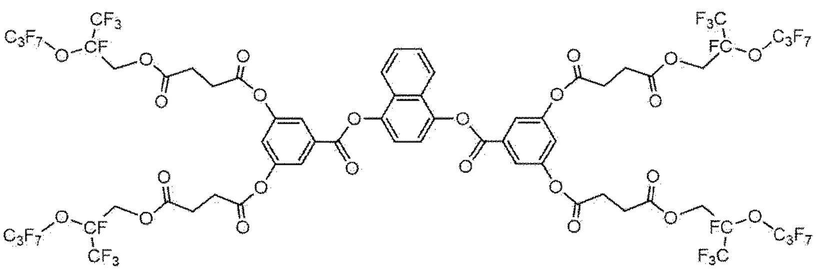

A-907

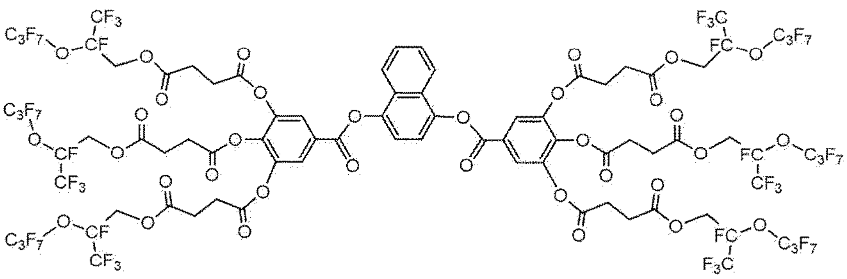

A-908

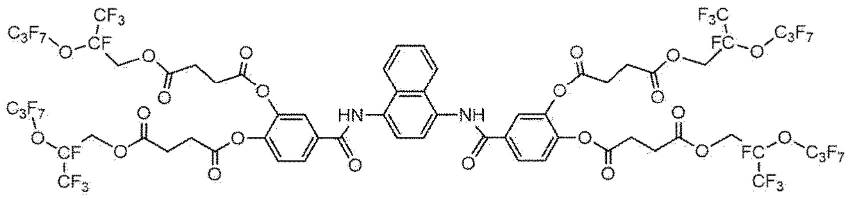

A-909

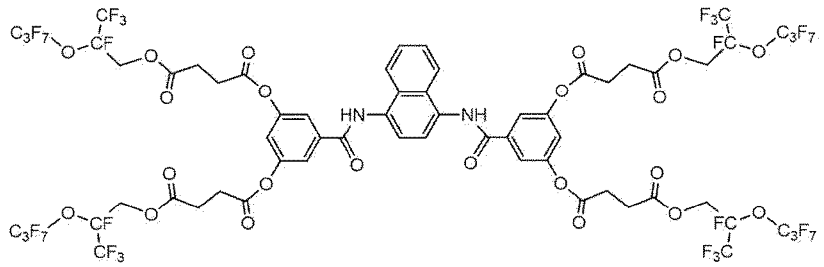

A-910

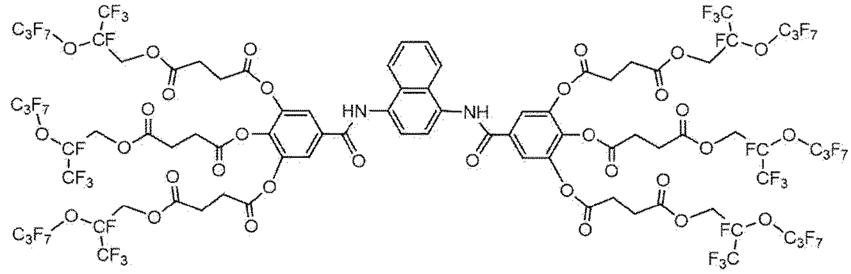

A-911

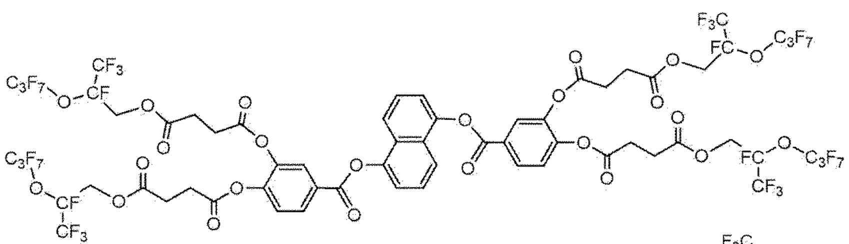

A-912

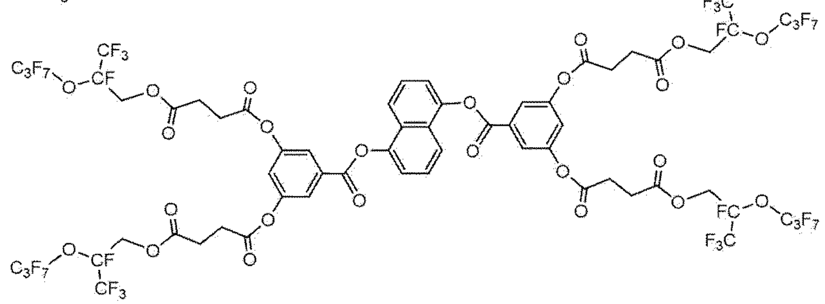

A-913

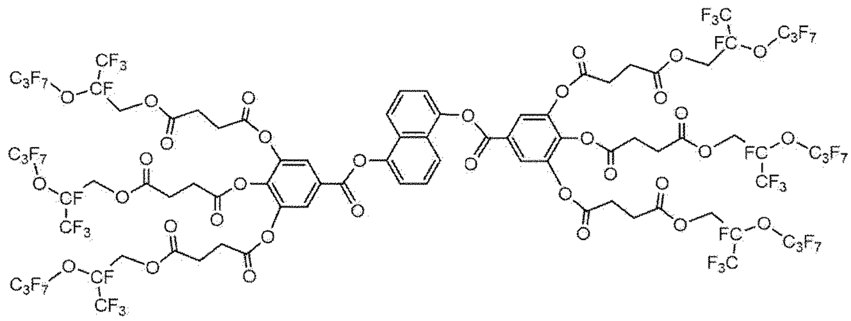

A-914

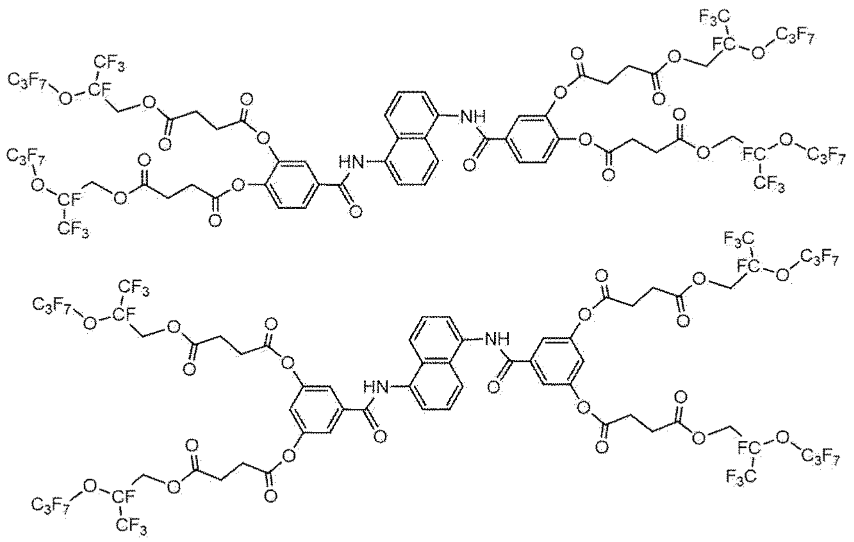

A-915

A-916

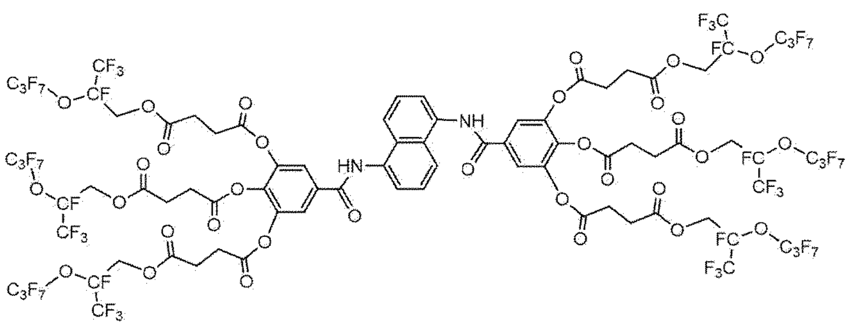

A-917

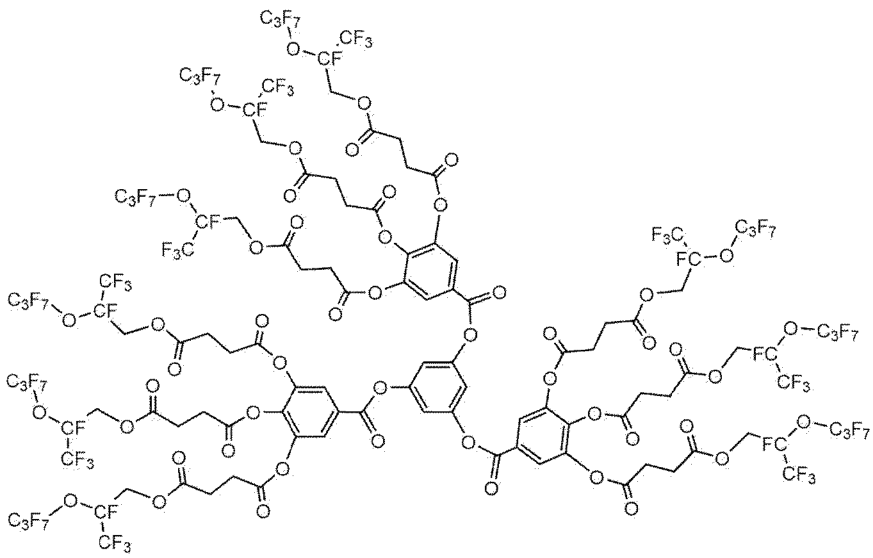

A-918

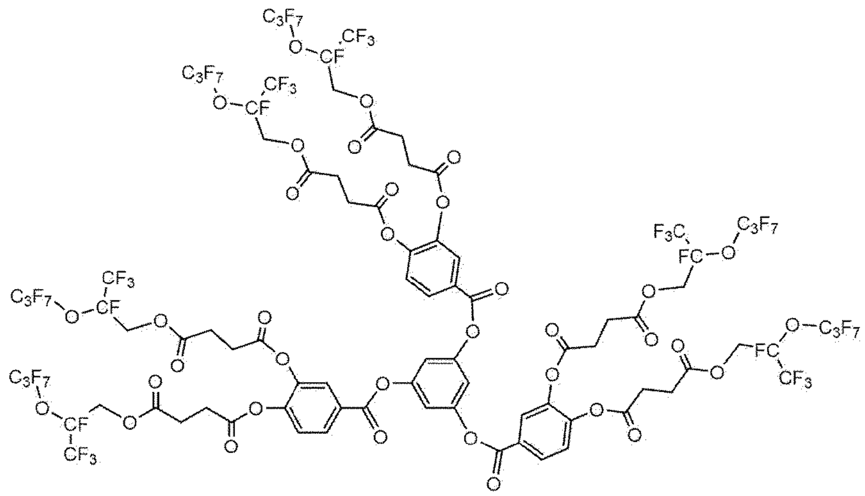

A-919

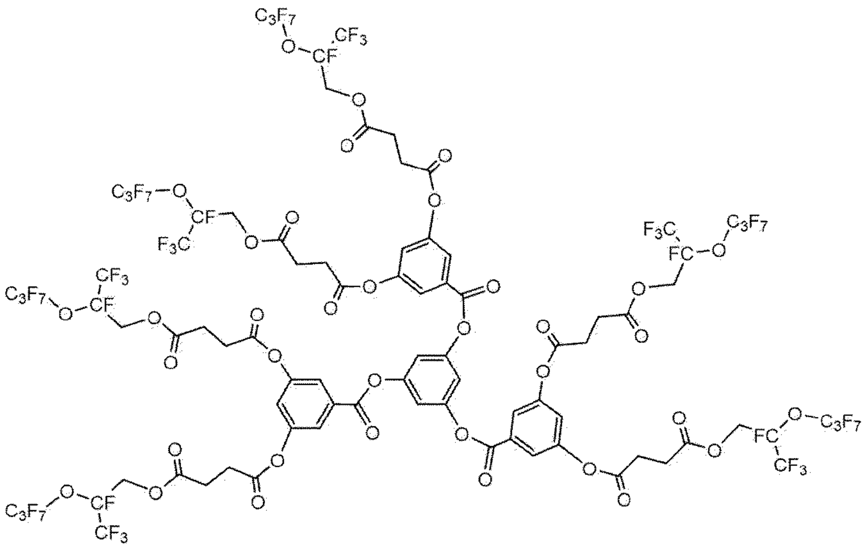

A-920

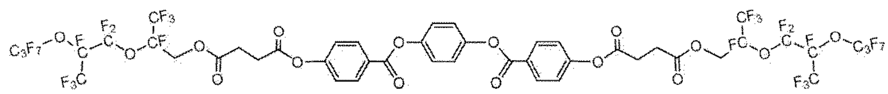

A-1001

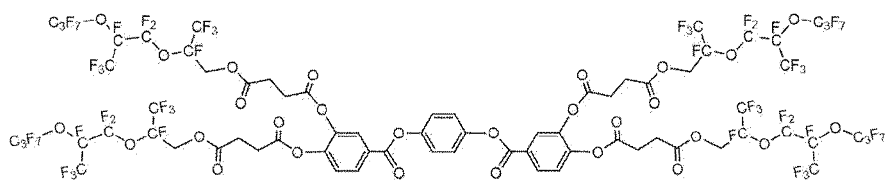

A-1002

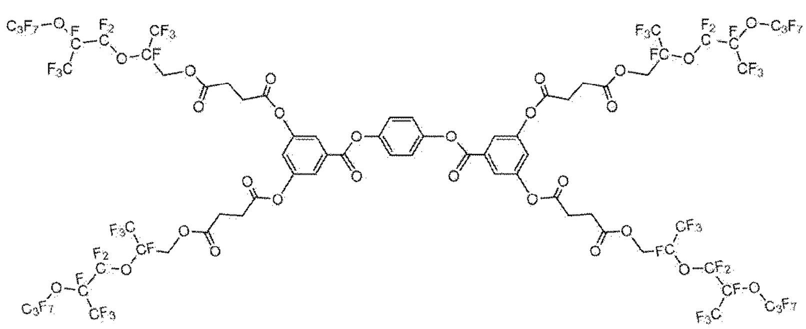

A-1003

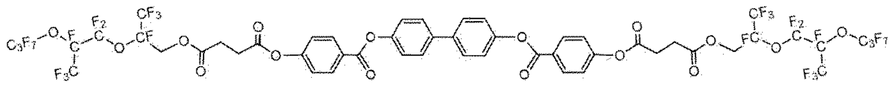

A-1004

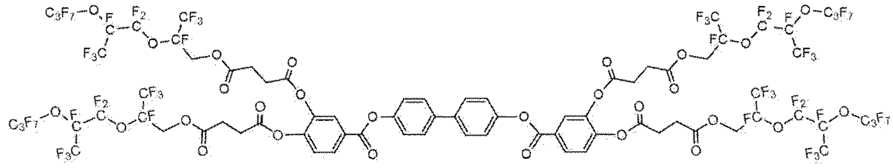

A-1005

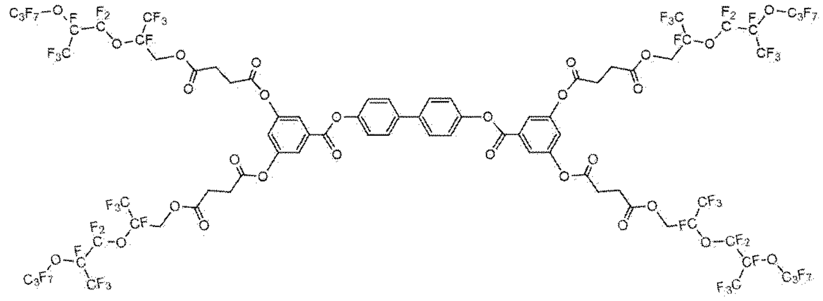

A-1006

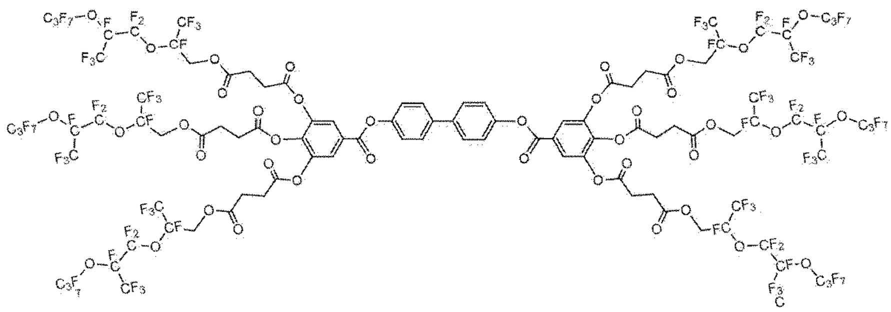

A-1007

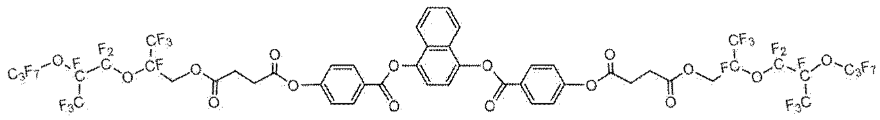

A-1008

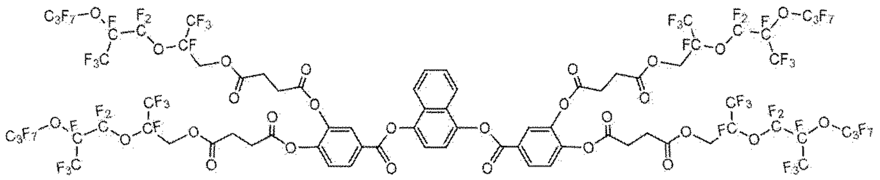

A-1009

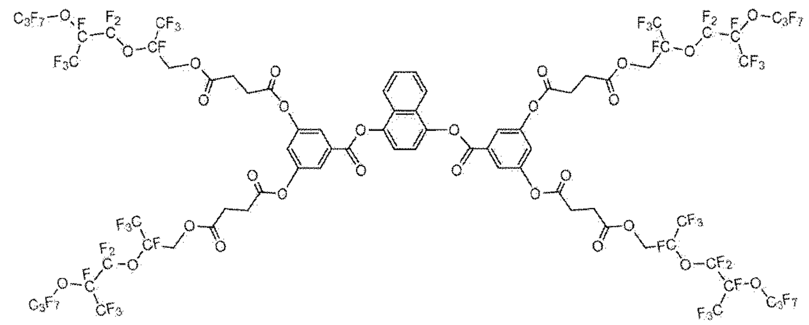

A-1010

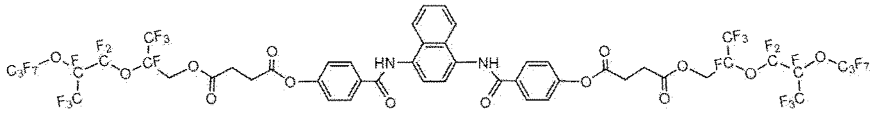

A-1011

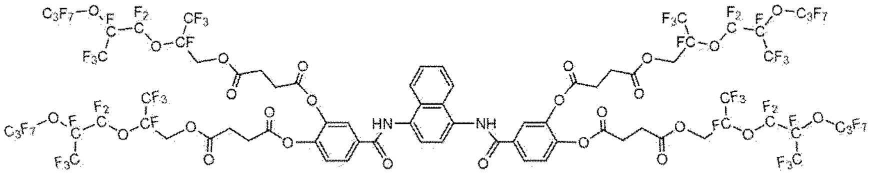

A-1012

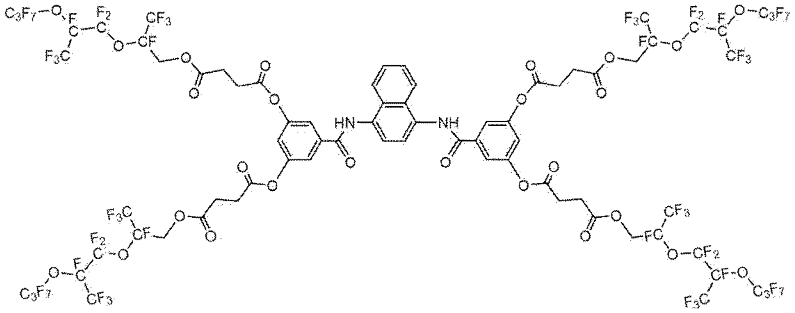

A-1013

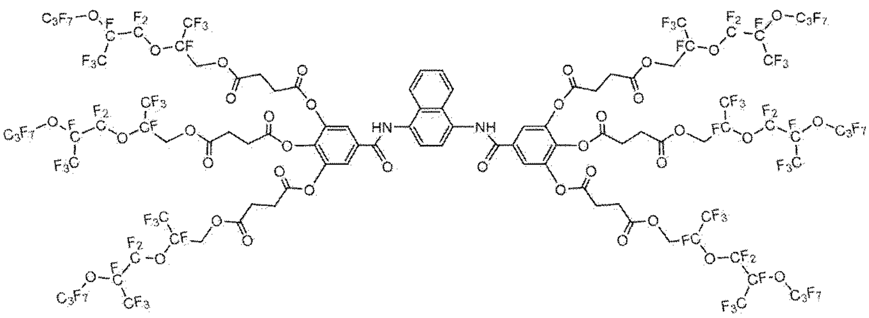

A-1014

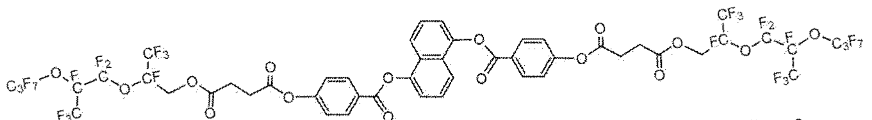

A-1015

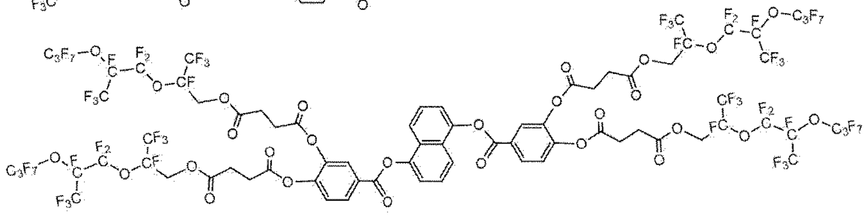

A-1016

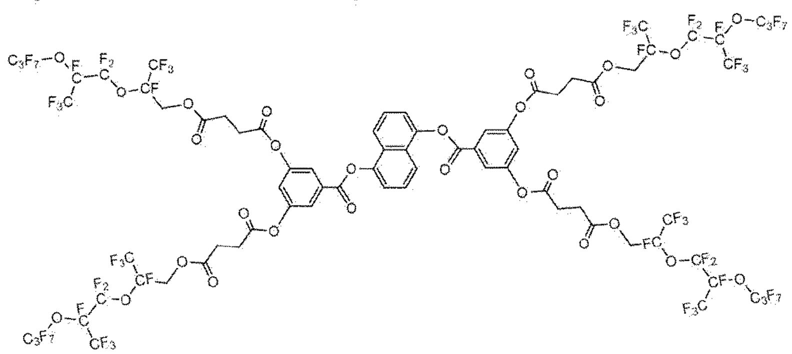

A-1017

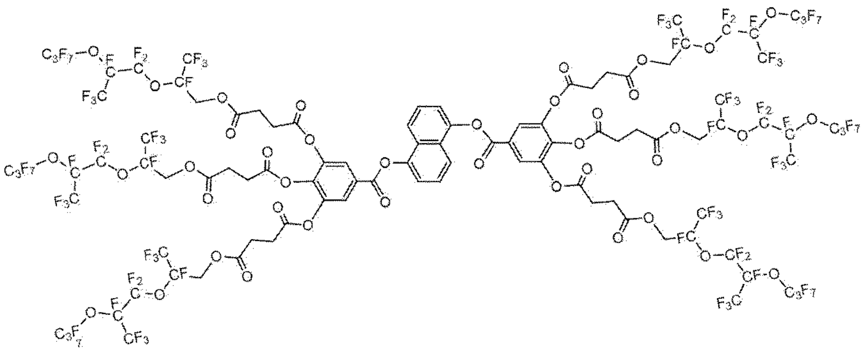

A-1018

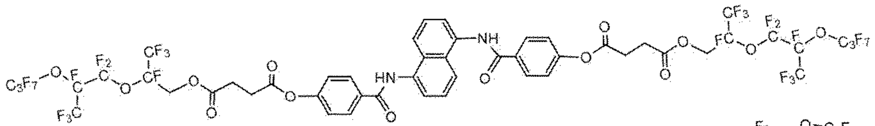

A-1019

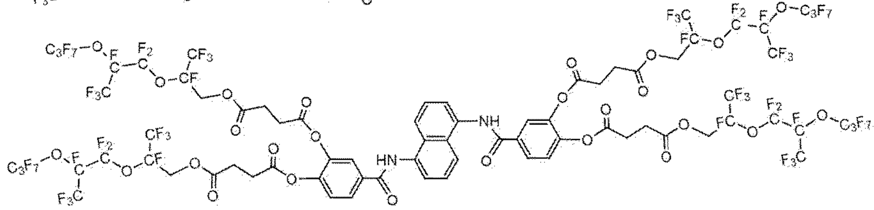

A-1020

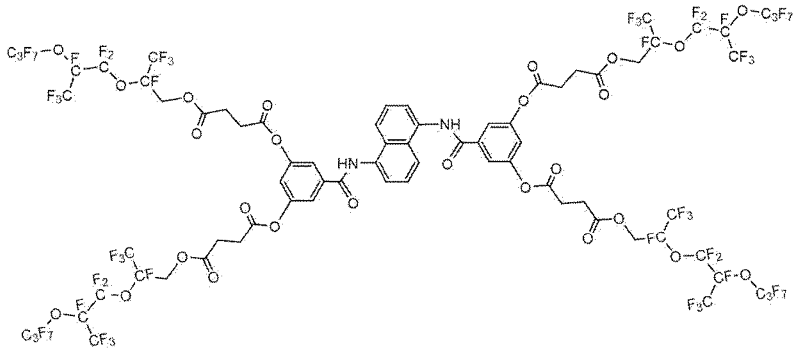

A-1021

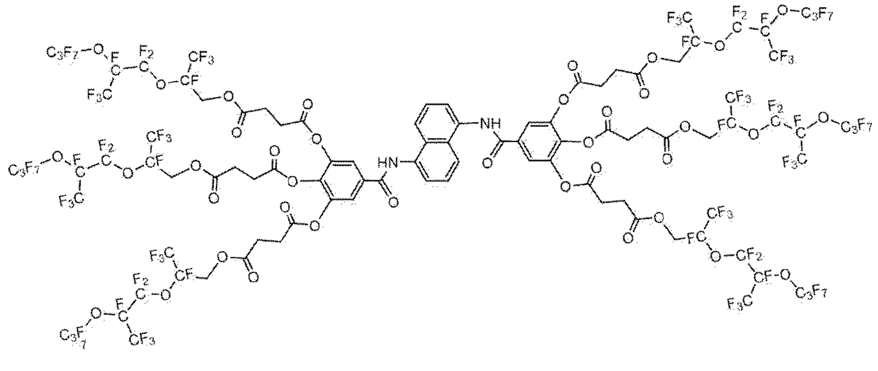

A-1022

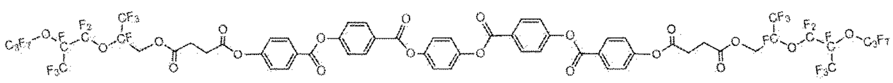

A-1023

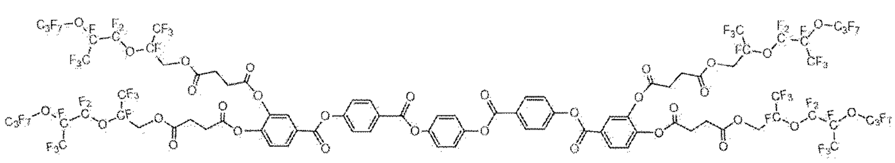

A-1024

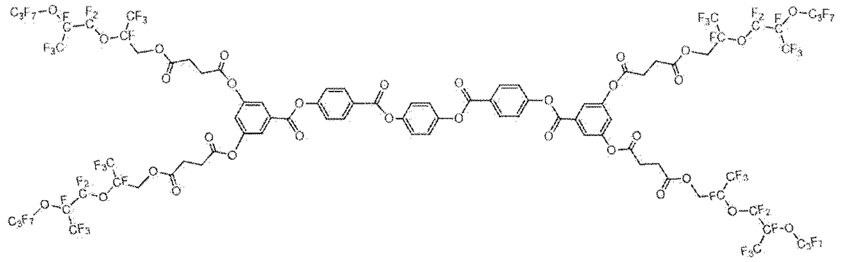

A-1025

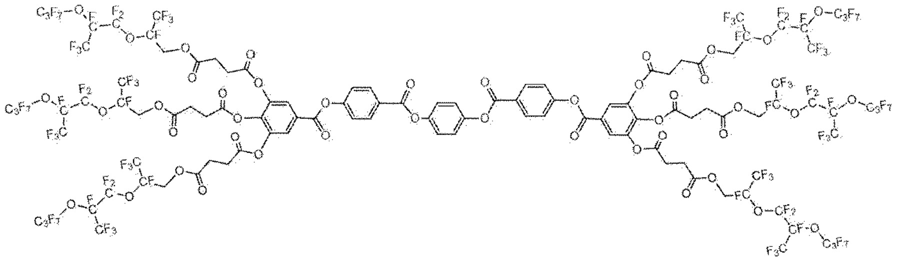

A-1026

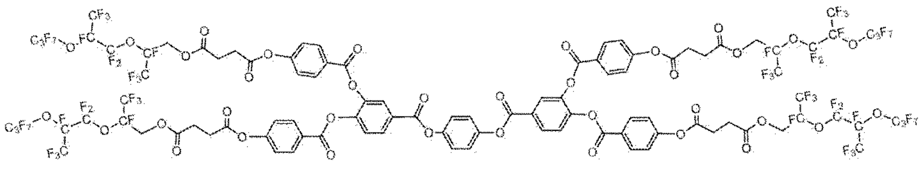

A-1027

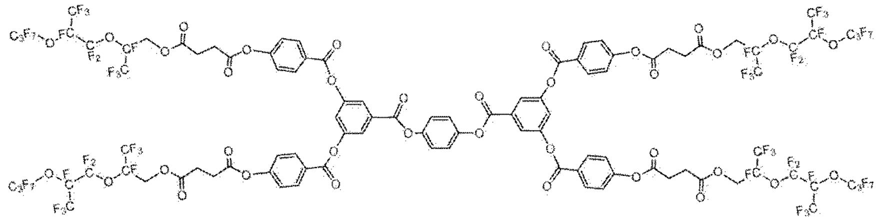

A-1028

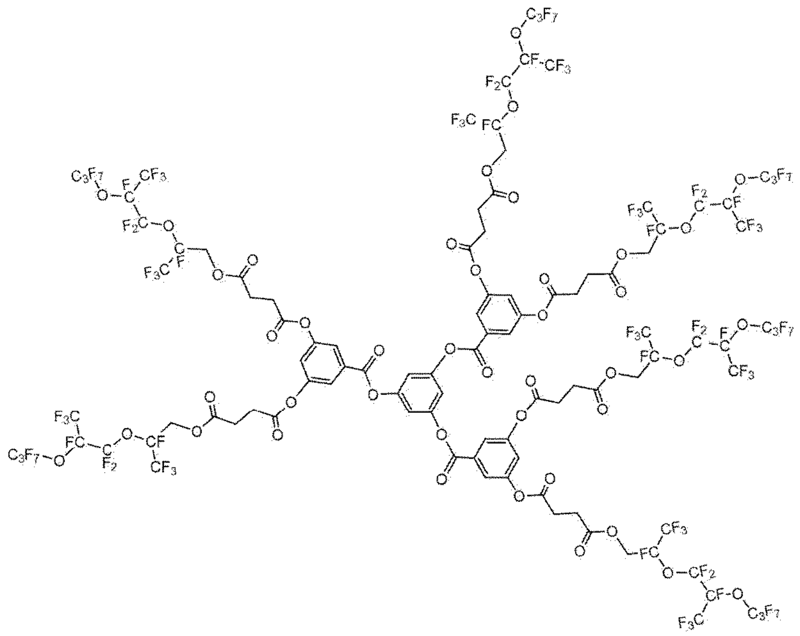

A-1029

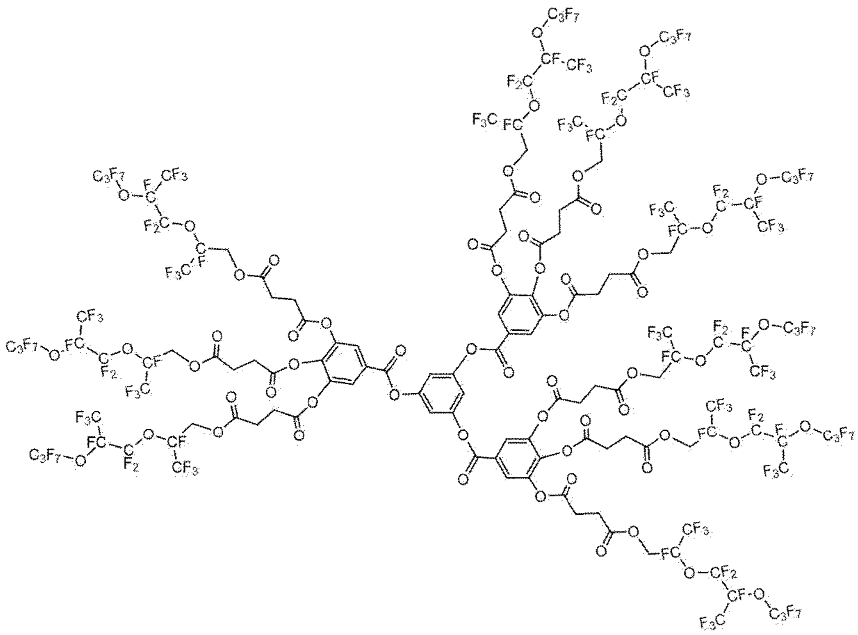

A-1030

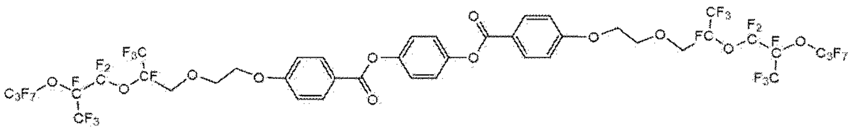

A-1101

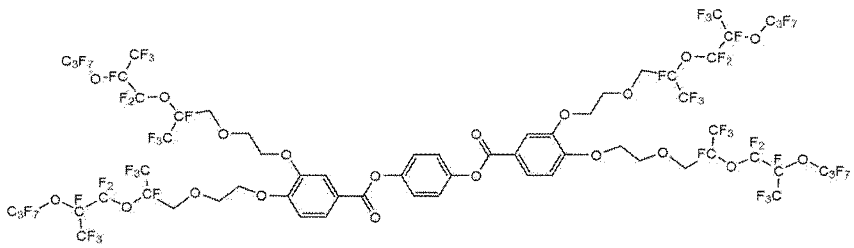

A-1102

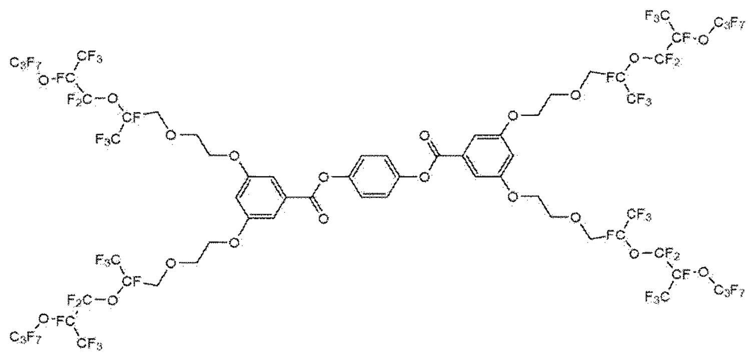

A-1103

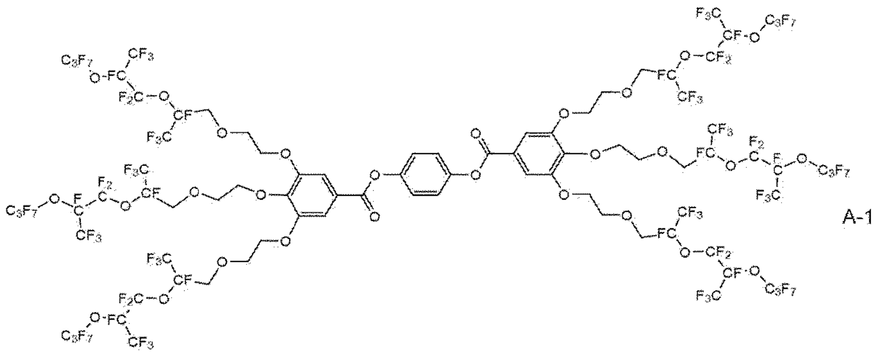

A-1104

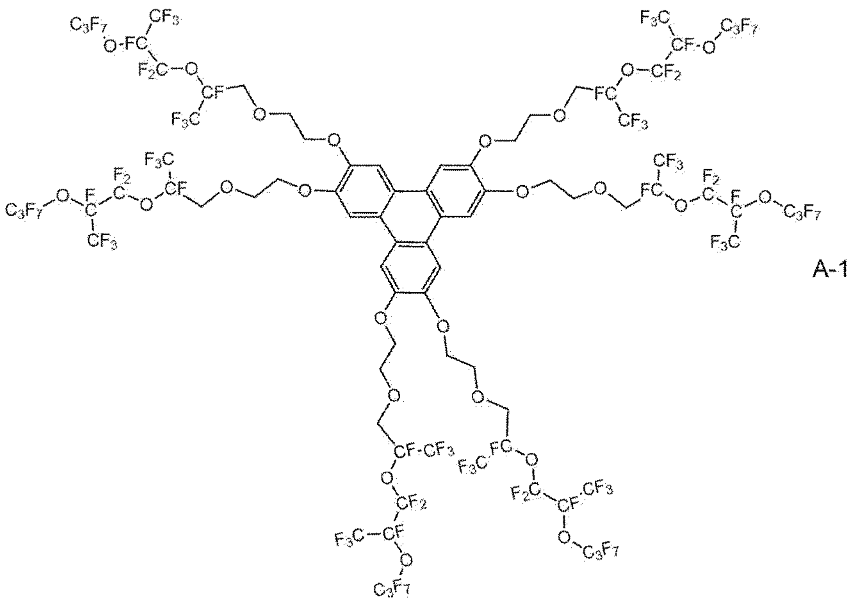

A-1105

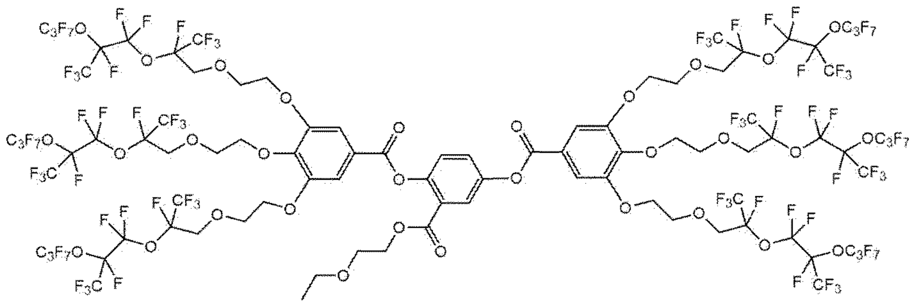

A-1106

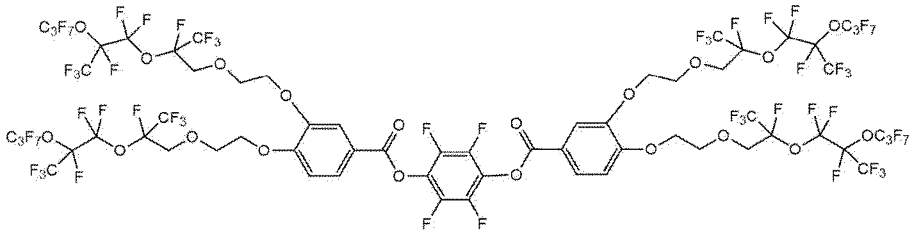

A-1107

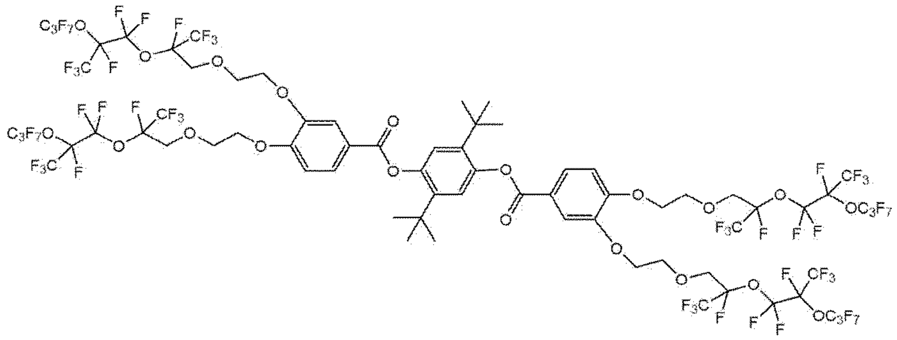

A-1108

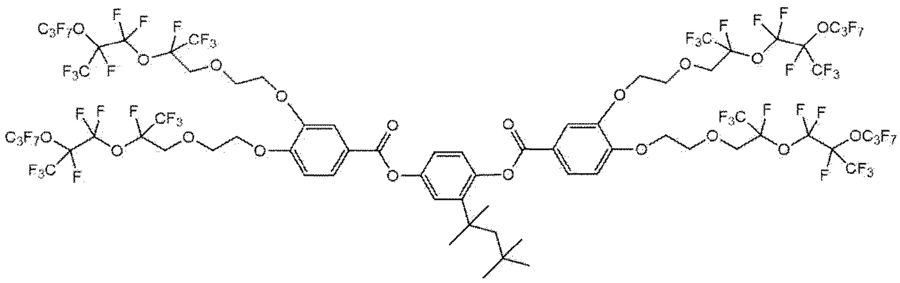

A-1109

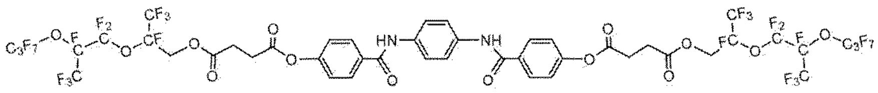

A-1110

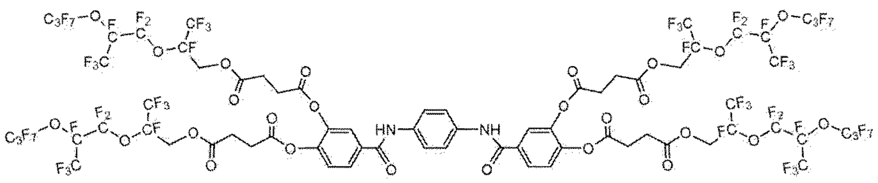

A-1111

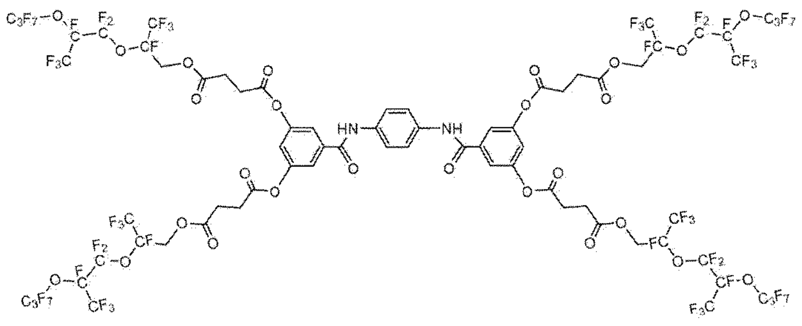

A-1112

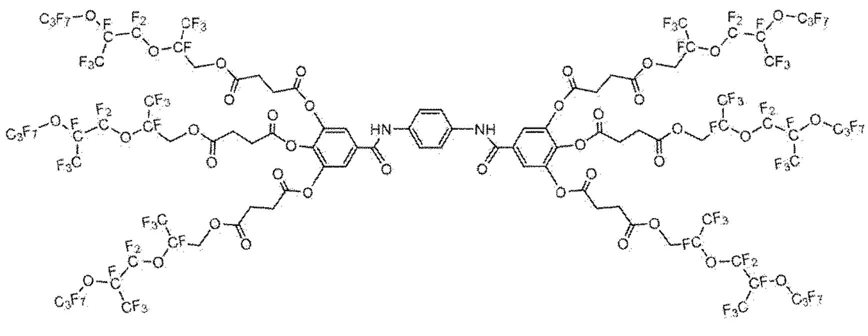

A-1113

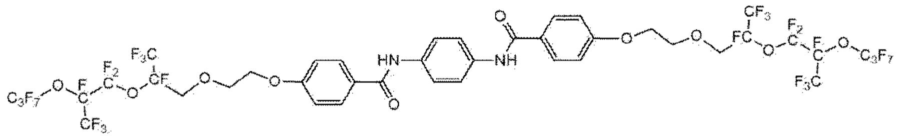
A-1114

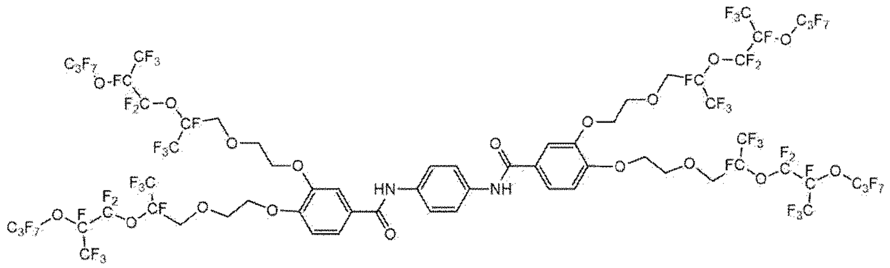
A-1115

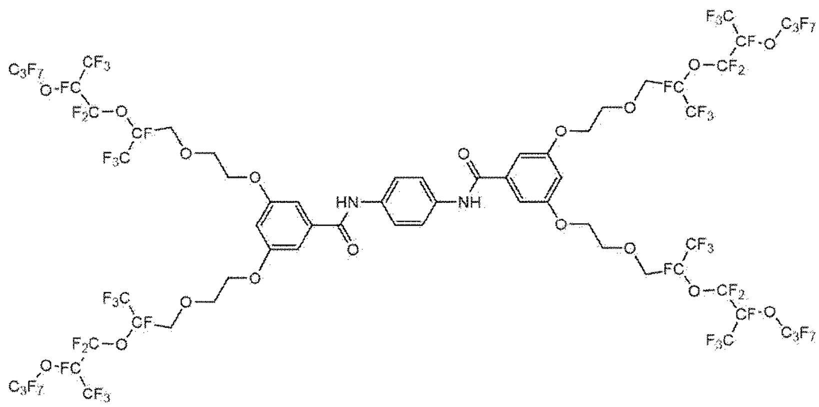
A-1116

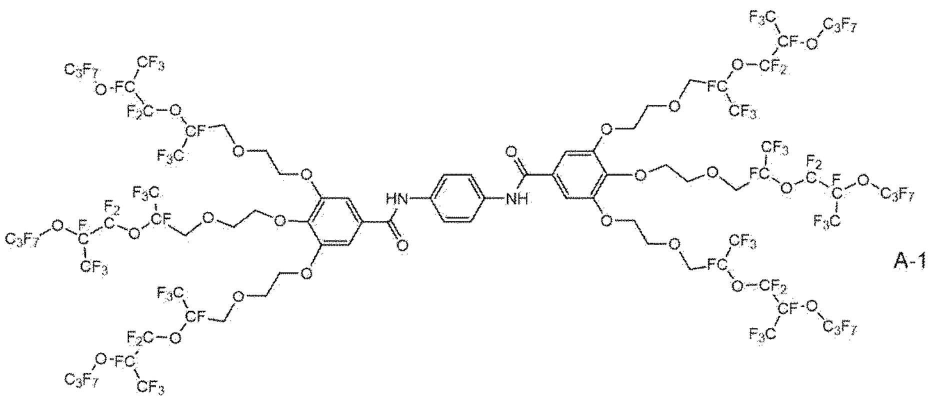
A-1117

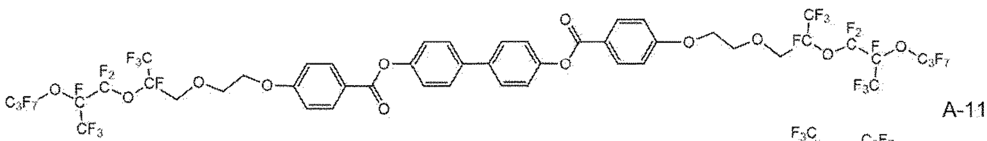
A-1118

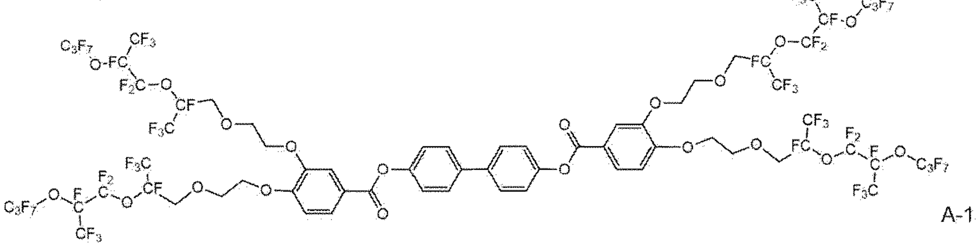
A-1119

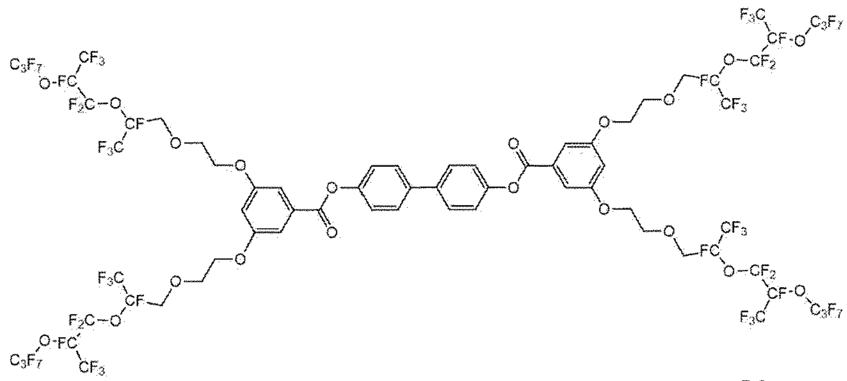

A-1120

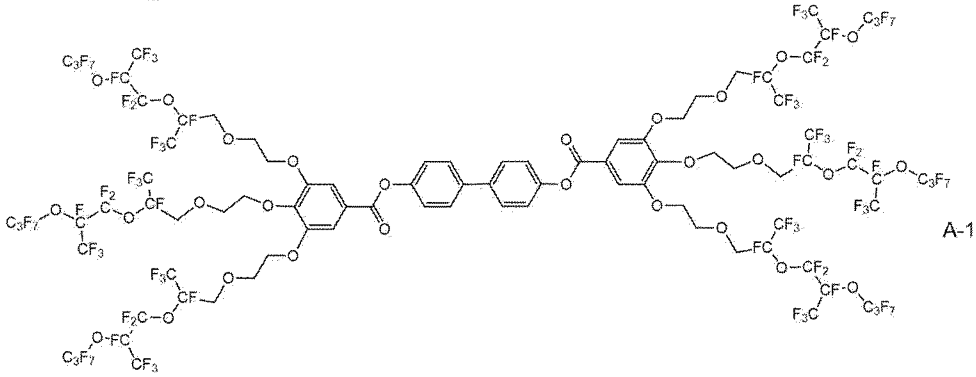

A-1121

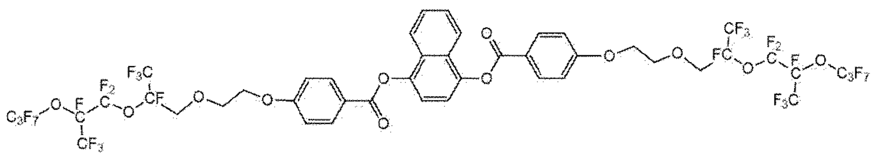

A-1122

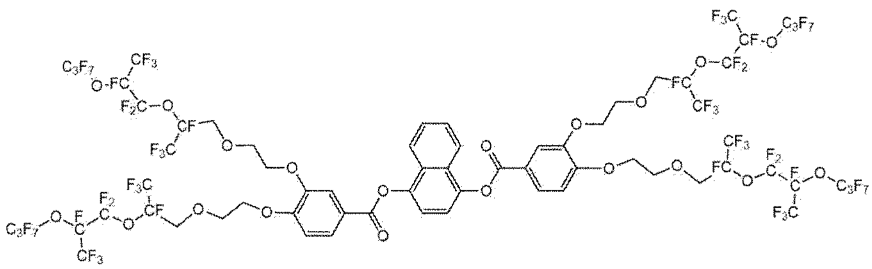

A-1123

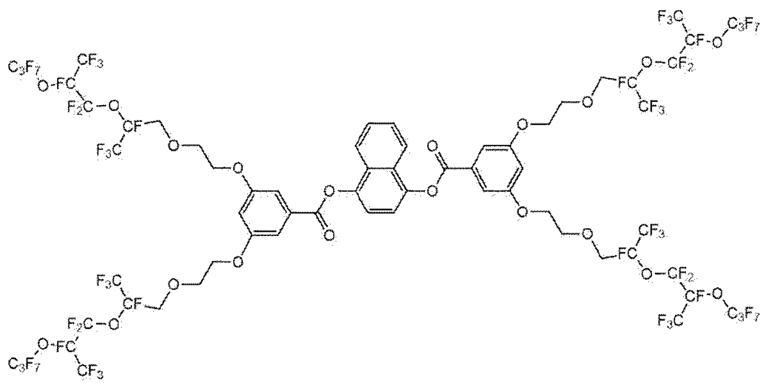

A-1124

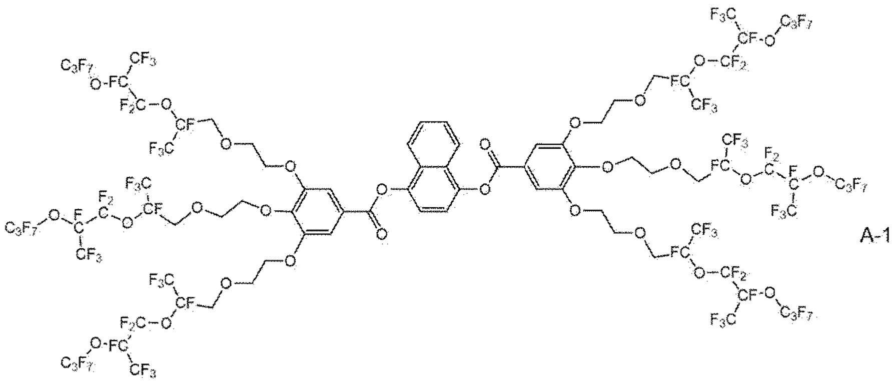
A-1125

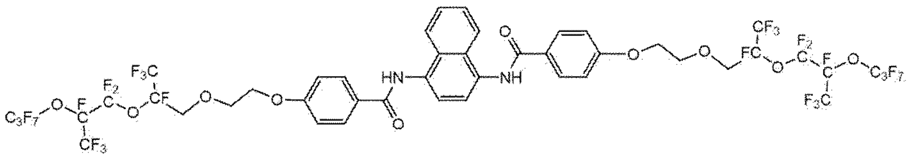
A-1126

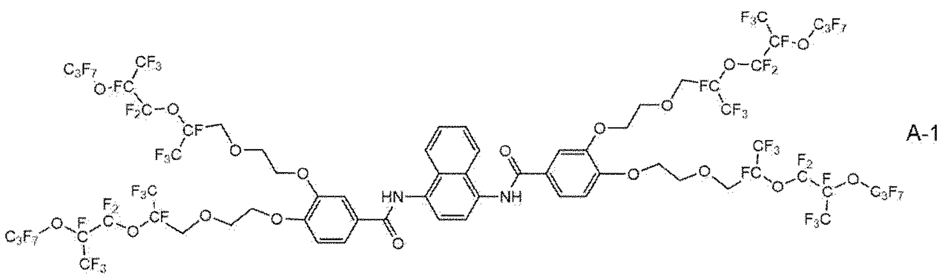
A-1127

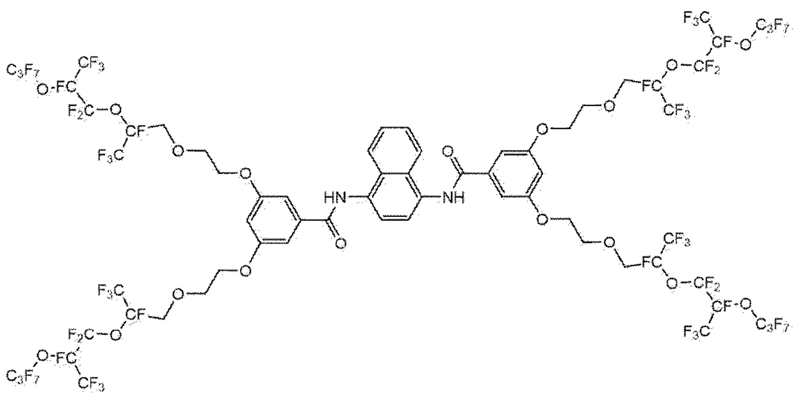
A-1128

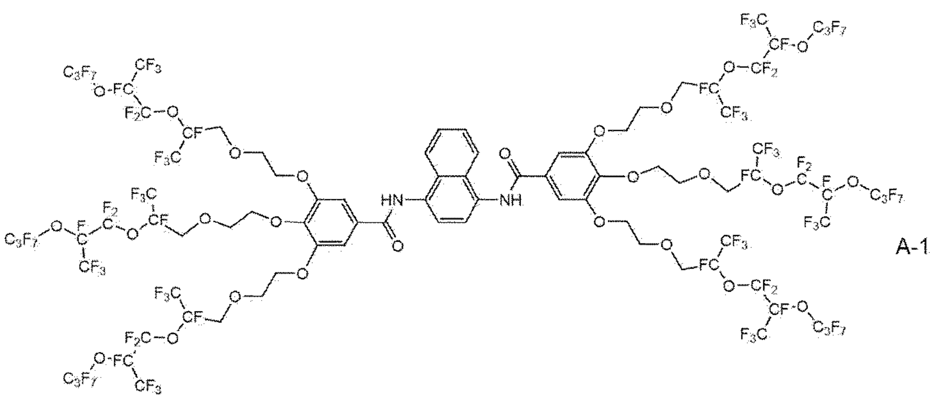
A-1129

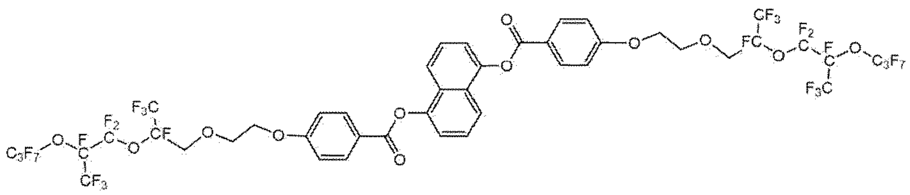

A-1130

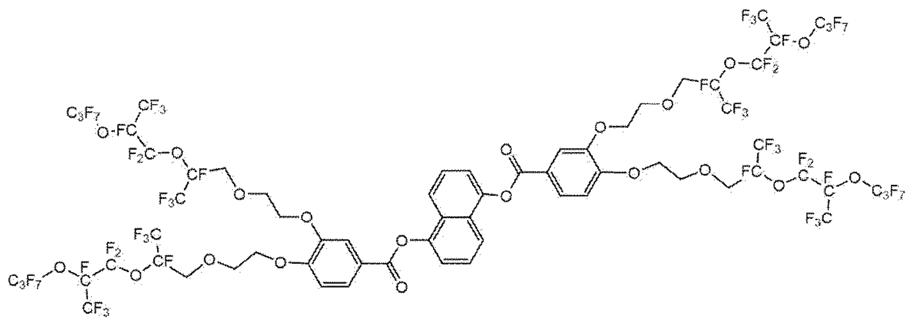

A-1131

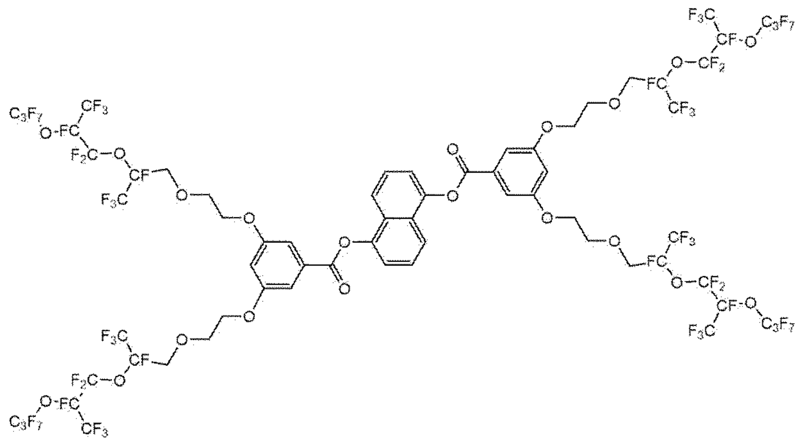

A-1132

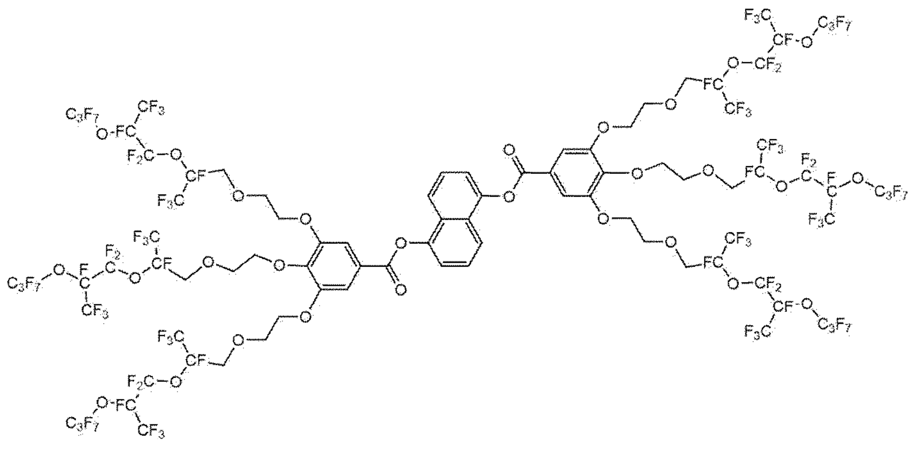

A-1133

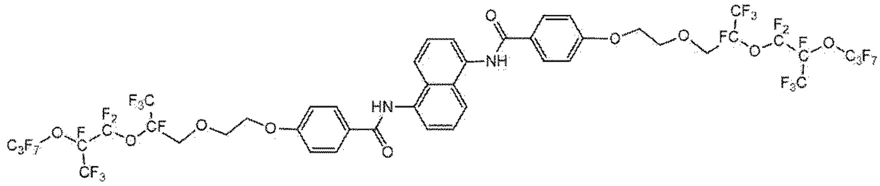

A-1134

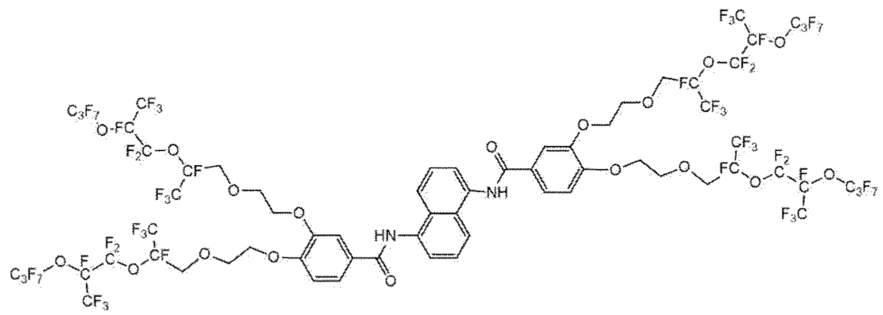
A-1135

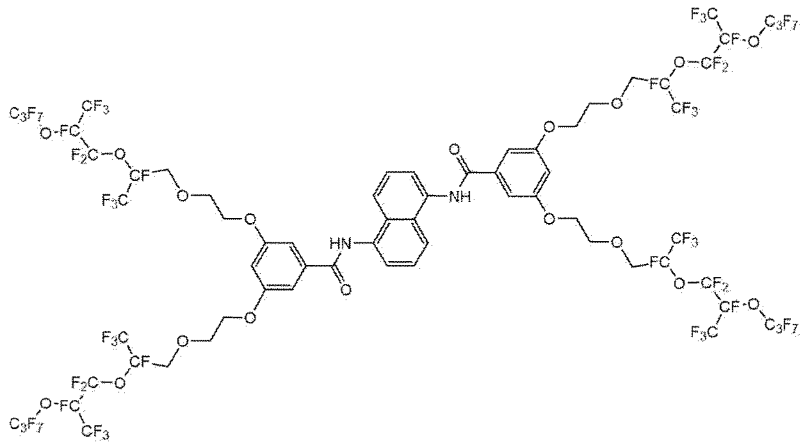
A-1136

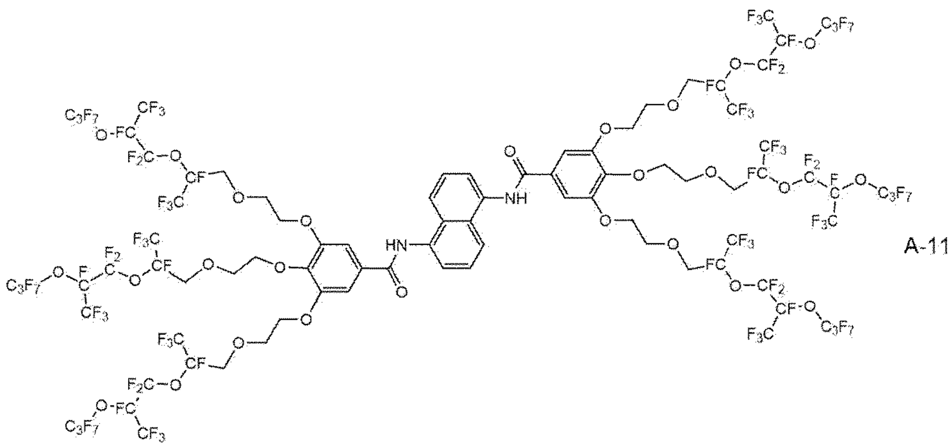
A-1137

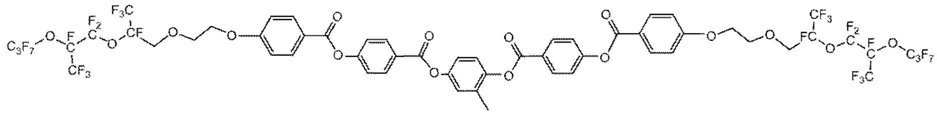
A-1138

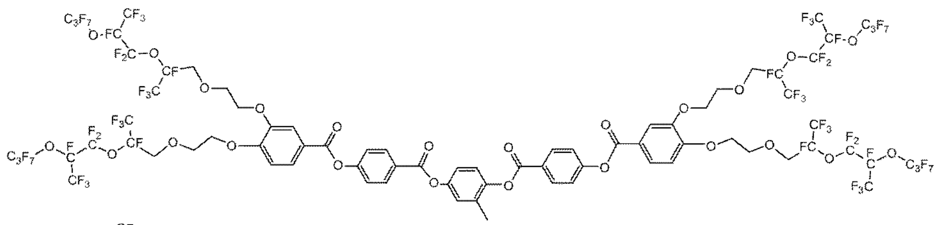

A-1139

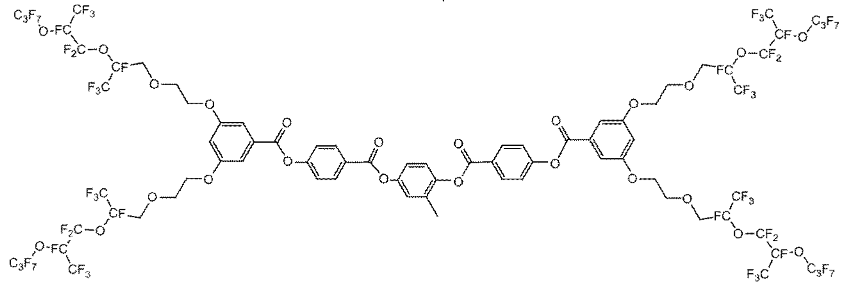

A-1140

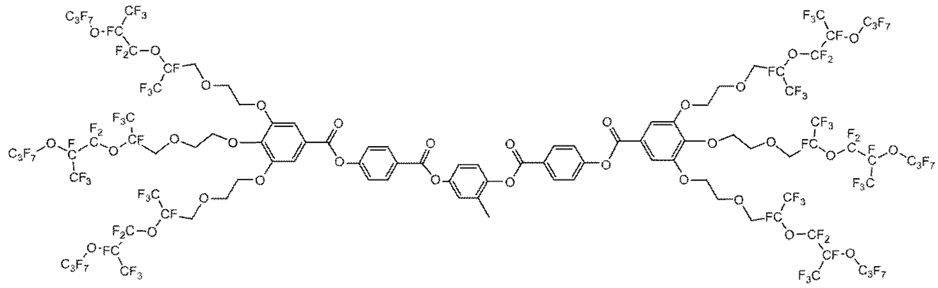

A-1141

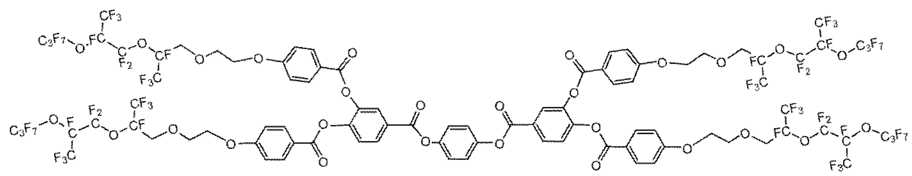

A-1142

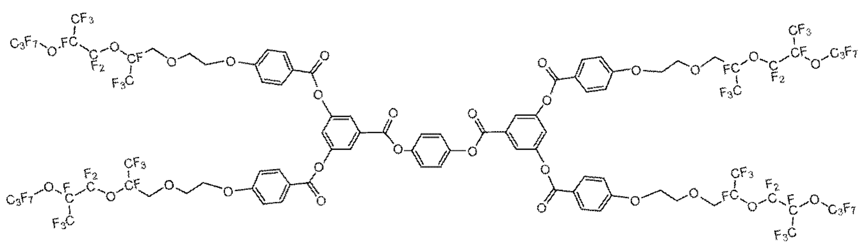

A-1143

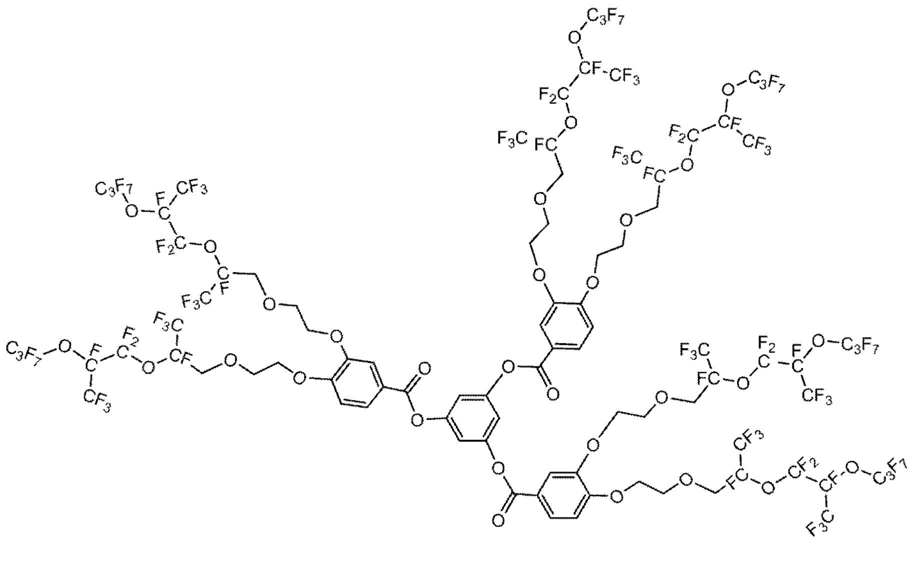

A-1144

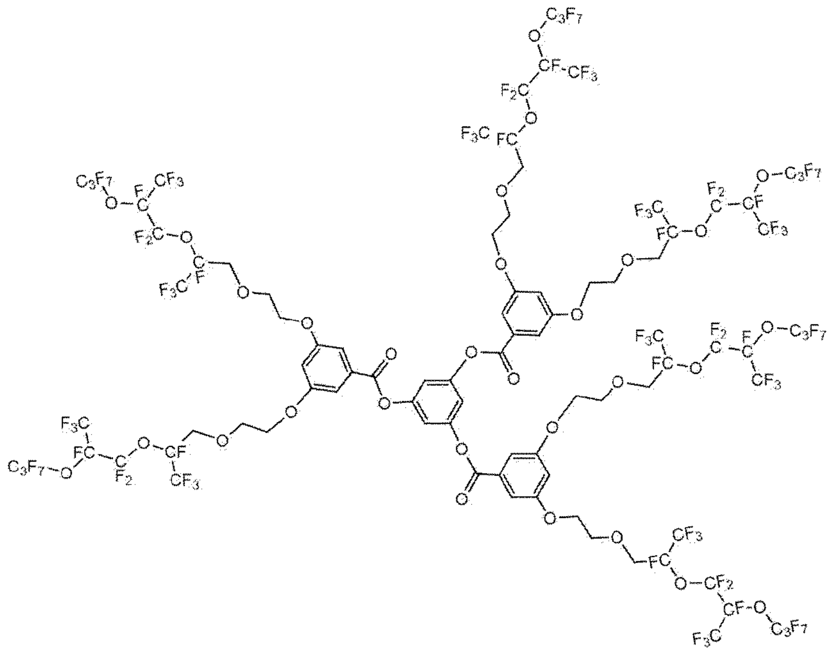

A-1145

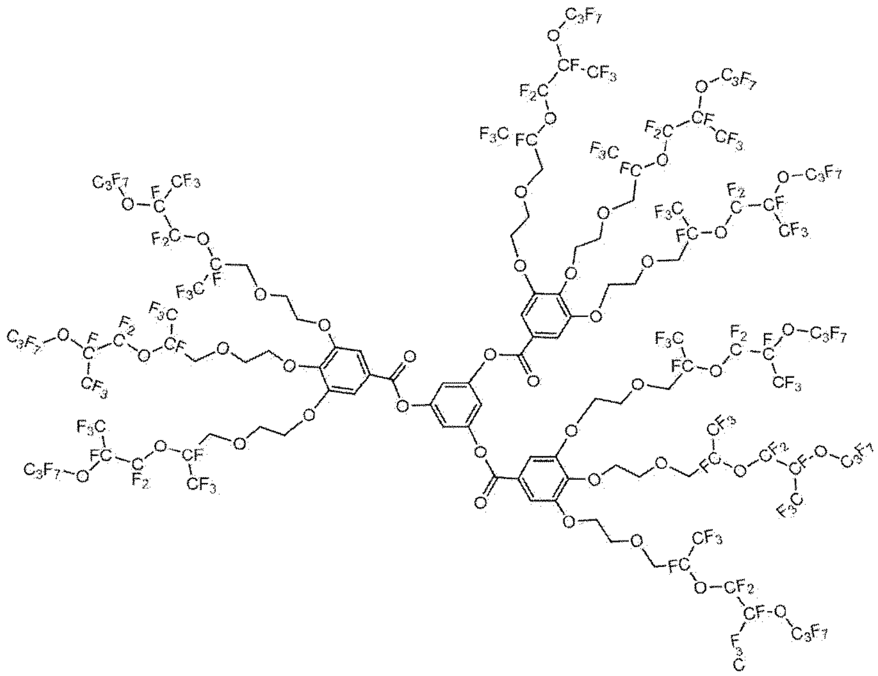

A-1146

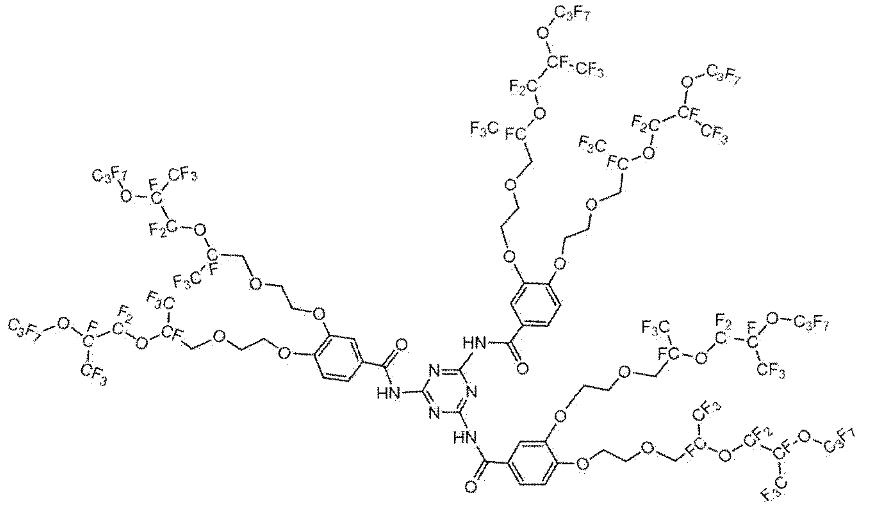

A-1147

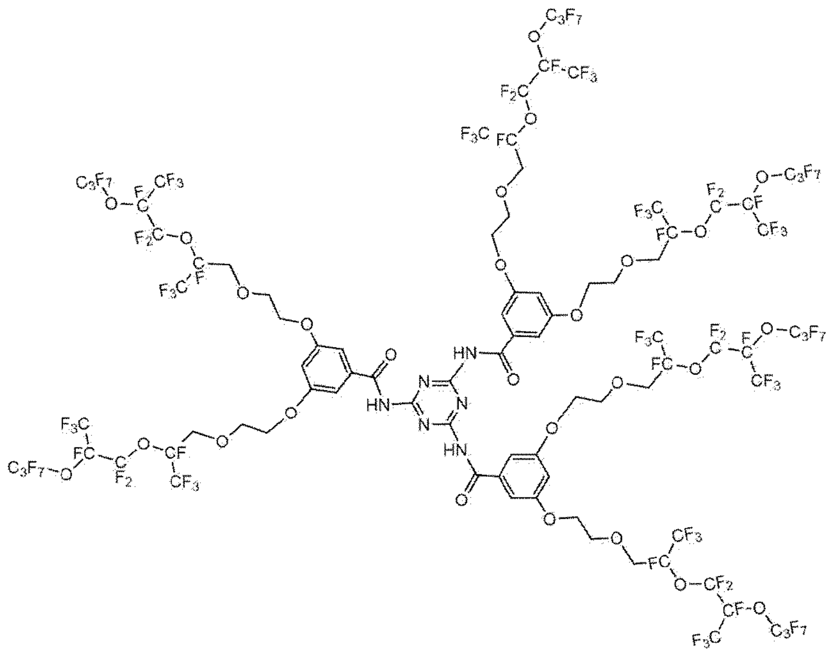

A-1148

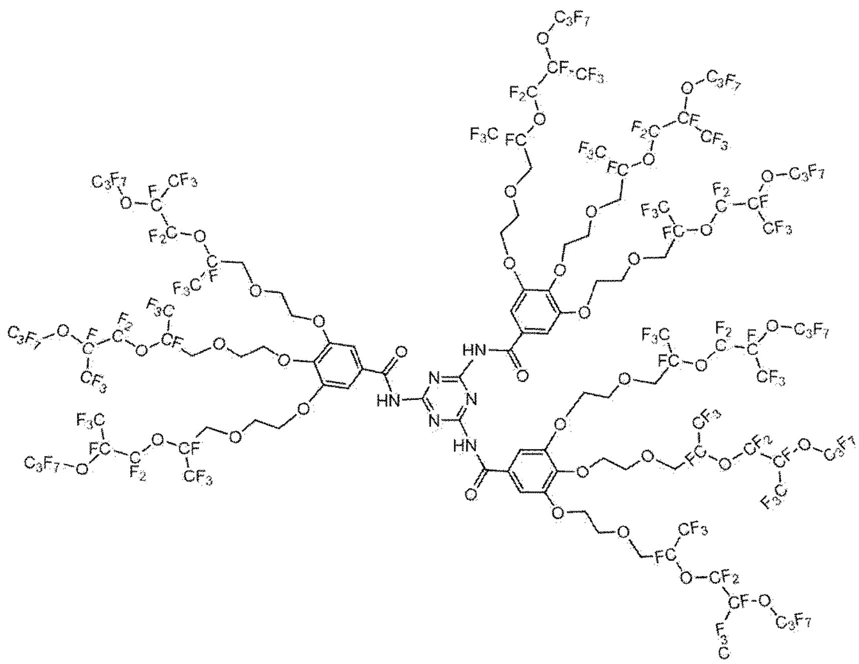

A-1149

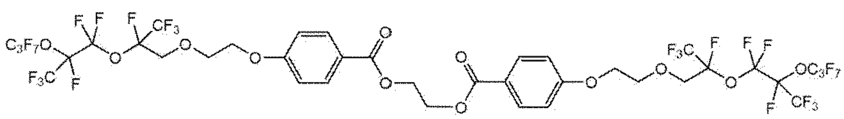

**A-1150**

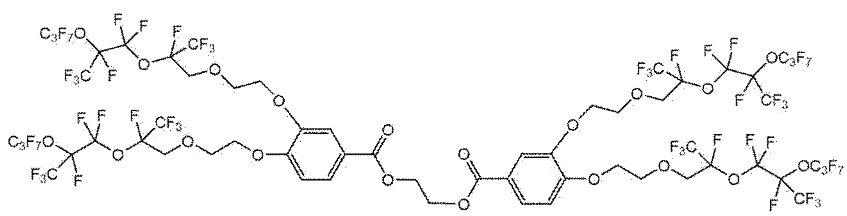

**A-1151**

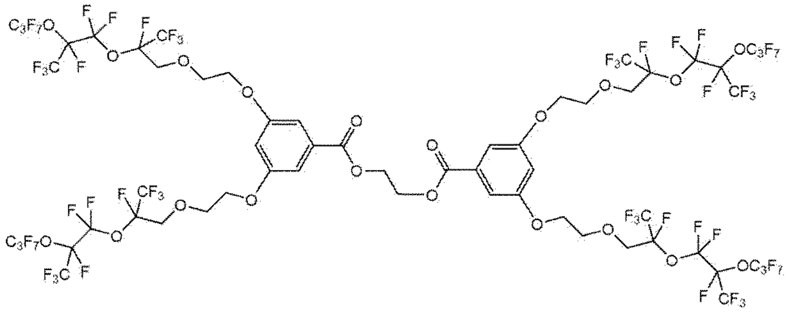

**A-1152**

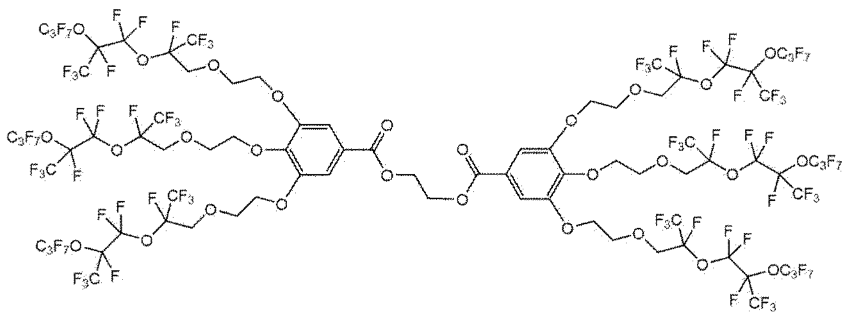

**A-1153**

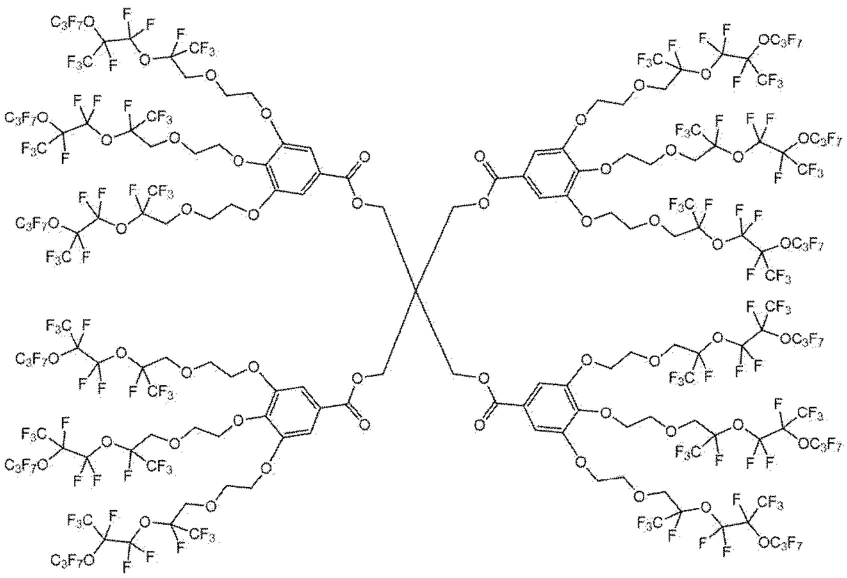

**A-1154**

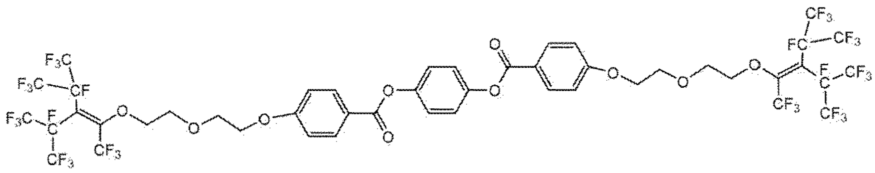

A-1155

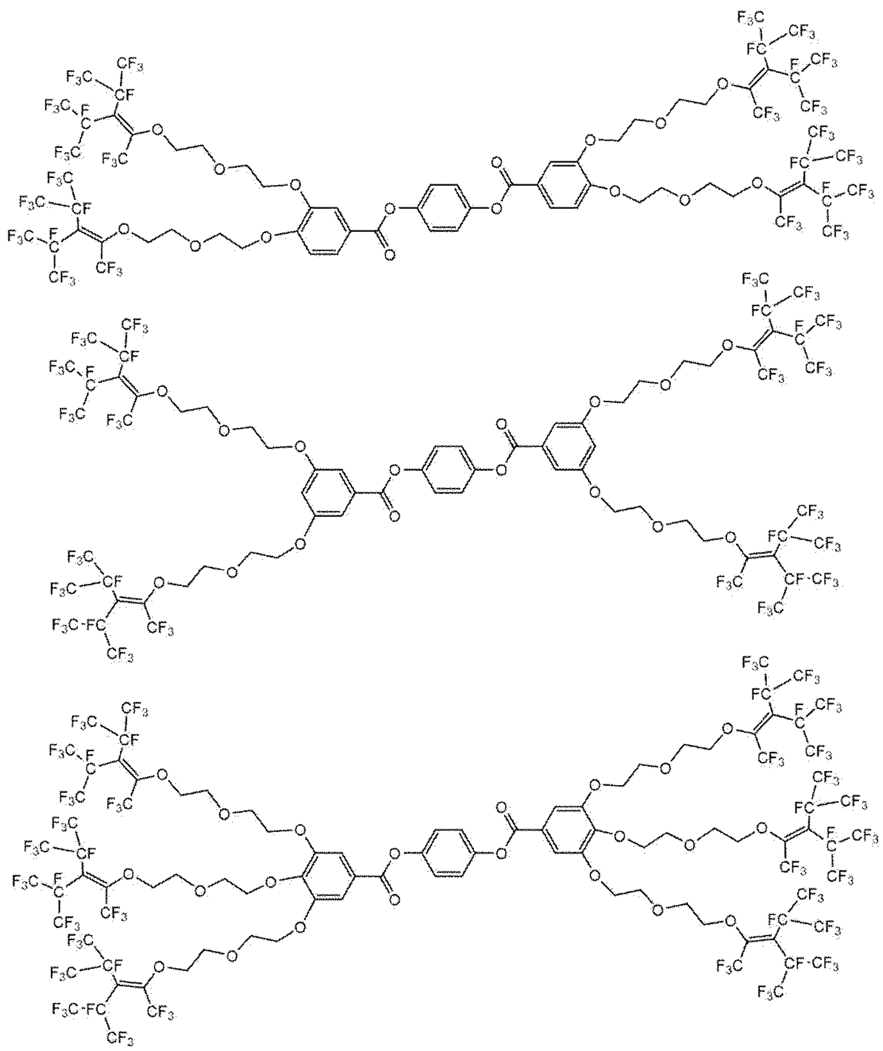

A-1156

A-1157

A-1158

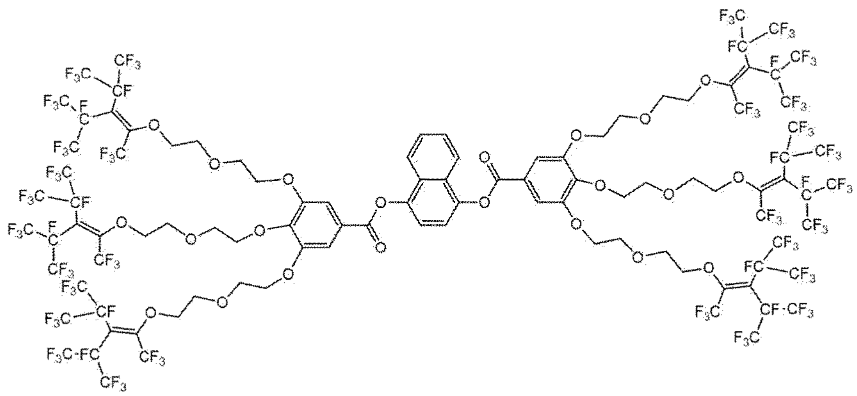

A-1159

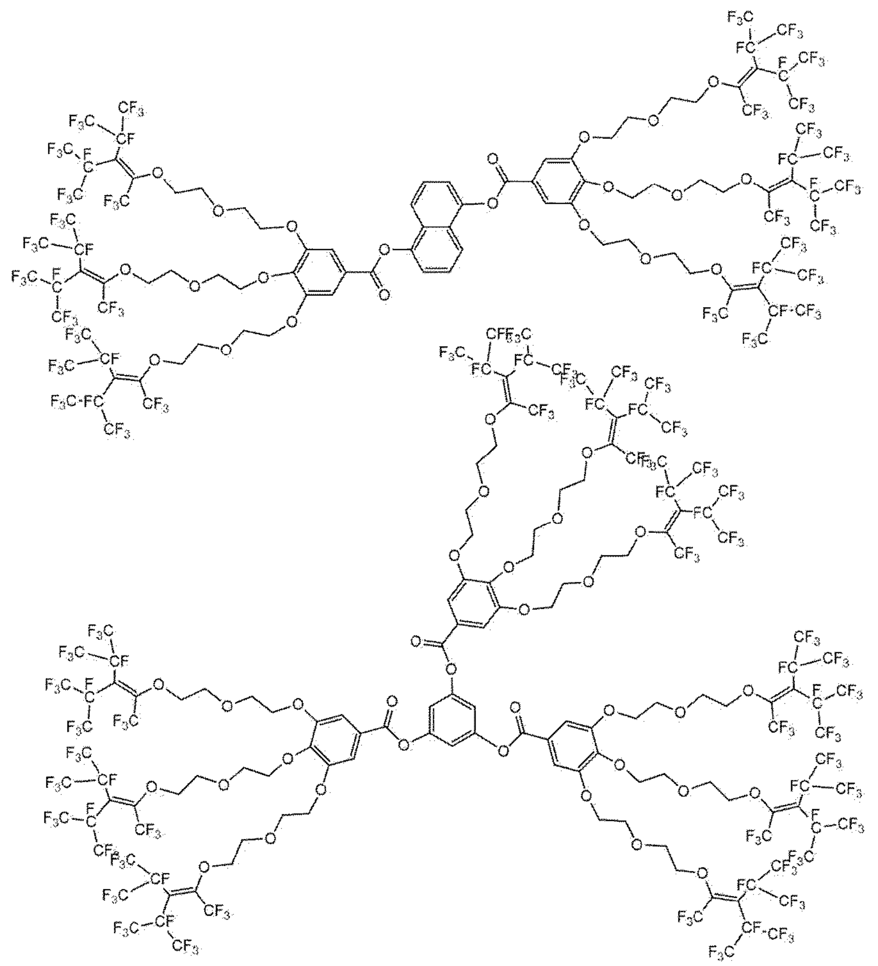

A-1160

A-1161

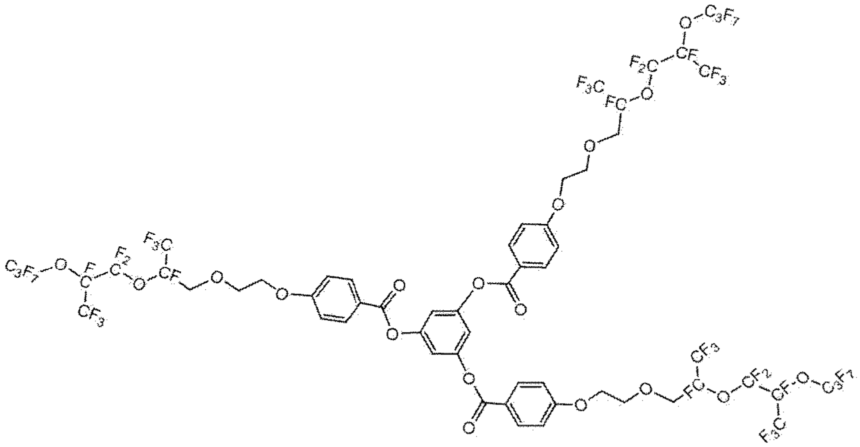

A-1201

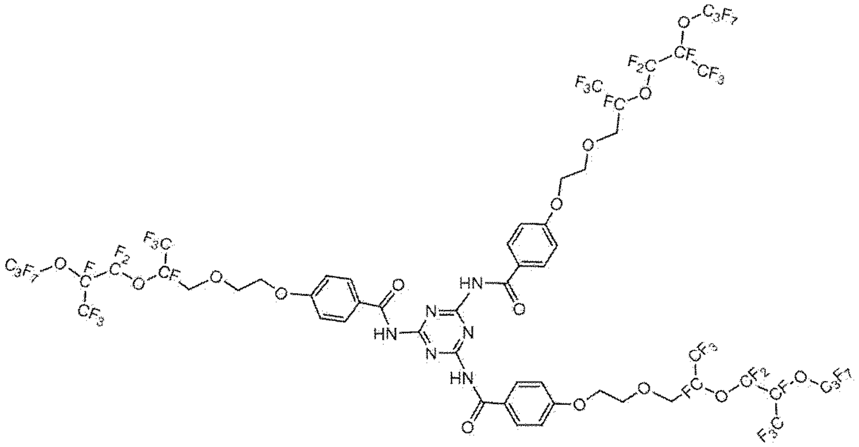

A-1202

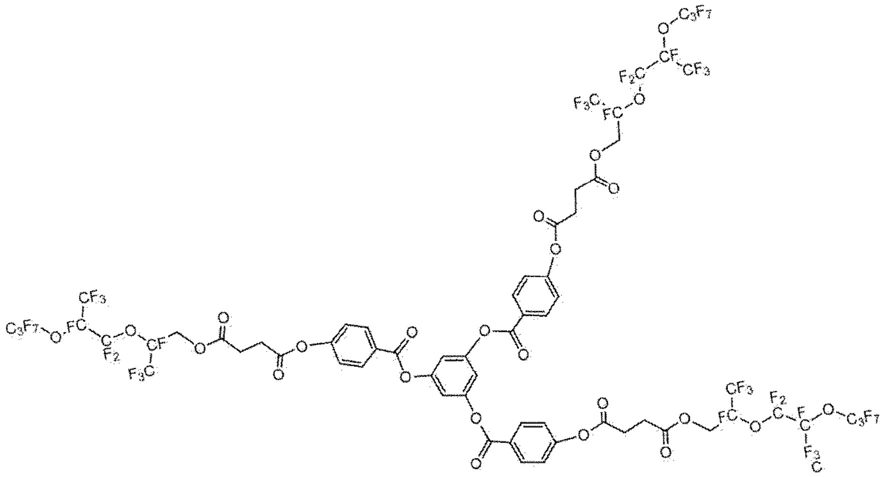

A-1203

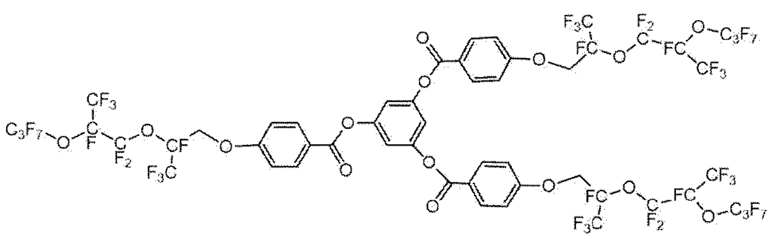

A-1204

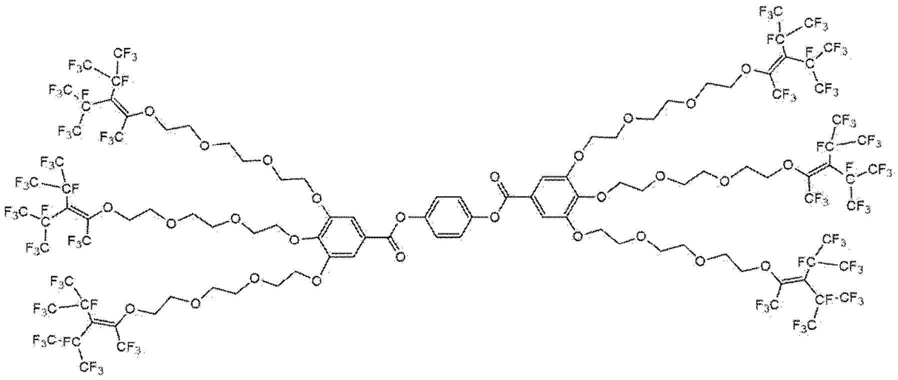

A-1205

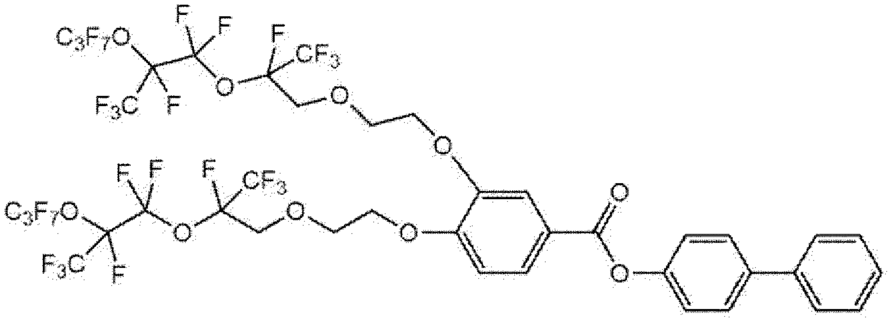

A-1301

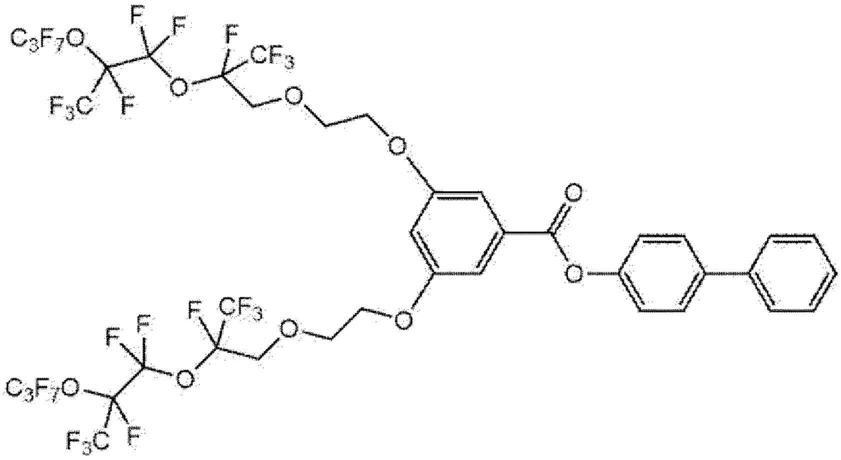

A-1302

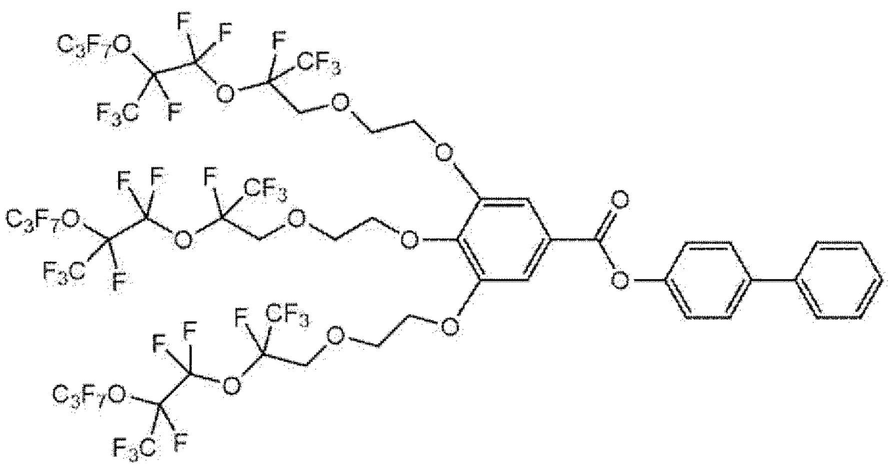

A-1303

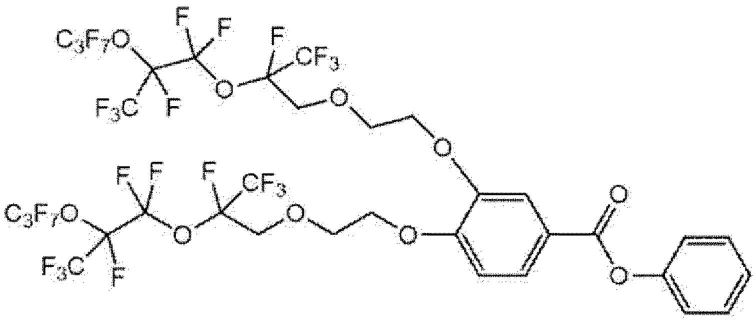

A-1304

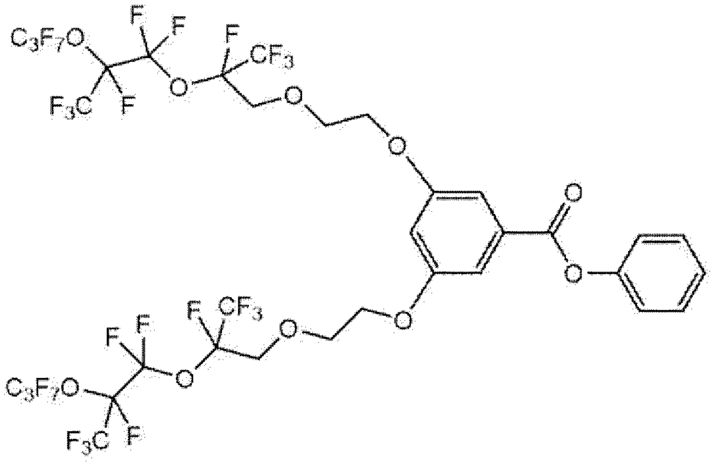

A-1305

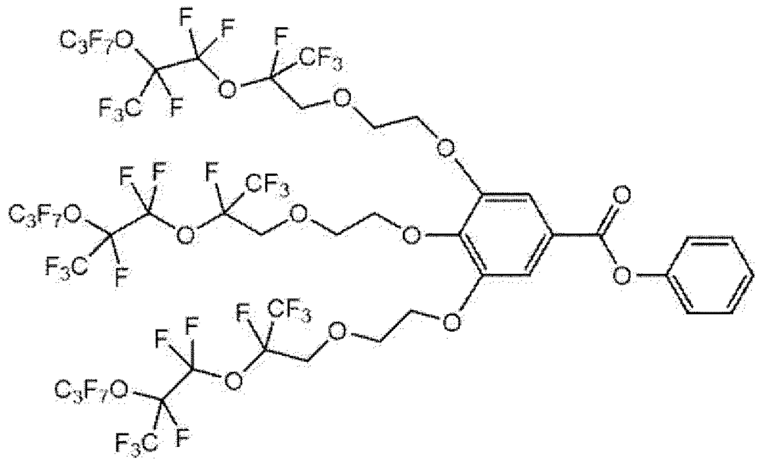

A-1306

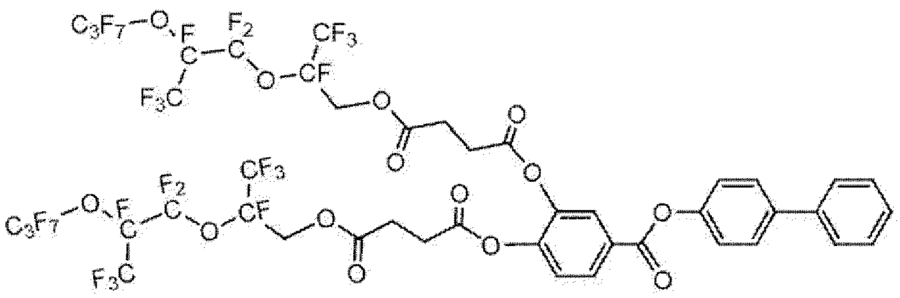

A-1307

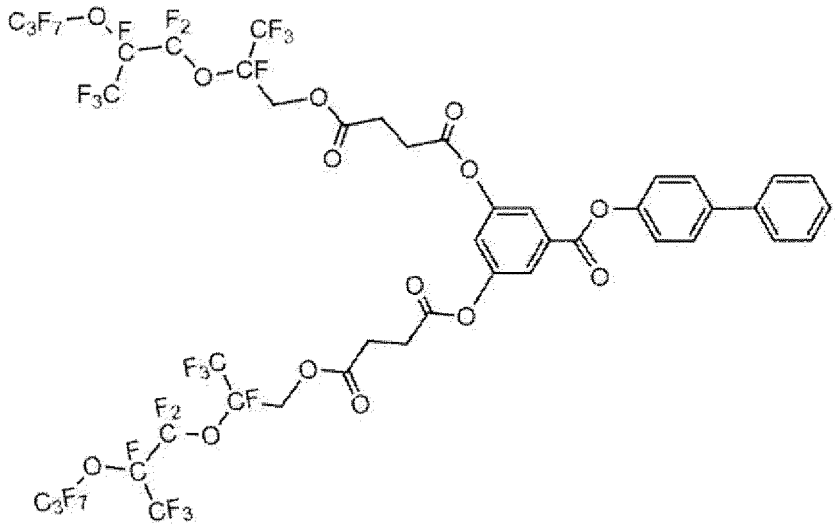

A-1308

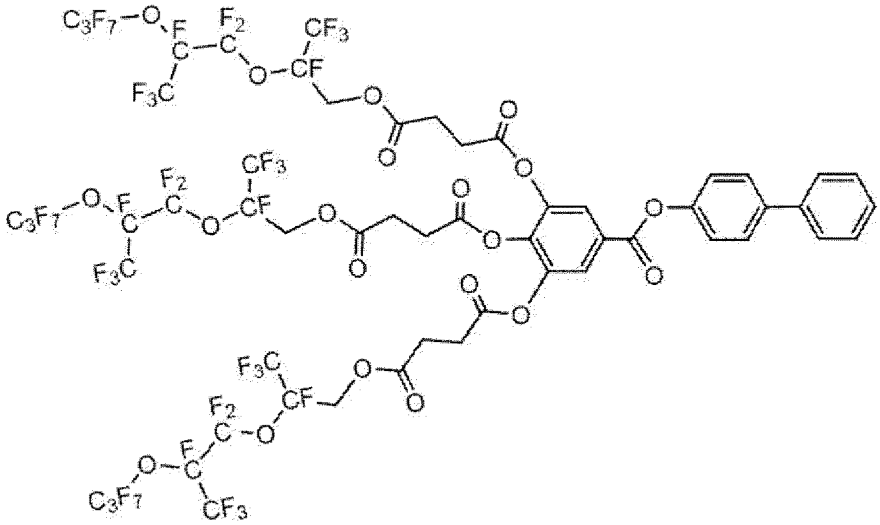

A-1309

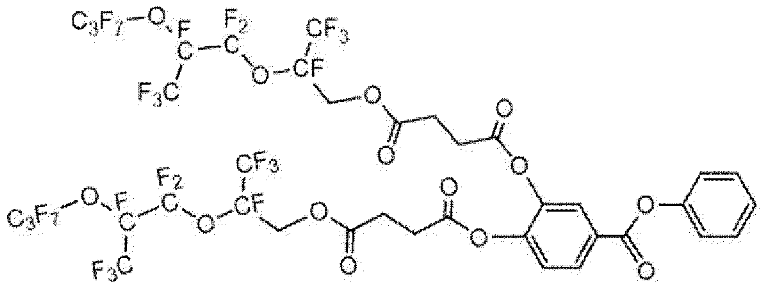

A-1310

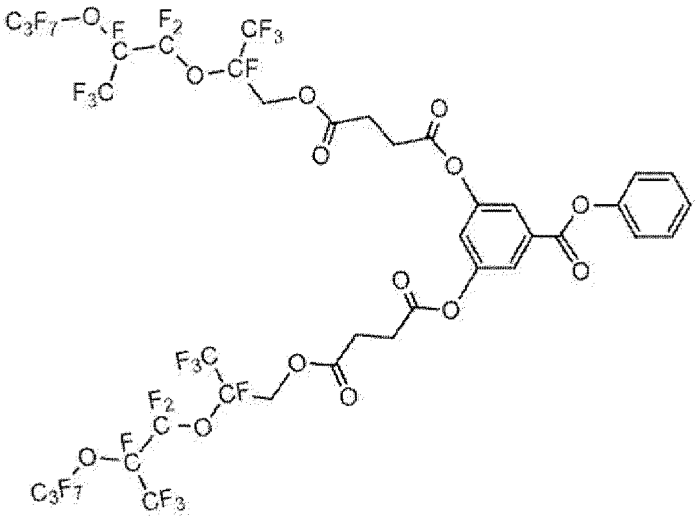

A-1311

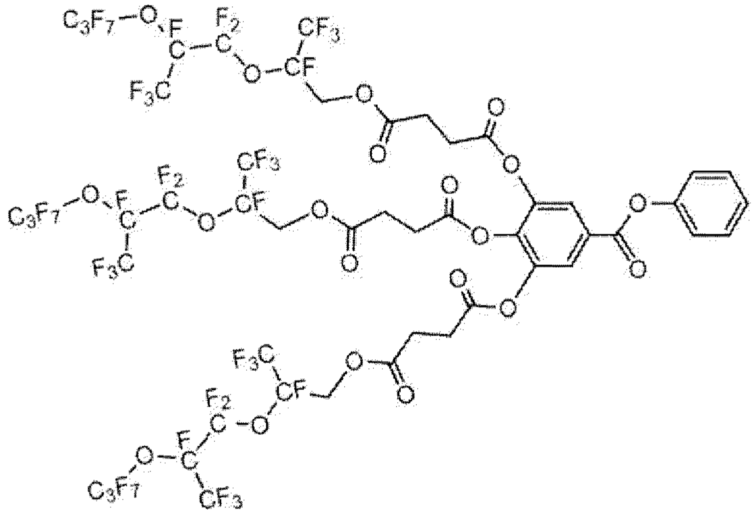

A-1312

The compound according to the embodiment of the present invention can be synthesized by appropriately selecting and combining the synthesis methods described in JP2002-129162A and JP2013-47204A, as well as the documents cited JP2002-129162A and JP2013-47204A. In addition, it can be synthesized by also combining another synthesis method known in the related art as necessary.

[Liquid crystal composition]

The liquid crystal composition according to the embodiment of the present invention is a liquid crystal composition containing the above-described compound according to the embodiment of the present invention and a polymerizable liquid crystal compound.

In the liquid crystal composition according to the embodiment of the present invention, the above-described compound according to the embodiment of the present invention can be used as a liquid crystal alignment accelerator.

In addition, in the liquid crystal composition according to the embodiment of the present invention, one or more kinds of non-polymerizable liquid crystal compounds may be further used in combination.

Further, in the liquid crystal composition according to the embodiment of the present invention, two or more kinds of the above-described compounds the embodiment of the present invention (liquid crystal alignment accelerators) may be used, or a liquid crystal alignment accelerator that does not correspond to the compound according to the embodiment of the present invention may be used in combination.

Here, the content of the compound according to the embodiment of the present invention (the liquid crystal alignment accelerator) contained in the liquid crystal composition according to the embodiment of the present invention is preferably 0.01 to 20 parts by mass and more preferably 0.03 to 2 parts by mass with respect to 100 parts by mass of the polymerizable liquid crystal compound described below.

[Polymerizable liquid crystal compound]

The polymerizable liquid crystal compound is a liquid crystal compound having a polymerizable group.
The kind of the polymerizable liquid crystal compound is not particularly limited.
Generally, liquid crystal compounds can be classified into a rod-like type (a rod-like liquid crystal compound) and a disk-like type (a discotic liquid crystal compound) depending on the shape thereof. Further, the liquid crystal compound can be classified into a low molecular weight type and a high molecular weight type. The high molecular weight generally refers to having a polymerization degree of 100 or more (Polymer Physics-Phase Transition Dynamics, Masao Doi, page 2, Iwanami Shoten, 1992).
In the present invention, any liquid crystal compound can be used. However, it is preferable to use at least one or more kinds of polymerizable rod-like liquid crystal compound or a polymerizable disk-like liquid crystal compound, and it is more preferable to use a polymerizable rod-like liquid crystal compound. Two or more kinds of polymerizable rod-like liquid crystal compounds, two or more kinds of polymerizable disk-like liquid crystal compounds, or a mixture of a polymerizable rod-like liquid crystal compound and a polymerizable disk-like liquid crystal compound may be used.
As the polymerizable disk-like liquid crystal compound, it is possible to preferably use, for example, those described in paragraphs 0161 to 0171 of JP2002-129162A, paragraphs 0020 to 0067 of JP2007-108732A, and paragraphs 0013 to 0108 of JP2010-244038A.
Preferably used examples of the polymerizable rod-like liquid crystal compound include azomethines, azoxys, cyanobiphenyls, cyanophenyl esters, benzoic acid esters, cyclohexanecarboxylic acid phenyl esters, cyanophenyl cyclohexanes, cyano-substituted phenyl pyrimidines, and alkoxy-substituted phenyl pyrimidines, phenyl dioxanes, tolanes, alkenylcyclohexyl benzonitriles.
Specific examples of the polymerizable rod-like liquid crystal compound include those described in Makromol. Chem., Vol. 190, p. 2255 (1989), Advanced Materials, Vol. 5, p. 107 (1993), US4683327A, US5622648A, US5770107A, WO95/22586A, WO95/24455A, WO97/00600A, WO98/23580A, WO98/52905A, JP1989A-272551 (JP-H1-272551A), JP1994-16616A (JP-H6-16616A), JP1995-110469A (JP-H7-110469A), JP1999-80081A (JP-H11-80081A), JP1999-513019A (JP-H11-513019A), JP2001-328973A, JP2005-289980A, JP2014-198815A, JP2014-198814A, and JP2014-198814A. Two or more kinds of polymerizable liquid crystal compounds may be used in combination. In a case of using two or more kinds of polymerizable liquid crystal compounds in combination, it is possible to decrease the alignment temperature.
The content of the polymerizable liquid crystal compound in the liquid crystal composition according to the embodiment of the present invention is not particularly limited; however, it is preferably 50% by mass or more and more preferably 70% by mass or more with respect to the total mass of the total solid content in the liquid crystal composition. The upper limit thereof is not particularly limited; however, it is 95% by mass or less in a large number of cases.
It is noted that the solid content means components capable of forming an optically anisotropic layer, from which a solvent has been removed, and even the components having properties of liquid shall be regarded as the solid content.
In addition to the polymerizable liquid crystal compound and the liquid crystal alignment accelerator, the liquid crystal composition according to the embodiment of the present invention can contain a solvent, a chiral agent and a polymerizable initiator described below, or another additive (for example, a cellulose ester) as necessary.

[Solvent]

The liquid crystal composition of the embodiment of the present invention may contain a solvent.
As the solvent, an organic solvent is preferably used.
Examples of the organic solvent include an amide (for example, N,N-dimethylformamide), a sulfoxide (for example, dimethyl sulfoxide), a hydrocarbon (for example, toluene, hexane, or the like), an alkyl halide (for example, chloroform, dichloromethane, or the like), an ester (for example, methyl acetate, butyl acetate, ethyl propionate, or the like), a ketone (for example, acetone, methyl ethyl ketone, cyclohexanone, methyl isobutyl ketone, cyclopentanone, or the like), and an ether (for example, tetrahydrofuran, 1,2-dimethoxyethane, or the like).
Among these organic solvents, an ester or a ketone is preferable.
In addition, two or more kinds of organic solvents may be used in combination as the solvent.

[Chiral agent]

The liquid crystal composition according to the embodiment of the present invention may contain a chiral agent.
The chiral agent has a function of inducing a helical structure of the cholesteric liquid crystalline phase.
In the chiral agent, the twisted direction of the induced helix or the helix pitch is different depending on the compound structure thereof, and thus the chiral agent may be selected depending on the intended purpose.

The chiral agent is not particularly limited, and known compounds (for example, those described in Handbook of Liquid Crystal Device, Chapter 3, Section 4-3, page 199, Chiral Agent for TN and STN, edited by the 142th Committee of the Society, Japan Society for the Promotion of Science, 1989), isosorbide derivatives, isomannide derivatives, binaphthol derivatives, and the like can be used.

The chiral agent generally contains an asymmetric carbon atom; however, an axially asymmetric compound or a planarly asymmetric compound, which does not contain an asymmetric carbon atom, can also be used as the chiral agent.

Examples of the axially asymmetric compound or planarly asymmetric compound include binaphthyl, helicene, paracyclophane, and derivatives thereof.

The chiral agent may have a polymerizable group. In a case where both the chiral agent and the liquid crystal compound have a polymerizable group, it is possible to form a polymer having the repeating unit derived from the polymerizable liquid crystal compound and the repeating unit derived from the chiral agent by a polymerization reaction between the polymerizable chiral agent and the polymerizable liquid crystal compound. In this aspect, it is preferable that the kind of the polymerizable group contained in the polymerizable chiral agent is the same as that of the polymerizable group contained in the polymerizable liquid crystal compound. As a result, the polymerizable group of the chiral agent is also preferably an unsaturated polymerizable group, an epoxy group, or an aziridinyl group, more preferably an unsaturated polymerizable group, and particularly preferably an ethylenically unsaturated polymerizable group.

Further, the chiral agent may be a liquid crystal compound.

In a case where the chiral agent is a photoisomerizable group, it is possible to form a desired reflection wavelength pattern corresponding to the luminescence wavelength by photomask irradiation with an actinic ray ray or the like after coating and alignment, which is preferable. The photoisomerizable group is preferably an isomerization moiety of a compound exhibiting photochromic properties, an azobenzene moiety, a cinnamoyl moiety, an $\alpha$ -cyanocinnamoyl moiety, a stilbene moiety, or a chalcone moiety. As the specific compound, it is possible to use the compound described in JP2002-80478A, JP2002-80851A, JP2002-179668A, JP2002-179669A, JP2002-179670A, JP2002-179681A, JP2002-179682A, JP2002-338575A, JP2002-338668A, JP2003-313189A, or JP2003-313292A.

Specific examples of the chiral agent include a compound represented by Formula (12). Here, X in Formula (12) is 2 to 5 (an integer).

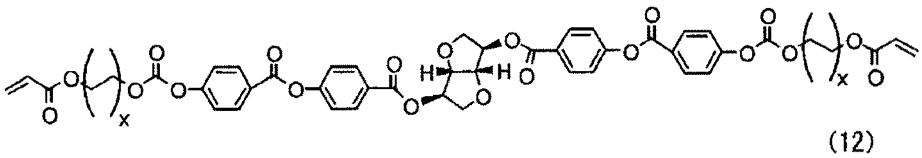

(12)

In a case where the liquid crystal composition according to the embodiment of the present invention contains a chiral agent, the content of the chiral agent is preferably 0.01% by mole to 200% by mole and more preferably 1% by mole to 30% by mole of the amount of the polymerizable liquid crystal compound.

[Polymerization initiator]

The liquid crystal composition according to the embodiment of the present invention may contain a polymerization initiator.

The polymerization reaction suitable for the present invention is a thermal polymerization reaction using a thermal polymerization initiator and a photopolymerization reaction using a photopolymerization initiator, where a photopolymerization reaction is more preferable.

Examples of the photopolymerization initiator include $\alpha$-carbonyl compounds (described in the specifications of US2367661A and US2367670A), an acyloin ether (described in the specification of US2448828A), an aromatic acyloin compound substituted with $\alpha$-hydrocarbon (described in the specification of US2722512A), polynuclear quinone compounds (described in the specifications of US3046127A and US2951758A), a combination of a triaryl imidazole dimer and a p-aminophenyl ketone (described in the specification of US3549367A), acridine and a phenazine compound (described in the specifications of JP1985-105667A (JP-S60-105667A) and US4239850A), an oxadiazole compound (described in the specification of US4212970A), acylphosphine oxide compounds (described in JP1988-40799B (JP-S63-40799B), JP1993-29234B (JP-H5-29234B), JP1998-95788B (JP-H10-95788B), and JP1998-29997B (JP-H10-29997B)), and oxime ester compounds (for example, OXE-01 and OXE-02 manufactured by Omni Inc. and NCI-1919 manufactured by ADEKA CORPORATION).

In a case where the liquid crystal composition according to the embodiment of the present invention contains a polymerization initiator, the content of the polymerization initiator is preferably 0.01% to 20% by mass and more preferably 0.4% to 8% by mass with respect to the total mass of the solid content of the liquid crystal composition.

[Cured substance]

The cured substance the embodiment of the present invention is a cured substance obtained by polymerizing the above-described liquid crystal composition according to the embodiment of the present invention.
The cured substance the embodiment of the present invention can be used as an optically anisotropic layer.
This optically anisotropic layer is a layer formed by aligning a polymerizable liquid crystal compound (a polymerizable rod-like liquid crystal compound or a polymerizable disk-like liquid crystal compound) and then immobilizing it by polymerization. The polymerizable liquid crystal compound is no longer necessary to exhibit liquid crystallinity after forming the layer.

[Film]

The film according to the embodiment of the present invention is a film containing at least one kind of the above-described cured substance the embodiment of the present invention.
In addition, the film according to the embodiment of the present invention is a film obtained by immobilizing a cholesteric liquid crystalline phase of the above-described liquid crystal composition according to the embodiment of the present invention.
The film according to the embodiment of the present invention can be formed by forming a film of the liquid crystal composition according to the embodiment of the present invention by a method such as coating. It is also possible to produce an optically anisotropic element by applying a liquid crystal composition onto an alignment film described below and forming a liquid crystal layer.
The coating with the liquid crystal composition can be carried out by a known method (for example, an extrusion coating method, a direct gravure coating method, a reverse gravure coating method, a die-coating method, or a bar coating method).
It is preferable that the liquid crystalline molecule is immobilized so that the alignment state is maintained.
In addition, it is preferable that the immobilization is carried out by a polymerization reaction of the polymerizable group introduced into the liquid crystalline molecule.
In the method of polymerizing the liquid crystal composition according to the embodiment of the present invention, photopolymerization by irradiating the above-described photopolymerization initiator with light is preferable, and it is preferable to use ultraviolet light as a light source. The irradiation energy is preferably 5 $mJ/cm^2$ to 100 $J/cm^2$, more preferably 30 to 600 $mJ/cm^2$, and still more preferably 100 to 400 $mJ/cm^2$. In order to promote the photopolymerization reaction, the irradiation with light may be carried out under heating conditions. The thickness of the liquid crystal layer is preferably 0.1 to 50 $\mu$m, more preferably 0.3 to 20 $\mu$m, and most preferably 1 to 10 $\mu$m.
The coating amount of the compound according to the embodiment of the present invention, contained in the liquid crystal composition (that is, the coating film) that forms the liquid crystal layer, is preferably 0.005 to 0.5 $g/m^2$, preferably 0.01 to 0.45 $g/m^2$, still more preferably 0.02 to 0.4 $g/m^2$, and most preferably 0.03 to 0.35 $g/m^2$.

[Alignment film]

The alignment film can be provided by means of a rubbing treatment of an organic compound (preferably a polymer), oblique vapor deposition of an inorganic compound, formation of a layer having microgrooves, or accumulation of an organic compound (for example, $\omega$-tricosanoic acid, dioctadecylmethylammonium chloride or methyl stearate) by the Langmuir-Blogette method (the LB film), or the like.
Furthermore, there has been also known as an alignment film having an orientation function that is imparted by applying an electrical field, applying a magnetic field, or by irradiation with light. Among these, a photo-alignment film in which an orientation function is generated by irradiation with light is preferable.
The rubbing treatment is carried out by rubbing the surface of the polymer layer with paper or cloth several times in a constant direction.
The kind of polymer to be used in the alignment film is determined depending on the alignment (particularly, the average tilt angle) of the liquid crystalline molecules. In order to align the liquid crystalline molecules horizontally (average tilt angle: 0° to 50°), a polymer that does not reduce the surface energy of the alignment film (a general polymer for an alignment film) is used. In order to align the liquid crystalline molecules vertically (average tilt angle: 50° to 90°), a polymer that reduces the surface energy of the alignment film is used. In order to reduce the surface energy of the alignment film, it is preferable to introduce a hydrocarbon group having 10 to 100 carbon atoms into the side chain of the polymer.
The specific kind of polymer is described in the document on an optical compensation sheet using a liquid crystalline molecule, which is compatible with various display modes.
The thickness of the alignment film is preferably 0.01 to 5 $\mu$m and more preferably 0.05 to 1 $\mu$m. It is noted

that the liquid crystal layer may be transferred onto a transparent support after aligning the liquid crystalline molecules of the optically anisotropic layer by using the alignment film. The liquid crystalline molecules immobilized in the aligned state can maintain the aligned state even in the absence of the alignment film.

Further, in a case of the alignment in which the average tilt angle is less than 5°, it is not necessary to carry out the rubbing treatment, and the alignment film is not required either. However, for the intended purpose of improving the adhesiveness between the liquid crystalline molecule and the transparent support, an alignment film (described in JP1997-152509A (JP-H9-152509A)) that forms a chemical bond with the liquid crystalline molecule at the interface may be used. In a case where the alignment film is used for the intended purpose of improving the adhesiveness, it is not necessary to carry out the rubbing treatment. In a case where the two kinds of liquid crystal layers are provided on the same side of the transparent support, it is also possible to make the liquid crystal layer formed on the transparent support function as an alignment film of the liquid crystal layer provided on the transparent support.

[Transparent support]

The film according to the embodiment of the present invention or the optically anisotropic element having the film according to the embodiment of the present invention may have a transparent support.

As the transparent support, a glass plate or a polymer film, preferably a polymer film is used. That a support is transparent means that the support has a light transmittance of 80% or more.

As the transparent support, an optically isotropic polymer film is generally used. The optical isotropy specifically means that the in-plane retardation (Re) is preferably less than 10 nm and more preferably less than 5 nm. Further, in the optically isotropic transparent support, the retardation (Rth) in the thickness direction is also preferably less than 10 nm and more preferably less than 5 nm. Regarding the transparent support, the in-plane retardation (Re) and the retardation (Rth) in the thickness direction are respectively defined by the following expressions.

$$Re = (nx - ny) \quad d$$

$$Rth = [\{(nx + ny)/2\} - nz] \quad d$$

In the expression, nx and ny are each the in-plane refractive index of the transparent support, nz is the refractive index of the transparent support in the thickness direction, and d is the thickness of the transparent support.

An optically anisotropic polymer film may be used as the transparent support.

In such a case, the transparent support preferably has optical uniaxiality or optical biaxiality. In a case of an optically uniaxial support, it may be optically positive (the refractive index in the optical axis direction is larger than the refractive index in the direction perpendicular to the optical axis) or may be negative (the refractive index in the optical axis direction is smaller than the refractive index in the direction perpendicular to the optical axis). In a case of an optically biaxial support, all the refractive indices nx, ny, and nz in the above expression are values different from each other ($nx \neq ny \neq nz$).

The in-plane retardation (Re) of the optically anisotropic transparent support is preferably 10 to 1,000 nm, more preferably 15 to 300 nm, and most preferably 20 to 200 nm. The retardation (Rth) of the optically anisotropic transparent support in the thickness direction is preferably 10 to 1,000 nm, more preferably 15 to 300 nm, and still more preferably 20 to 200 nm.

A material for forming a transparent support is determined depending on whether an optically isotropic support or an optically anisotropic support is formed. In a case of an optically isotropic support, glass or a cellulose ester is generally used. In a case of an optically anisotropic support, a synthetic polymer (for example, polycarbonate, polysulfone, polyethersulfone, polyacrylate, polymethacrylate, a norbornene resin) is generally used. However, it is also possible to produce an optically anisotropic (high retardation) cellulose ester film by (1) the use of the retardation-increasing agent described in EP0911656A2, (2) the decrease in the acetylation degree of cellulose acetate, or (3) the manufacture of the film by a cooling dissolution method. The transparent support consisting of a polymer film is preferably formed by a solvent casting method.

In order to obtain an optically anisotropic transparent support, it is preferable to carry out a stretching treatment on the polymer film. In a case of producing an optically uniaxial support, a general momoaxial stretching treatment or biaxial stretching treatment may be carried out. In a case of producing an optically biaxial support, it is preferable to carry out an unbalanced biaxial stretching treatment. In unbalanced biaxial stretching, the polymer film is stretched in a certain direction at a constant stretching ratio (for example, 3% to 100%, preferably 5 to 30%) and stretched in a direction perpendicular thereto at a stretching ratio (for example, 6% to 200%, preferably 10% to 90%) equal to or higher than the constant stretching ratio. A bidirectional stretching treatment may be carried out at the same time. It is preferable

that the stretching direction (the direction in which the stretching ratio is high in unbalanced biaxial stretching) and the in-plane slow axis of the film after stretching are substantially the same directions. The angle between the stretching direction and the slow axis is preferably less than 10°, more preferably less than 5°, and most preferably less than 3°.

The thickness of the transparent support is preferably 10 to 500 μm and more preferably 50 to 200 μm. In order to improve the adhesion between the transparent support and a layer (an adhesive layer, an alignment film, or an optically anisotropic layer) provided on the transparent support, the transparent support may be subjected to a surface treatment (for example, a glow discharge treatment, a corona discharge treatment, an ultraviolet (UV) treatment, or a flame treatment). An ultraviolet absorbing agent may be added to the transparent support. An adhesive layer (an undercoat layer) may be provided on the transparent support. The adhesive layer is described in JP1995-333433A (JP-H7-333433A). The thickness of the adhesive layer is preferably 0.1 to 2 μm and more preferably 0.2 to 1 μm . The transparent support may be peeled off after the film is formed.

[Use application]

The film according to the embodiment of the present invention, particularly a film obtained by immobilizing a cholesteric liquid crystalline phase of the liquid crystal composition according to the embodiment of the present invention, can be used as a layer exhibiting selective reflection characteristics with respect to light in a predetermined wavelength range.

The layer (the cholesteric liquid crystal layer) obtained by immobilizing a cholesteric liquid crystalline phase functions as a circularly polarized selective reflective layer that selectively reflects either the dextrorotatory circularly polarized light or the levorotatory circularly polarized light in the selective reflection wavelength range of the layer and transmits the other sense circularly polarized light.

A film including one or two or more cholesteric liquid crystal layers can be used for various use applications. In a film including two or more layers of a cholesteric liquid crystal layer, the senses of circularly polarized light reflected by the cholesteric liquid crystal layers may be the same or opposite to each other depending on the use application. In addition, the central wavelengths of the selective reflection of the cholesteric liquid crystal layers, which will be described later, may be the same or different from each other depending on the use application.

In the present specification, in a case of "sense" is referred to regarding circularly polarized light, it means dextrorotatory circularly polarized light or levorotatory circularly polarized light. The sense of circularly polarized light is defined such that, in a case where light is viewed as it proceeds toward a viewer, the sense is dextrorotatory circularly polarized light in a case where the distal end of the electric field vector rotates clockwise as time increases, and the sense is levorotatory circularly polarized light in a case where it rotates counterclockwise. In the present specification, the term "sense" may be used for the twisted direction of the helix of the cholesteric liquid crystal. Selective reflection by the cholesteric liquid crystal reflects dextrorotatory circularly polarized light and transmits levorotatory circularly polarized light in a case where the twisted direction (sense) of the helix of the cholesteric liquid crystal is right-handed, whereas it reflects levorotatory circularly polarized light and transmits dextrorotatory circularly polarized light in a case where the sense is left-handed.

For example, a film including a cholesteric liquid crystal layer exhibiting selective reflection characteristics in the visible light wavelength range (wavelength of 400 to 750 nm) can be used as a screen for projected image display and a half mirror.

In addition, it can be used as a heat shield film or as an infrared cut filter for a sensor by carrying out control to exhibit selective reflection characteristics in the infrared wavelength range.

In addition, the reflective layer can be used for various use applications such as a polarizing element, a reflective film, an anti-reflection film, a viewing angle compensating film, holography, and an alignment film, which are constituent elements of an optical element.

By the above-described function of the cholesteric liquid crystal layer, a projected image can be formed by reflecting circularly polarized light of either sense at a wavelength at which selective reflection of the projected light is exhibited. The projected image may be visually recognized as it is displayed, by being displayed on the surface of the projected image display member or may be a virtual image which appears to float above the projected image display member as viewed by an observer.

The central wavelength $\lambda$ of the selective reflection depends on the pitch P of the helical structure (= the period of the helix) in a cholesteric liquid crystalline phase and follows the relationship of the average refractive index n of the cholesteric liquid crystal layer and $\lambda = n \times P$. Here, the central wavelength $\lambda$ of the selective reflection of the cholesteric liquid crystal layer means a wavelength at the centroid position of the reflection peak of a circularly polarized reflection spectrum measured from the normal direction of the cholesteric liquid crystal layer. As can be seen from the above Expression, the central wavelength of the selective reflection can be adjusted by adjusting the pitch of the helical structure. That is, for example, in order to selectively reflect either the dextrorotatory circularly polarized light or the levorotatory circularly polarized light with respect to blue light by adjusting the n value and the P value, it is possible to adjust the

central wavelength λ so that the apparent central wavelength of the selective reflection is in a wavelength range of 450 to 495 nm. It is noted that the apparent central wavelength of the selective reflection is a wavelength at the centroid position of the reflection peak of a circularly polarized light reflection spectrum of the cholesteric liquid crystal layer, where the circularly polarized reflection spectrum being measured from the observation direction in practical use (in a case of being used as a projected image display member). The pitch of the cholesteric liquid crystalline phase depends on the kind of the chiral agent to be used together with the liquid crystal compound or the addition concentration thereof, and thus in a case of adjusting these, a desired pitch can be obtained. As a method of measuring the sense or the pitch of a helix, methods described in "Easy Steps in Liquid Crystal Chemistry Experiment" p 46, edited by The Japanese Liquid Crystal Society, Sigma Publishing, published in 2007, and "Liquid Crystal Handbook" p 196, Editorial Committee of Liquid Crystal Handbook, Maruzen can be used.

In addition, a projected image display member capable of displaying full color projected images can be produced by preparing and laminating cholesteric liquid crystal layers having apparent central wavelengths of the selective reflection in the red light wavelength range, the green light wavelength range, and the blue light wavelength range, respectively.

By adjusting the central wavelength of the selective reflection of each cholesteric liquid crystal layer according to the luminescence wavelength range of the light source used for projection and the mode of use of the projected image display member, a clear projected image can be displayed with high efficiency of light utilization. In particular, by adjusting the central wavelengths of the selective reflection of the respective cholesteric liquid crystal layers according to the individual luminescence wavelength ranges of the light source used for projection or the like, a clear color projected image can be displayed with high efficiency of light utilization.

In addition, for example, in a case where the projected image display member is configured to be transmissive to light in the visible light region, a half mirror of a head-up display for a projected image display can be obtained. The half mirror for a projected image display can display the image projected from the projector so as to be visible, and in a case where the half mirror for a projected image display is observed from the same surface side where the image is displayed, the information or scenery on the opposite surface side can be observed at the same time.

## Examples

Hereinafter, the characteristics of the present invention will be described more specifically with reference to Examples and Comparative Examples. In Examples below, the material, the using amount, the proportion, the details of treatment, the treatment procedure, and the like can be suitably modified without departing from the scope of the present invention. Accordingly, the scope of the present invention should not be interpreted restrictively by the following specific examples.

[Synthesis of compound (A-101)]

A compound (A-101) was synthesized by the following route.

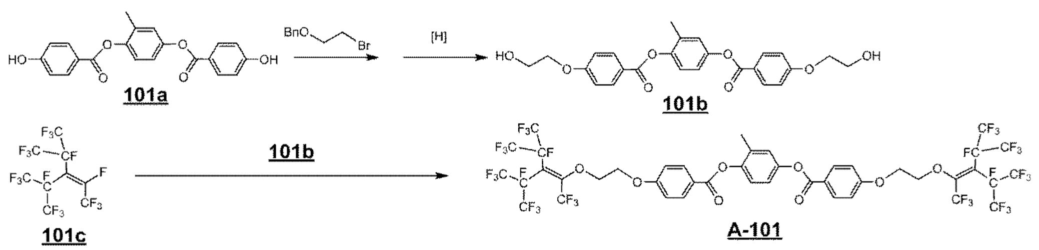

101a
101b
101c
A-101

<Synthesis of dialcohol (101b)>

A diphenol (101a) (40 mmol) consisting of hydroxybenzoic acid and methyl hydroquinone and 2-bromoethylbenzyl ether (85 mmol) was reacted at 90°C for 3 hours in 80 ml of dimethylacetamide (DMAc) in the presence of potassium carbonate (90 mmol). Ethyl acetate and hydrochloric acid water were added to carry out a liquid separation treatment, methanol was added, and the resulting solid was collected by filtration.

The entire solid collected by filtration was used to be reacted with hydrogen in 200 ml of ethyl acetate in the presence of a palladium catalyst (1.2 g, 5% palladium/activated carbon, Degussa type E 101 O/W 5% Pd, manufactured by FUJIFILM Wako Pure Chemical Corporation).

After completion of the reaction, the palladium catalyst was removed by filtration through celite, methanol was added, and the resulting solid was collected by filtration. Recrystallization was carried out using ethyl acetate and methanol to obtain a dialcohol (101b) (two step yield: 82%).

<Synthesis of compound (A-101)>

The dialcohol (101b) (10 mmol) and triethylamine (24 mmol) were dissolved in 50 mL of DMAc, and a fluorine compound (101c) (26 mmol) was added dropwise thereto, and the reaction was carried out at 40°C for 4 hours.

Then, ethyl acetate and hydrochloric acid water were added to carry out a liquid separation treatment, concentration was carried out with an evaporator, and column purification was carried out to obtain a compound (A-101) (yield: 65%).

The $^1$H-nuclear magnetic resonance (NMR) data of the obtained compound (A-101) is shown below.

$^1$H-NMR (400 MHz, $CDCl_3$) $\delta$ (ppm): 2.3 (s, 3H), 4.3-4.6 (m, 8H), 7.1-7.3 (m, 7H), 8.1 (d, 2H)

[Synthesis of compound (A-410)]

A compound (A-410) was synthesized by the following route.

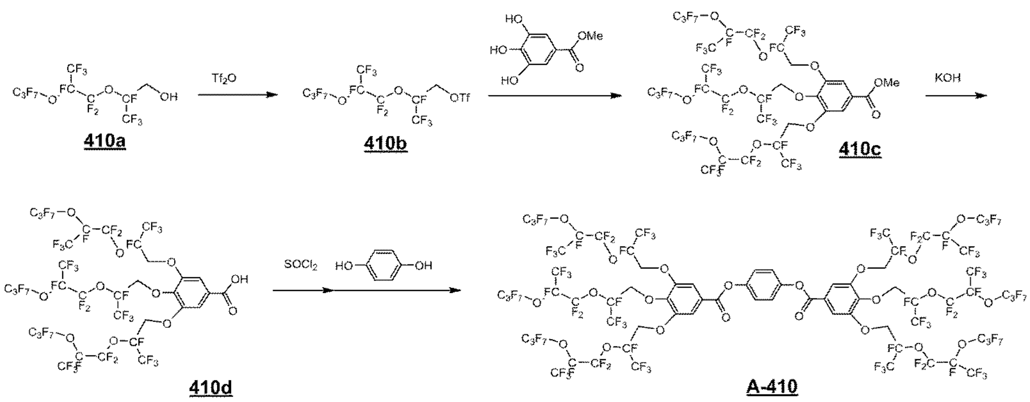


<Synthesis of ester (410b)>

An alcohol (410a) (100 mmol) was added to 100 ml of methylene chloride, and triethylamine (105 mmol) was added thereto. This solution was immersed in ice water, trifluoromethanesulfonic acid anhydride (105 mmol) was added thereto dropwise so that the internal temperature was 20°C or lower, and subjected to a reaction under ice cooling for 1 hour. The reaction solution was subjected to a liquid separation operation, and the organic layer was concentrated with an evaporator. The obtained liquid was distilled under reduced pressure to obtain a corresponding trifluoromethanesulfonic acid ester (410b) (yield: 88%).

<Synthesis of ester (410c)>

An Ester (410b) (46.5 mmol) and a gallic acid methyl ester (15 mmol) were reacted at 90°C for 2 hours in 15 ml of DMAc in the presence of potassium carbonate (46.5 mmol). After the liquid separation treatment, column purification was carried out to obtain an ester (410c) (yield: 80%).

The $^1$H-NMR data of the obtained ester (410c) is shown below.

$^1$H-NMR (400 MHz, $CDCl_3$) $\delta$ (ppm): 3.9 (s, 3H), 4.1-4.5 (m, 6H), 7.1 (s, 2H)

<Synthesis of carboxylic acid (410d)>

The ester (410c) (10 mmol) was added to 30 ml of ethanol and 3 ml of water. To this solution, potassium hydroxide (15 mmol) was added and heated for 2 hours. This reaction solution was added dropwise to an aqueous hydrochloric acid solution, ethyl acetate was added to carry out a liquid separation treatment, concentration was carried out with an evaporator, and column purification was carried out to obtain a carboxylic acid (410d) (yield: 79%).

<Synthesis of compound (A-410)>

The carboxylic acid (410d) (4.0 mmol) was reacted with thionyl chloride (6.0 mmol) in 20 ml of toluene and a catalytic amount of dimethylformamide (DMF) to obtain an acid chloride, and after removing excess thionyl chloride and toluene, 8 ml of tetrahydrofuran (THF) and a catalytic amount of 4-dimethylaminopyridine (DMAP) were added to the system.

Hydroquinone (2.0 mmol) dissolved in 8 ml of THF was added thereto, and a mixed solution of diisopropylethylamine (4.2 mmol) and 3 ml of THF was added dropwise thereto, and a reaction was carried out at room temperature for 3 hours.

After the liquid separation operation, concentration was carried out with an evaporator, and column purification was carried out to obtain a compound (A-410) (yield: 49%).

The $^1$H-NMR data of the obtained compound (A-410) is shown below.

$^1$H-NMR (400 MHz, $CDCl_3$) $\delta$ (ppm): 4.1-4.5 (m, 12H), 7.1-7.3 (s $\times$ 2,4H + 4H)

A compound (A-501) was synthesized by the following route.

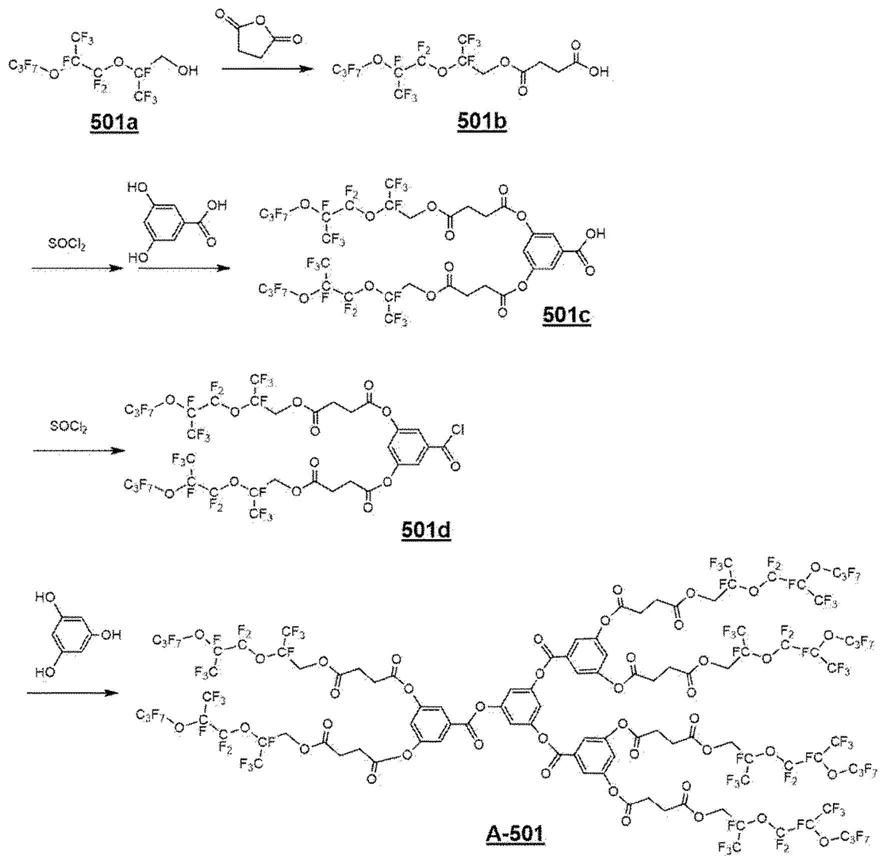


501a 501b 501c 501d A-501

<Synthesis of carboxylic acid (501b)>

An alcohol (501a) (20 mmol) and a succinic acid anhydride (20 mmol) were refluxed in tetrahydrofuran, together with a catalytic amount of triethylamine, for 2 hours. A liquid separation operation was carried out with toluene and 1 M hydrochloric acid water, and then the toluene was concentrated under reduced pressure to obtain 501b (yield: 98%).

<Synthesis of carboxylic acid (501c)>

A catalytic amount of DMF was added to a solution of a carboxylic acid (501b) (15 mmol) and 40 ml of toluene, and a reaction was carried out with thionyl chloride (18 mmol) to obtain an acid chloride, and excess thionyl chloride and toluene were removed.

Next, 3,5-dihydroxybenzoic acid (5 mmol) and the acid chloride of the 501b were added to tetrahydrofuran, and pyridine (20 mmol) was added dropwise thereto under ice cooling, followed by a reaction at room temperature for 2 hours. After adding water (5 mL), stirring was further carried out at 40°C for 30 minutes, a liquid separation operation was subsequently carried out with ethyl acetate and 1 M hydrochloric acid water. Finally, methanol and water were added, and the resulting solid was filtered and dried to obtain a carboxylic acid (501c) (yield: 74%).

The $^1$H-NMR data of the obtained carboxylic acid (501c) is shown below.

$^1$H-NMR (400 MHz, $CDCl_3$) $\delta$ (ppm): 2.7-2.9 (m, 8H), 4.2-4.5 (m, 4H), 7.6 (s, 1H), 7.8 (s, 2H)

<Synthesis of acid chloride (501d)>

A catalytic amount of DMF was added to a solution of a carboxylic acid (501c) (10 mmol) and 30 ml of toluene, and a reaction was carried out with thionyl chloride (14 mmol) to obtain an acid chloride, and excess thionyl chloride and toluene were removed, thereby obtaining an acid chloride (501d) was obtained (yield: 99%).

<Synthesis of A-501>

The acid chloride (501d) (9 mmol) and a catalytic amount of DMAP were prepared as a solution of 20 ml of THF, 1,3,5-trihydroxybenzene (2.9 mmol) dissolved in 8 ml of THF was added thereto, and a mixed solution of diisopropylethylamine (9.5 mmol) was and 5 ml of THF was added dropwise thereto, and then a reaction was carried out at room temperature for 3 hours. After the liquid separation operation, concentration was carried out with an evaporator, and column purification was carried out to obtain a compound (A-501) (yield: 62%).

The $^1$H-NMR data of the obtained compound (A-501) is shown below.

$^1$H-NMR (400 MHz, $CDCl_3$ $\delta$ (ppm): 2.7-2.9 (m, 24H), 4.2-4.5 (m, 12H), 7.3 (s, 3H), 7.6 (s, 3H), 7.8 (s, 6H)

[Synthesis of fluorine-containing compound]

<Synthesis of compound (S-2-b)>

A compound (S-2-b) was synthesized by the following route.

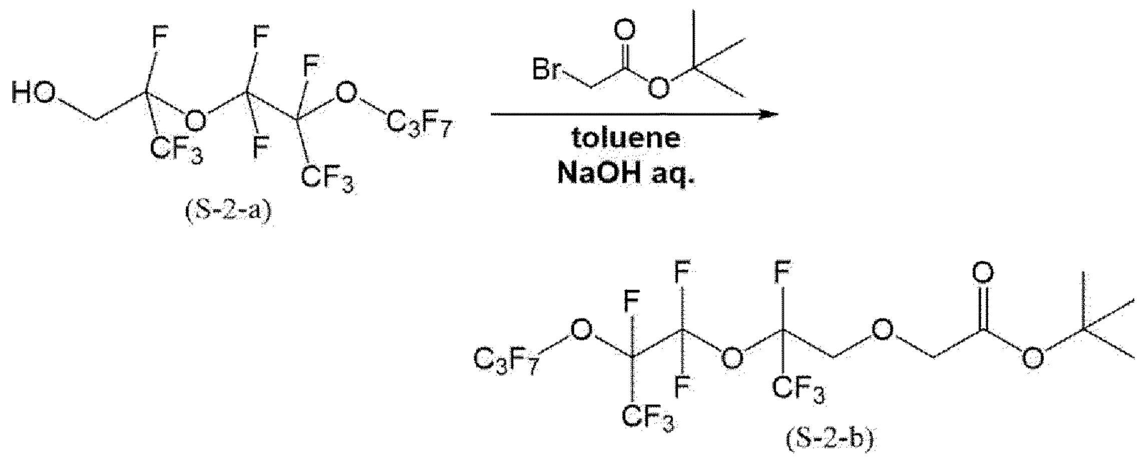


(S-2-a)

(S-2-b)

200 g (0.42 mol) of a compound (S-2-a), 28.2 g (0.08 mol) of a tetrabutylammonium hydrogen sulfate, 13 mL of water, and 180 mL of toluene were placed in a 1 L three-neck flask. Next, 96.8 g (0.50 mol) of t-butyl bromoacetate was added at room temperature, and the internal temperature was raised to 30°C after the addition. A solution obtained by dissolving 66.4 g (1.66 mol) of sodium hydroxide in 66 mL of water was added dropwise thereto while maintaining an internal temperature of 40°C or lower. The reaction solution was stirred at an internal temperature of 35°C for 2 hours and cooled to 20°C. Next, a solution obtained by mixing 145 mL of concentrated hydrochloric acid with 145 mL of water was added dropwise thereto, the resultant mixture was allowed to stand, and then the aqueous layer was removed. 51 g of salt, 460 mL of water, and 180 mL of toluene were added to the organic layer, and stirring was carried out. After being allowed to stand, the aqueous layer was removed again, and the organic layer was washed with 350 mL of a 25% saline solution. The organic layer was dried with anhydrous magnesium sulfate, the desiccant was removed by filtration, and the organic layer was concentrated with a rotary evaporator. 250 g of a colorless oily compound (S-2-b) was quantitatively obtained.

<Synthesis of compound (S-2-c)>

A compound (S-2-c) was synthesized by the following route.

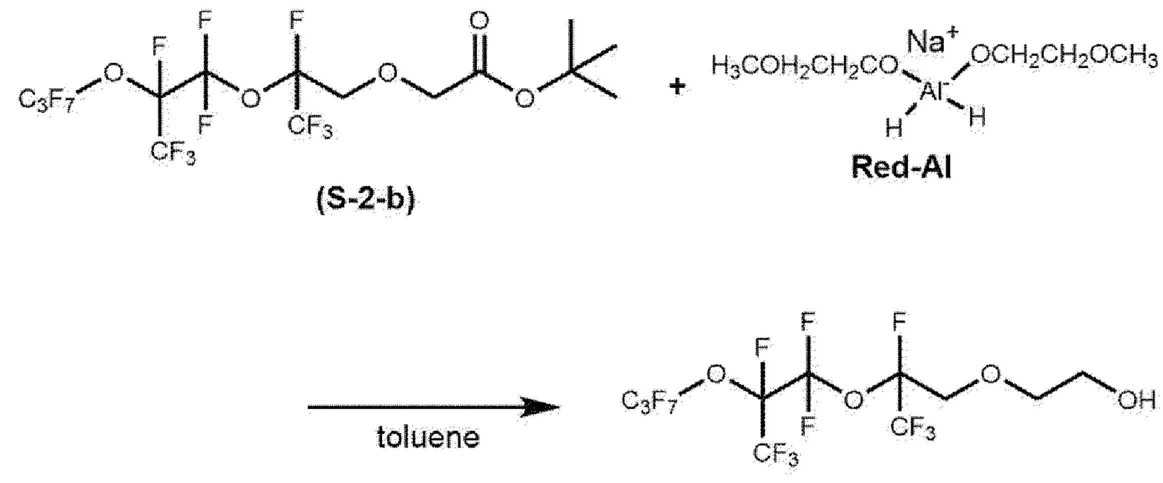


(S-2-b) Red-Al (S-2-c)

Under a nitrogen stream, 122.2 g (0.42 mol) of Red-Al and 196 mL of toluene were placed in a 2 L three-neck flask and cooled to an internal temperature of 10°C or lower. 247 g (0.41 mol) of the compound (S-2-b) was added dropwise thereto, and careful washing was carried out with 50 mL of toluene. After the dropwise addition, the internal temperature was raised to 40°C, stirring was carried out for 30 minutes, and then 12.0 g (0.04 mol) of Red-Al was added. After the addition, the reaction solution was further stirred at 40°C for 30 minutes and cooled to 15°C or lower. A solution obtained by dissolving 132.5 g (0.94 mol) of Rochelle salt tetrahydrate in 153 mL of water was added dropwise thereto, and stirring was carried out for 30 minutes. After being allowed to stand, the aqueous layer was removed, a solution obtained by dissolving 66.3 g (0.47 mol) of Rochelle salt tetrahydrate in 76 mL of water was added to the organic layer, and stirring was carried out for 5 minutes. After being allowed to stand, the aqueous layer was removed, and the organic layer was washed with 260 mL of a 10% saline solution. The organic layer was dried with anhydrous magnesium sulfate, the desiccant was removed by filtration, and the organic layer was concentrated with a rotary evaporator. 217 g (yield: 99%) of a pale yellow oily compound (S-2-c) was obtained.

<Synthesis of compound (S-2-d)>

A compound (S-2-d) was synthesized by the following route.

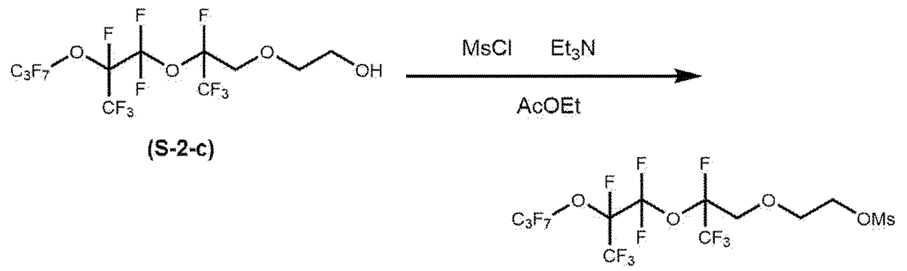


(S-2-c) (S-2-d)

205 g (0.39 mol) of the compound (S-2-c), 246 mL of ethyl acetate, and 53.6 g (0.47 mol) of methanesulfonyl chloride were placed in a 2 L three-neck flask and cooled to an internal temperature of 10°C or lower. 42.8 g (0.42 mol) of triethylamine was added dropwise thereto while maintaining an internal temperature of 20°C or lower. After the dropwise addition, the internal temperature was raised to 25°C, and stirring was carried out for 2 hours. A solution obtained by mixing 246 mL of hexane, 246 mL of a 10% saline solution, and 6.2 g of sodium bicarbonate was added thereto, and stirring was carried out for 5 minutes. After being allowed to stand, the aqueous layer was removed, and the organic layer was washed with 246 mL of a 10% saline solution. The organic layer was dried with anhydrous magnesium sulfate, the desiccant was removed by filtration, and the organic layer was concentrated with a rotary evaporator. 233 g (yield: 99%) of a pale yellow oily compound (S-2-d) was obtained.

<Synthesis of compound (S-2-e-1)>

A compound (S-2-e-1) was synthesized by the following route.

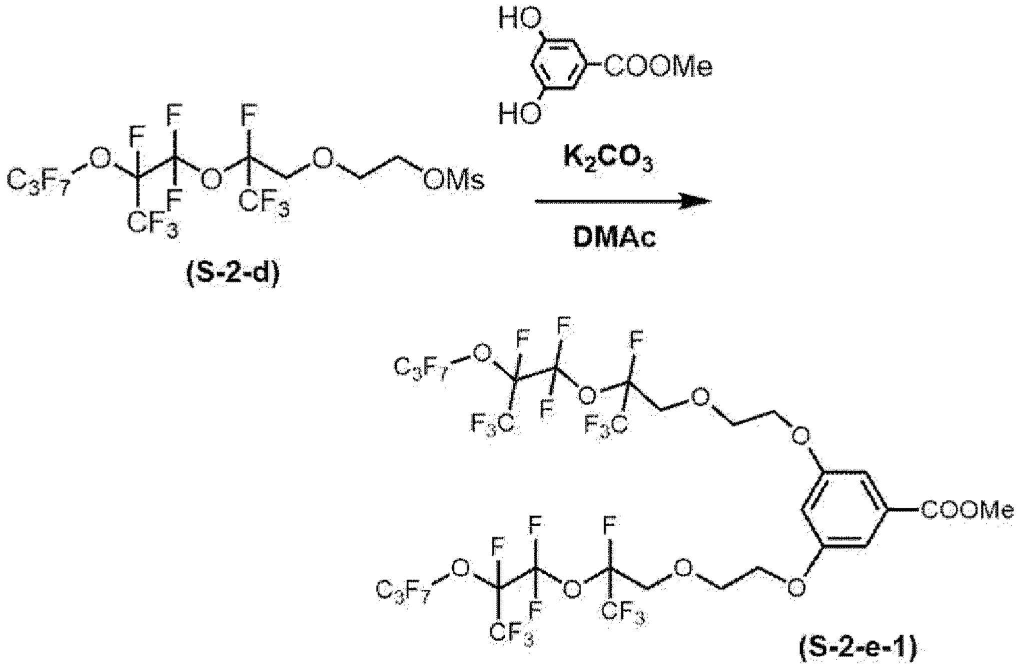


(S-2-d)

(S-2-e-1)

Under a nitrogen stream, 14.0 g (83 mmol) of methyl 3,5-dihydroxybenzoate, 324 mL of dimethylacetamide, and 36.8 g (266 mmol) of potassium carbonate were placed in a 1 L three-neck flask, and the temperature was raised to an internal temperature of 90°C. 104 g (172 mmol) of the compound (S-2-d) was added dropwise thereto, and stirring was carried out for 2 hours. The reaction solution was cooled to 25°C, 300 mL of ethyl acetate, 31 mL of concentrated hydrochloric acid, and 500 mL of water were added thereto, followed by stirring. After being allowed to stand, the aqueous layer was removed, and the organic layer was washed with 150 mL of a 10% saline solution. The organic layer was dried with anhydrous magnesium sulfate, the desiccant was removed by filtration, and the organic layer was concentrated with a rotary evaporator. 96.5 g (yield: 98%) of a pale yellow oily compound (S-2-e-1) was obtained.

<Synthesis of compound (S-2-e-2)>

A compound (S-2-e-2) was synthesized by the following route.

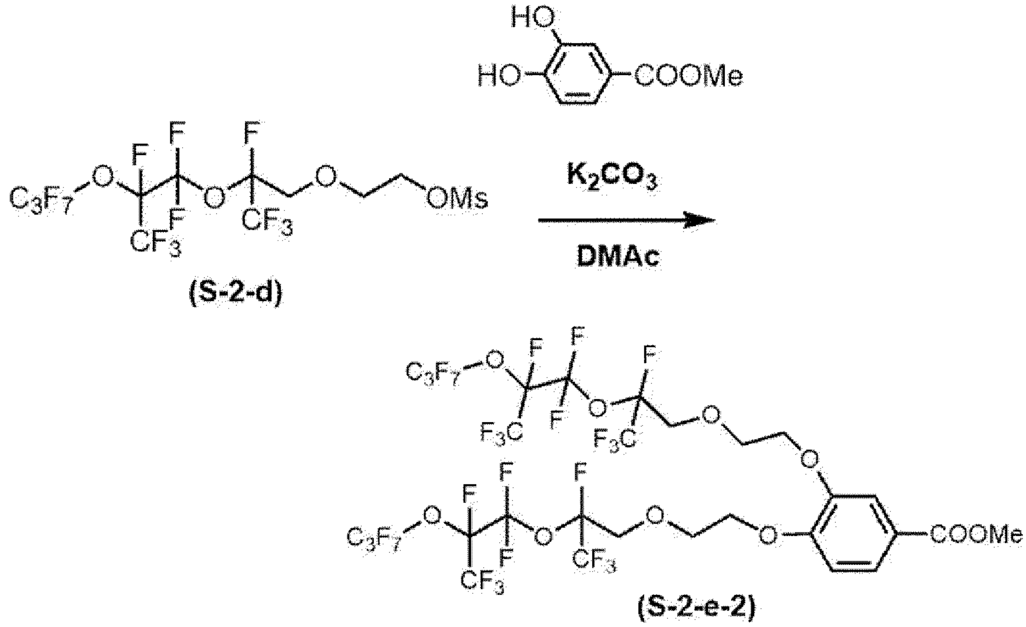


(S-2-d)

(S-2-e-2)

The synthesis was carried out in the same manner as in the compound (S-2-e-1) except that methyl 3,5-dihydroxybenzoate as a raw material was changed to methyl 3,4-dihydroxybenzoate, thereby obtaining 96.5 g (yield: 98%) of a pale yellow oily compound (S-2-e-1).

<Synthesis of compound (S-2-e-3)>

A compound (S-2-e-3) was synthesized by the following route.

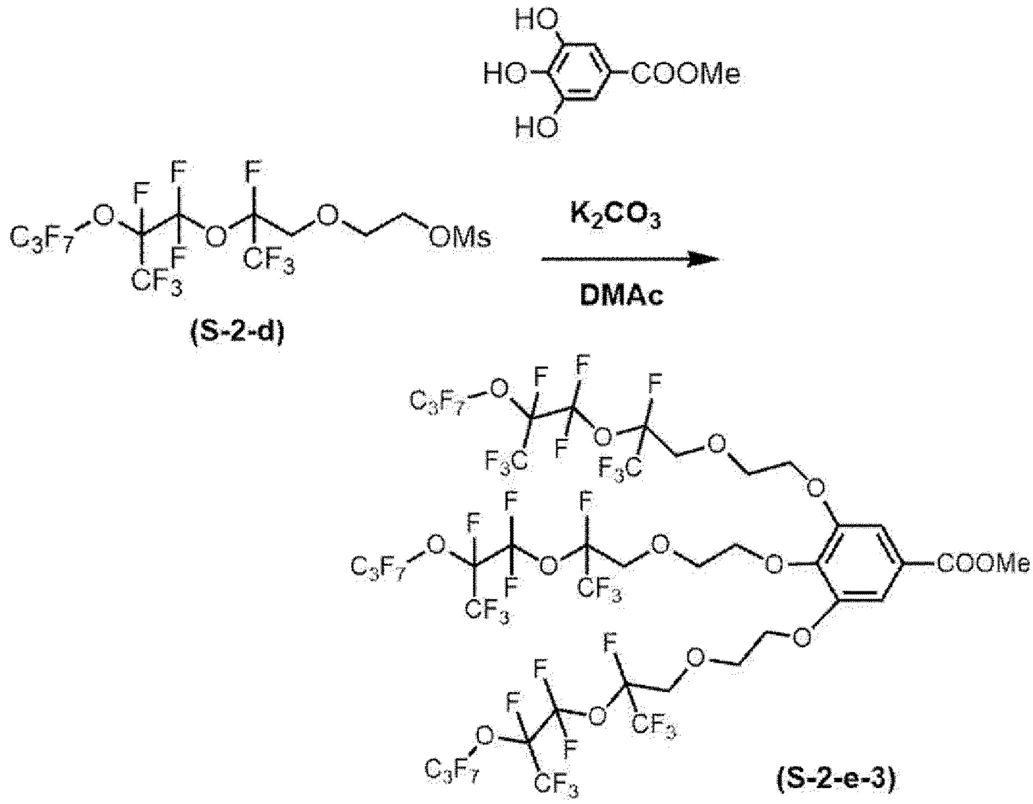


(S-2-d)

(S-2-e-3)

The synthesis was carried out in the same manner as in the compound (S-2-e-1) except that methyl 3,5-dihydroxybenzoate as a raw material was changed to methyl 3,4,5-dihydroxybenzoate and the using amounts of the

compound (S-2-d) and potassium carbonate were changed to be 1.5 times, thereby obtaining 116 g (yield: 90%) of a pale yellow oily compound (S-2-e-3).

<Synthesis of compound (S-2-e-4)>

A compound (S-2-e-4) was synthesized by the following route.

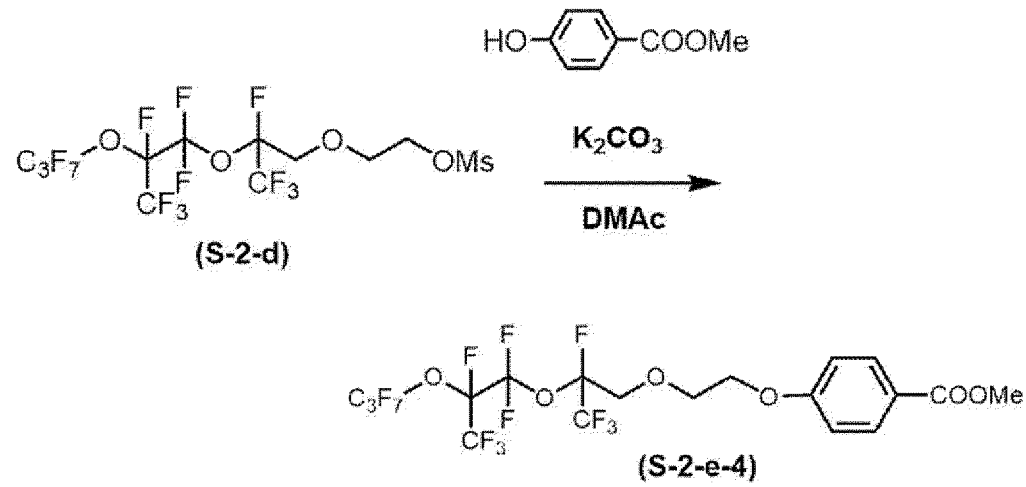


(S-2-d)

(S-2-e-4)

The synthesis was carried out in the same manner as in the compound (S-2-e-1) except that methyl 3,5-dihydroxybenzoate as a raw material was changed to methyl 4-dihydroxybenzoate and the using amounts of the compound (S-2-d) and potassium carbonate were changed to be half, thereby obtaining 50.4 g (yield: 92%) of a pale yellow oily compound (S-2-e-4).

<Synthesis of compound (S-2-f-1)>

A compound (S-2-f-1) was synthesized by the following route.

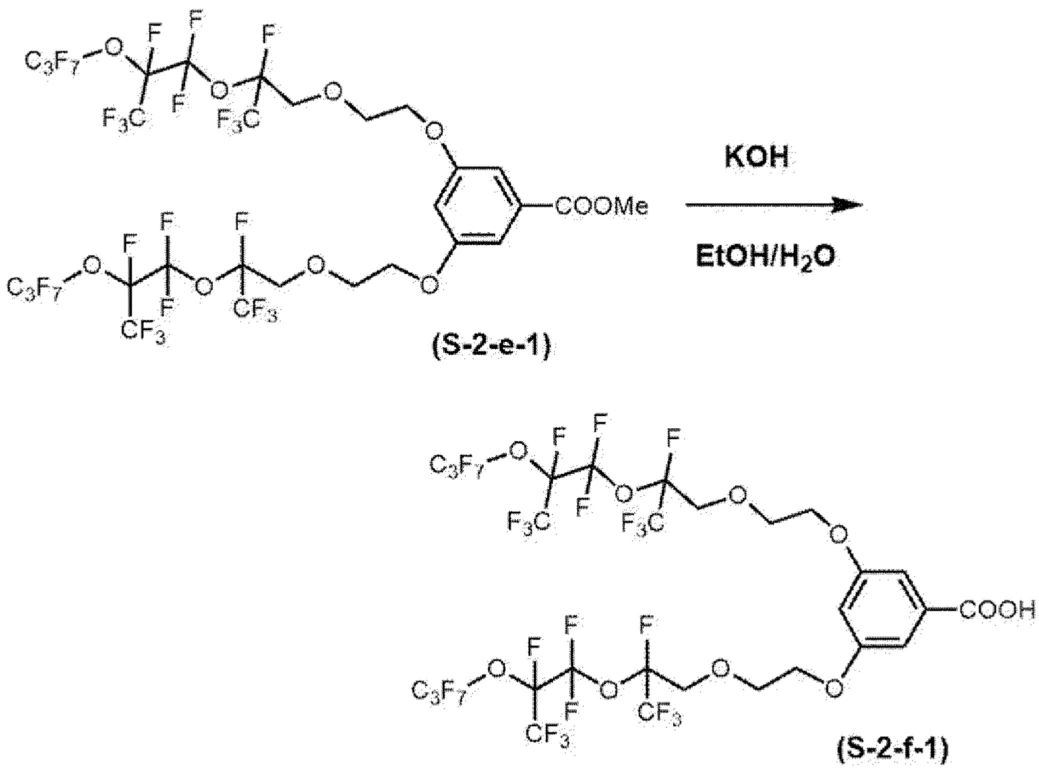


(S-2-e-1)

(S-2-f-1)

45.0 g (38 mmol) of the compound (S-2-e-1), 145 mL of ethanol, 5.3 g (95 mmol) of potassium hydroxide, and 53 mL of water were placed in a 1 L three-neck flask, and stirring was carried out at an internal temperature of 70°C for 1 hour. The reaction solution was cooled to 20°C, 240 mL of ethyl acetate, 7 mL of concentrated hydrochloric acid, and 241 mL of water were subsequently added thereto, and stirring was carried out. After being allowed to stand, the aqueous layer was removed, and the organic layer was washed with 150 mL of a 10% saline solution. The organic layer was dried with anhydrous magnesium sulfate, the desiccant was removed by filtration, and the organic layer was concentrated with a rotary evaporator. 41.1 g (yield: 97%) of a brown oily compound (S-2-f-1) was obtained.

<Synthesis of compound (S-2-f-2)>

A compound (S-2-f-2) was synthesized by the following route.

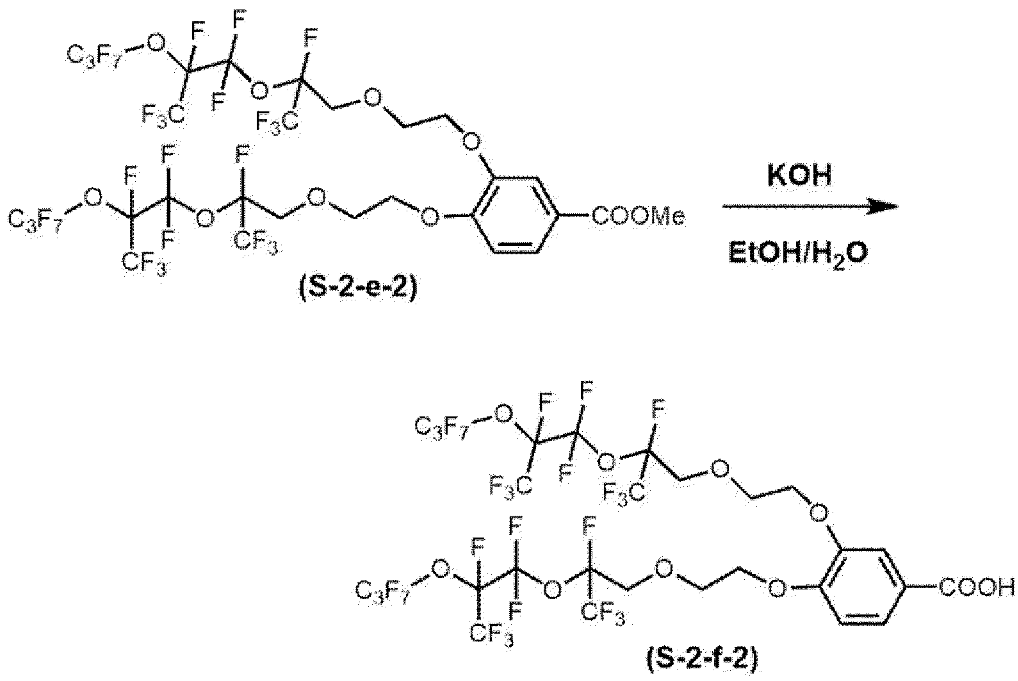


(S-2-e-2)

(S-2-f-2)

The synthesis was carried out in the same manner as in the compound (S-2-f-1) except that the compound (S-2-e-1) as a raw material was changed to a compound (S-2-e-2), thereby obtaining 40.5 g (yield: 96%) of a brown oily compound (S-2-f-1).

<Synthesis of compound (S-2-f-3)>

A compound (S-2-f-3) was synthesized by the following route.

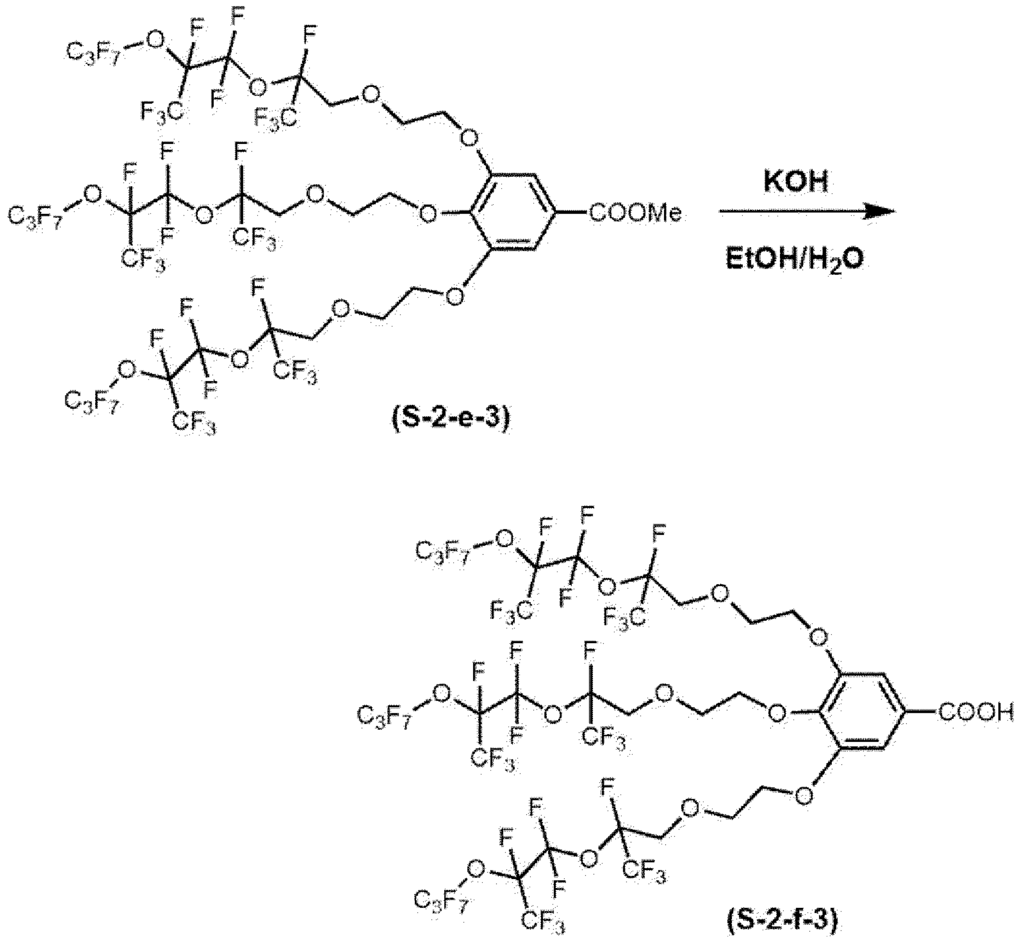


(S-2-e-3)

(S-2-f-3)

The synthesis was carried out in the same manner as in the compound (S-2-f-1) except that the compound (S-2-e-1) as a raw material was changed to a compound (S-2-e-3), thereby obtaining 113.8 g (yield: 85%) of a brown oily compound (S-2-f-3).

<Synthesis of compound (S-2-f-4)>

A compound (S-2-f-4) was synthesized by the following route.

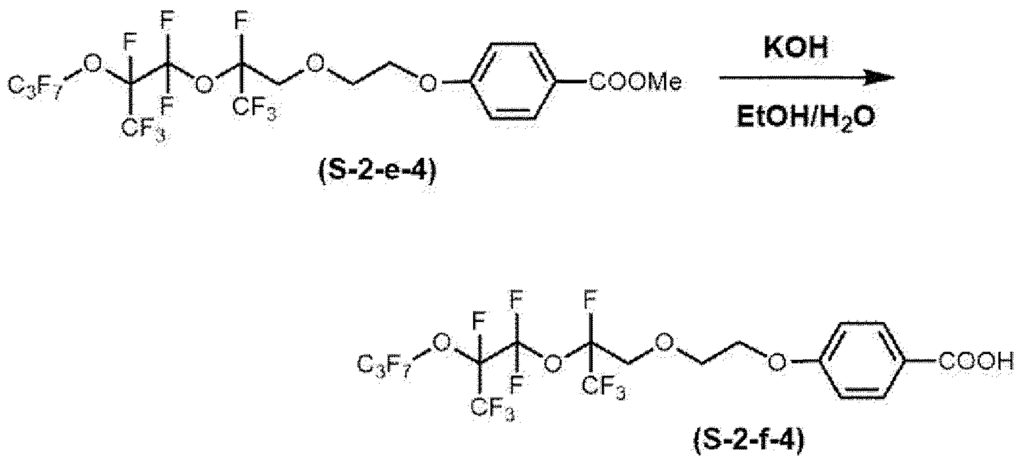


(S-2-e-4)

(S-2-f-4)

The synthesis was carried out in the same manner as in the compound (S-2-f-1) except that the compound (S-2-e-1) as a raw material was changed to a compound (S-2-e-4), thereby obtaining 51.5 g (yield: 95%) of a brown oily compound (S-2-f-4).

<Synthesis of compound (S-2-g-1)>

A compound (S-2-g-1) was synthesized by the following route.

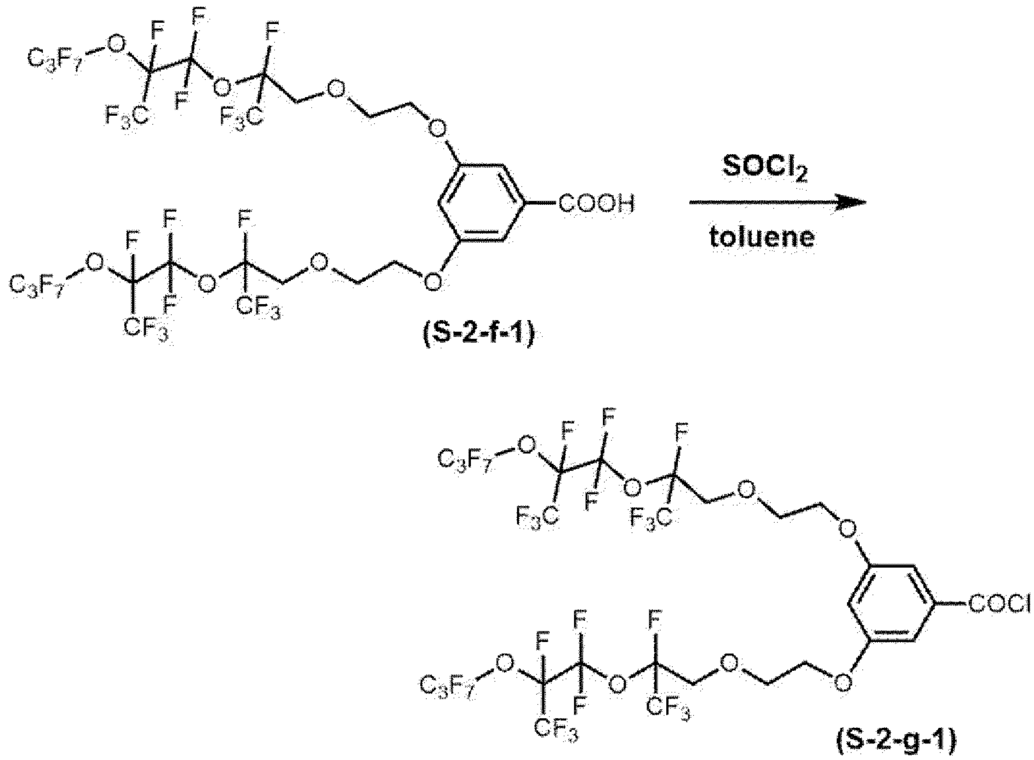


(S-2-f-1)

(S-2-g-1)

9.5 g (8.1 mmol) of the compound (S-2-f-1), 0.13 mL of dimethylformamide, and 23 mL of toluene were placed in a 100 mL three-neck flask, and the temperature was raised to an internal temperature of 70°C. 1.2 mL (18 mmol) of thionyl chloride was added thereto, and stirring was carried out for 1 hour. Toluene was distilled off under reduced pressure to quantitatively obtain 9.7 g of a brown oily compound (S-2-g-1).

<Synthesis of compound (S-2-g-2)>

A compound (S-2-g-2) was synthesized by the following route.

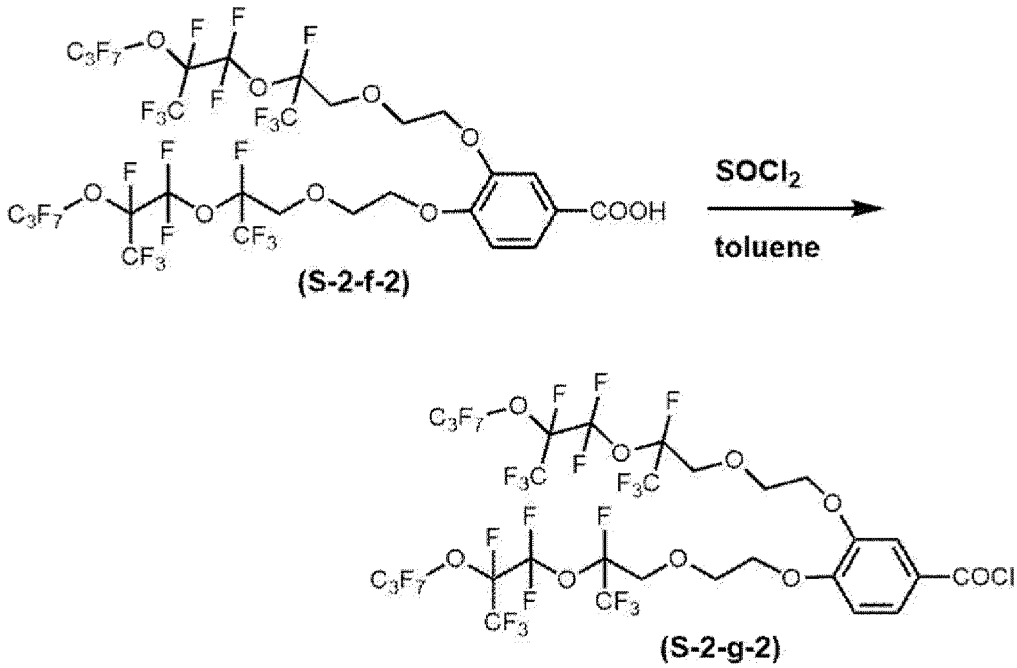


(S-2-f-2)

(S-2-g-2)

The synthesis was carried out in the same manner as in the compound (S-2-g-1) except that the compound (S-2-f-1) as a raw material was changed to a compound (S-2-f-2), thereby quantitatively obtaining 9.8 g of a brown oily compound (S-2-g-2).

<Synthesis of compound (S-2-g-3)>

A compound (S-2-g-3) was synthesized by the following route.

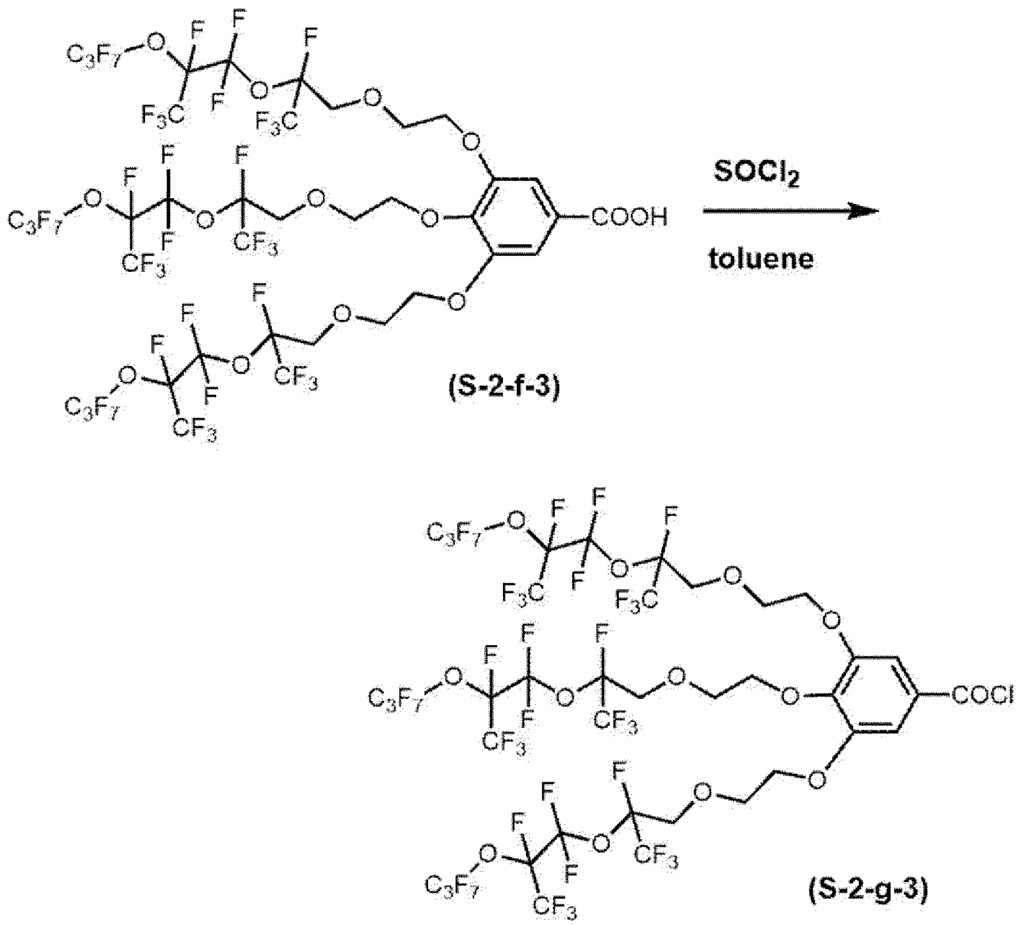


(S-2-f-3)

(S-2-g-3)

The synthesis was carried out in the same manner as in the compound (S-2-g-1) except that the compound (S-2-f-1) as a raw material was changed to a compound (S-2-f-3), thereby quantitatively obtaining 9.9 g of a brown oily compound (S-2-g-3).

<Synthesis of compound (S-2-g-4)>

A compound (S-2-g-4) was synthesized by the following route.

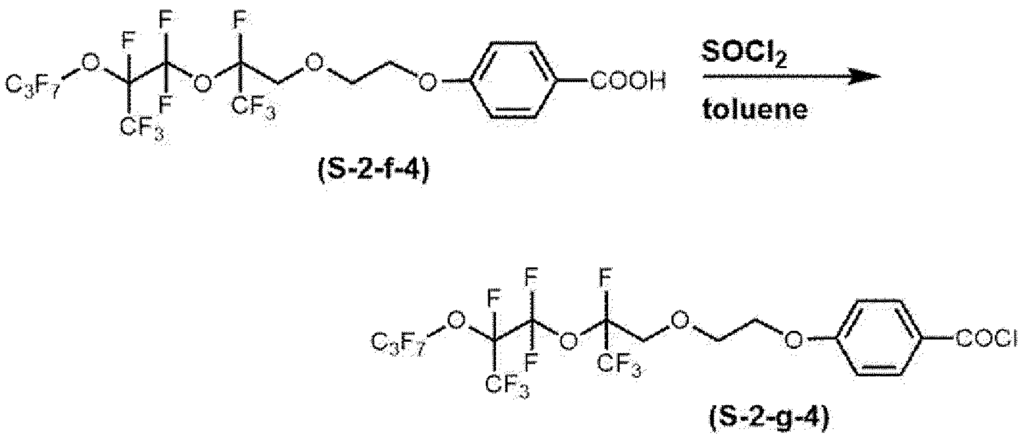


(S-2-f-4)

(S-2-g-4)

The synthesis was carried out in the same manner as in the compound (S-2-g-1) except that the compound (S-2-f-1) as a raw material was changed to a compound (S-2-f-4), thereby quantitatively obtaining 9.6 g of a brown oily compound (S-2-g-4).

[Synthesis of compound (A-1003)]

A compound (A-1003) was synthesized by the following route.

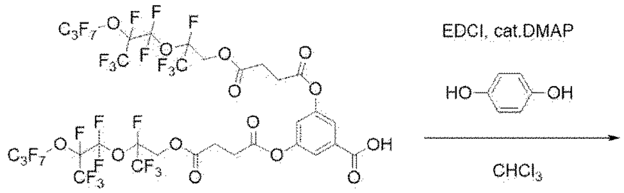


(501c)

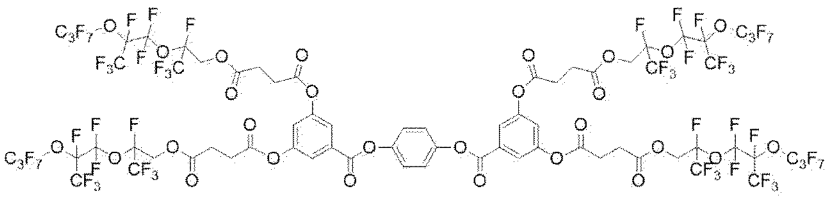

(A-1003)

3.0 g (2.3 mmol) of the compound (501c), 117 mg (1.0 mmol) of hydroquinone, 13 mg (0.1 mmol) of N-dimethylaminopyridine, 20 mL of chloroform, and 0.62 g (33 mmol) of 1-ethyl-3-(3-dimethylaminopropyl)carbodiimide (EDC) hydrochloride were placed in a 100 mL three-neck flask, and stirring was carried out at 25°C for 1 hour. The reaction solution was purified by silica gel column chromatography to obtain 1.41 g (yield: 50%) of a white solid compound (A-1003).

The $^1$H-NMR result of the compound (A-1003) is shown below.

$^1$H-NMR (solvent: heavy acetone) δ (ppm): 7.85 (d, 4H), 7.46 (s, 4H), 7.32 (t, 2H), 5.02-4.88 (m, 8H), 3.06-3.03 (m, 8H), 2.94-2.80 (m, 8H)

[Synthesis of compound (A-1017)]

The synthesis was carried out in the same manner as in the compound (A-1003) except that the raw material was changed to 1,5-dihydroxynaphthalene, thereby obtaining 1.5 g (yield: 52%) of a white solid compound (A-1017).

The $^1$H-NMR result of the compound (A-1017) is shown below.

$^1$H-NMR (solvent: heavy acetone) δ (ppm): 7.98 (t, 6H), 7.69-7.60 (m, 4H), 7.38 (t, 2H), 5.02-4.88 (m, 8H), 3.08-3.05 (m, 8H), 2.95-2.92 (m, 8H)

[Synthesis of compound (A-1101)]

A compound (A-1101) was synthesized by the following route.

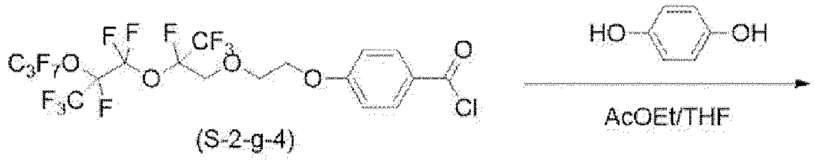


(S-2-g-4)

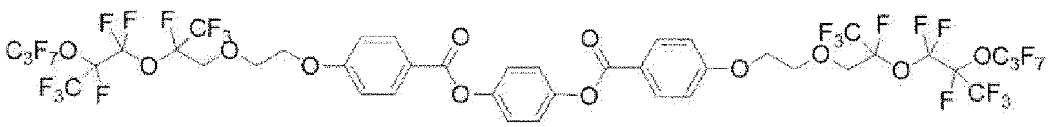

(A-1101)

152.7 g (0.23 mol) of the compound (S-2-g-4), 466 mL of tetrahydrofuran, 467 mL of ethyl acetate, and 11.5 g

(0.10 mol) of hydroquinone were placed in a 2 L three-neck flask, and cooling was carried out to an internal temperature of 10°C or lower. 31.7 g (0.31 mol) of triethylamine was added dropwise thereto, and the internal temperature was raised to 25°C after completion of the dropwise addition. Stirring was carried out at 25°C for 2 hours, and 30 mL of methanol was added, followed by stirring for 5 minutes. 305 mL of ethyl acetate and 200 mL of 1 M hydrochloric acid water were added thereto, and stirring was carried out. After being allowed to stand, the aqueous layer was removed, and the organic layer was washed with 305 mL of a 10% saline solution. The organic layer was dried with anhydrous magnesium sulfate, the desiccant was removed by filtration, and the organic layer was concentrated with a rotary evaporator. 176.2 g of a brown oily crude product was obtained. The crude product was purified by silica gel column chromatography to obtain 107.0 g (yield: 75%) of a white solid compound (A-1101).

The $^1$H-NMR result of the compound (A-1101) is shown below.

$^1$H-NMR (solvent: heavy acetone) δ (ppm): 8.17-8.14 (m, 4H), 7.37 (s, 4H), 7.15 (d, 4H), 4.45-4.11 (m, 8H), 4.13-4.11 (m, 4H)

[Synthesis of compound (A-1102)]

The synthesis was carried out in the same manner as in the compound (A-1101) except that the compound (S-2-g-4) as a raw material was changed to a compound (S-2-g-2), thereby obtaining 164 g (yield: 65%) of a colorless waxy compound (A-1102).

The $^1$H-NMR result of the compound (A-1102) is shown below.

$^1$H-NMR (solvent: heavy acetone) δ (ppm): 7.87 (dd, 2H), 7.80 (d, 2H), 7.36 (s, 4H), 7.21 (d, 2H), 4.47-4.44 (m, 8H), 4.40-4.34 (m, 8H), 4.14-4.09 (m, 8H)

[Synthesis of compound (A-1103)]

The synthesis was carried out in the same manner as in the compound (A-1101) except that the compound (S-2-g-4) as a raw material was changed to a compound (S-2-g-1), thereby obtaining 162 g (yield: 64%) of a colorless waxy compound (A-1103).

The $^1$H-NMR result of the compound (A-1103) is shown below.

$^1$H-NMR (solvent: heavy acetone) δ (ppm): 7.40 (s, 4H), 7.37 (d, 4H), 6.88 (s, 2H), 4.44-4.10 (m, 8H), 4.34-4.31 (m, 8H), 4.11-4.09 (m, 8H)

[Synthesis of compound (A-1104)]

The synthesis was carried out in the same manner as in the compound (A-1101) except that the compound (S-2-g-4) as a raw material was changed to a compound (S-2-g-3), thereby obtaining 160 g (yield: 63%) of a colorless oily compound (A-1104).

The $^1$H-NMR result of the compound (A-1104) is shown below.

$^1$H-NMR (solvent: heavy acetone) δ (ppm): 7.54 (s, 4H), 7.37 (s, 4H), 4.46-4.33 (m, 24H), 4.14-4.12 (m, 8H), 4.03-4.00 (m, 4H)

[Synthesis of compound (A-1106)]

A compound (A-1106) was synthesized by the following route.

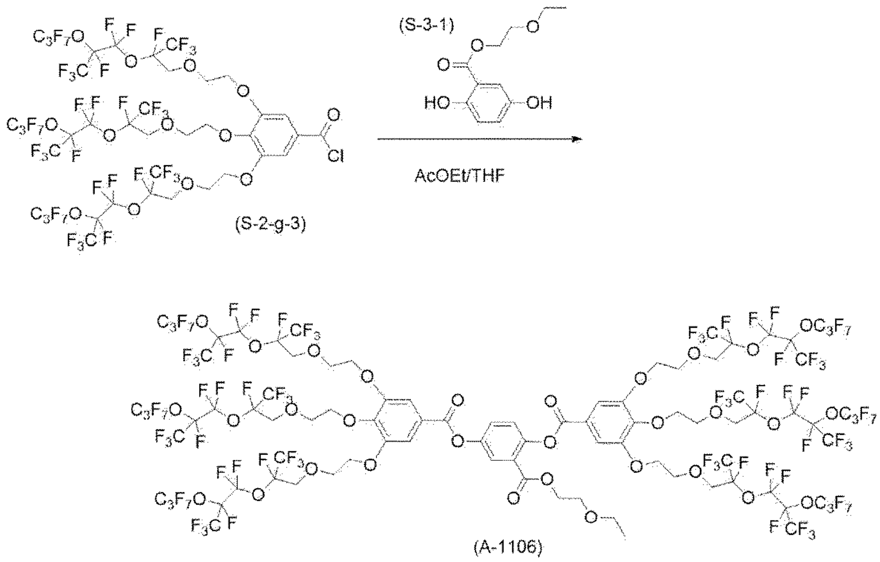


(S-2-g-3)

(A-1106)

The synthesis was carried out in the same manner as in the compound (A-1104) except that hydroquinone as raw material was changed to a compound (S-3-1), thereby obtaining 220.4 g (yield: 59%) of a colorless oily compound (A-1106).

The $^1$H-NMR result of the compound (A-1106) is shown below.

$^1$H-NMR (solvent: heavy acetone) δ (ppm): 7.93 (d, 1H), 7.65 (dd, 1H), 7.57 (d, 4H), 7.46 (d, 1H), 4.47-4.30 (m, 26H), 4.15-4.13 (m, 8H), 4.03-4.01 (m, 4H), 3.53 (t, 2H), 3.33 (t, 2H), 1.05 (t, 3H)

[Synthesis of compound (A-1107)]

The synthesis was carried out in the same manner as in the compound (A-1102) except that hydroquinone as a raw material was changed to tetrafluorohydroquinone, thereby obtaining 166 g (yield: 64%) of a colorless oily compound (A-1107).

The $^1$H-NMR result of the compound (A-1107) is shown below.

$^1$H-NMR (solvent: heavy acetone) δ (ppm): 7.94 (dd, 2H), 7.78 (s, 2H), 7.28 (d, 2H), 4.48-4.37 (m, 16H), 4.15-4.10 (m, 8H)

[Synthesis of compound (A-1108)]

The synthesis was carried out in the same manner as in the compound (A-1102) except that hydroquinone as a raw material was changed to 2,5-di-tert-butylhydroxyquinone, thereby obtaining 177 g (yield: 67%) of a colorless oily compound (A-1108).

The $^1$H-NMR result of the compound (A-1108) is shown below.

$^1$H-NMR (solvent: heavy acetone) δ (ppm): 7.90 (dd, 2H), 7.77 (s, 2H), 7.27 (d, 2H), 7.20 (s, 2H), 4.48-4.35 (m, 16H), 4.14-4.11 (m, 8H), 1.36 (s, 18H)

[Synthesis of compound (A-1109)]

A compound (A-1109) was synthesized by the following route.

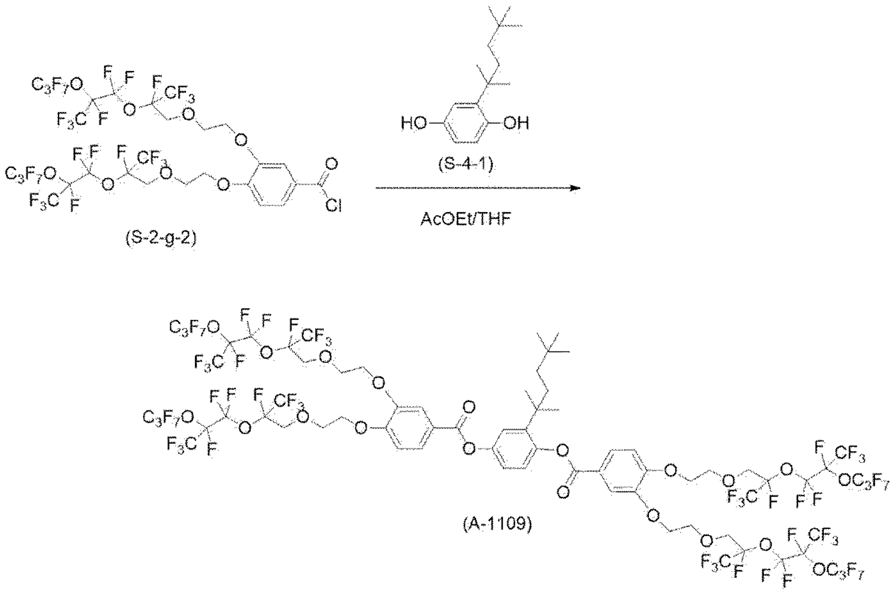


The synthesis was carried out in the same manner as in the compound (A-1102) except that hydroquinone as a raw material was changed to a compound (S-4-1), thereby obtaining 164 g (yield: 62%) of a colorless oily compound (A-1109).

The $^1$H-NMR result of the compound (A-1109) is shown below.

$^1$H-NMR (solvent: heavy acetone) $\delta$ (ppm): 7.92-7.85 (m, 2H), 7.76 (dd, 2H), 7.37 (s, 1H), 7.22-7.20 (m, 4H), 4.48-4.35 (m, 16H), 4.14-4.10 (m, 8H), 1.95 (s, 2H), 1.42 (s, 6H), 0.82 (s, 9H)

[Synthesis of compound (A-1115)]

The synthesis was carried out in the same manner as in the compound (A-1102) except that hydroquinone as a raw material was changed to p-phenylenediamine, thereby obtaining 171 g (yield: 68%) of a colorless solid compound (A-1115).

The $^1$H-NMR result of the compound (A-1115) is shown below.

$^1$H-NMR (solvent: heavy acetone) $\delta$ (ppm): 9.42 (s, 2H), 7.79 (s, 4H), 7.67 (d, 4H), 7.12 (d, 2H), 4.47-4.44 (m, 8H), 4.34-4.31 (m, 8H), 4.11-4.09 (m, 8H)

[Synthesis of compound (A-1117)]

The synthesis was carried out in the same manner as in the compound (A-1104) except that hydroquinone as a raw material was changed to p-phenylenediamine, thereby obtaining 216 g (yield: 60%) of a colorless waxy compound (A-1117).

The $^1$H-NMR result of the compound (A-1117) is shown below.

$^1$H-NMR (solvent: heavy acetone) $\delta$ (ppm): 9.47 (s, 2H), 7.76 (s, 4H), 7.37 (s, 4H), 4.46-4.33 (m, 20H), 4.28-4.26 (m, 4H), 4.13-4.11 (m, 8H), 4.01-3.98 (m, 4H)

[Synthesis of compound (A-1119)]

The synthesis was carried out in the same manner as in the compound (A-1102) except that hydroquinone as a raw material was changed to 4,4'-dihydroxybiphenyl, thereby obtaining 182 g (yield: 71%) of a colorless waxy compound (A-1119).

The $^1$H-NMR result of the compound (A-1119) is shown below.

$^1$H-NMR (solvent: heavy acetone) $\delta$ (ppm): 7.86 (d, 2H), 7.81-7.76 (m, 6H), 7.38 (d, 4H), 7.22 (d, 2H), 4.48-4.35 (m, 16H), 4.14-4.10 (m, 8H)

[Synthesis of compound (A-1121)]

The synthesis was carried out in the same manner as in the compound (A-1104) except that hydroquinone as a raw material was changed to 4,4'-dihydroxybiphenyl, thereby obtaining 232 g (yield: 63%) of a colorless waxy compound

(A-1121).
The $^1$H-NMR result of the compound (A-1121) is shown below.
$^1$H-NMR (solvent: heavy acetone) δ (ppm): 7.82 (d, 4H), 7.55 (s, 4H), 7.38 (d, 4H), 4.47-4.33 (m, 24H), 4.15-4.13 (m, 8H), 4.03-4.01 (m, 4H)

[Synthesis of compound (A-1131)]

The synthesis was carried out in the same manner as in the compound (A-1102) except that hydroquinone as a raw material was changed to 1,5-dihydroxynaphthalene, thereby obtaining 188 g (yield: 73%) of a colorless solid compound (A-1131).
The $^1$H-NMR result of the compound (A-1131) is shown below.
$^1$H-NMR (solvent: heavy acetone) δ (ppm): 8.01 (d, 2H), 8.00-7.87 (m, 2H), 7.62 (d, 2H), 7.51 (d, 2H), 7.28 (d, 2H), 4.49-4.38 (m, 16H), 4.16-4.11 (m, 8H)

[Synthesis of compound (A-1132)]

The synthesis was carried out in the same manner as in the compound (A-1103) except that hydroquinone as a raw material was changed to 1,5-dihydroxynaphthalene, thereby obtaining 178 g (yield: 69%) of a colorless solid compound (A-1132).
The $^1$H-NMR result of the compound (A-1132) is shown below.
$^1$H-NMR (solvent: heavy acetone) δ (ppm): 7.93 (d, 2H), 7.65 (t, 2H), 7.54 (d, 2H), 7.49 (s, 4H), 6.94 (s, 2H), 4.45-4.35 (m, 16H), 4.13-4.11 (m, 8H)

[Synthesis of compound (A-1133)]

The synthesis was carried out in the same manner as in the compound (A-1104) except that hydroquinone as a raw material was changed to 1,5-dihydroxynaphthalene, thereby obtaining 235 g (yield: 64%) of a colorless solid compound (A-1133).
The $^1$H-NMR result of the compound (A-1133) is shown below.
$^1$H-NMR (solvent: heavy acetone) δ (ppm): 7.91 (d, 2H), 7.67 (s, 4H), 7.64 (t, 2H), 7.51 (d, 2H), 4.47-4.37 (m, 24H), 4.16-4.14 (m, 8H), 4.04-4.03 (m, 4H)

[Synthesis of compound (A-1135)]

The synthesis was carried out in the same manner as in the compound (A-1102) except that hydroquinone as a raw material was changed to 1,5-diaminonaphthalene, thereby obtaining 154 g (yield: 60%) of a colorless solid compound (A-1135).
The $^1$H-NMR result of the compound (A-1135) is shown below.
$^1$H-NMR (solvent: heavy acetone) δ (ppm): 9.57 (s, 2H), 8.02 (d, 2H), 7.85-7.79 (m, 6H), 7.55 (t, 2H), 7.18 (d, 2H), 4.48-4.45 (m, 8H), 4.38-4.35 (m, 8H), 4.13-4.10 (m, 8H)

[Synthesis of compound (A-1137)]

The synthesis was carried out in the same manner as in the compound (A-1104) except that hydroquinone as a raw material was changed to 1,5-diaminonaphthalene, thereby obtaining 227 g (yield: 60%) of a colorless solid compound (A-1137).
The $^1$H-NMR result of the compound (A-1137) is shown below.
$^1$H-NMR (solvent: heavy acetone) δ (ppm): 9.64 (s, 2H), 8.02 (d, 2H), 7.78 (d, 2H), 7.55 (s, 4H), 7.55 (t, 2H), 4.47-4.38 (m, 24H), 4.15-4.14 (m, 8H), 4.03-4.02 (m, 4H)

[Synthesis of compound (A-1139)]

A compound (A-1139) was synthesized by the following route.

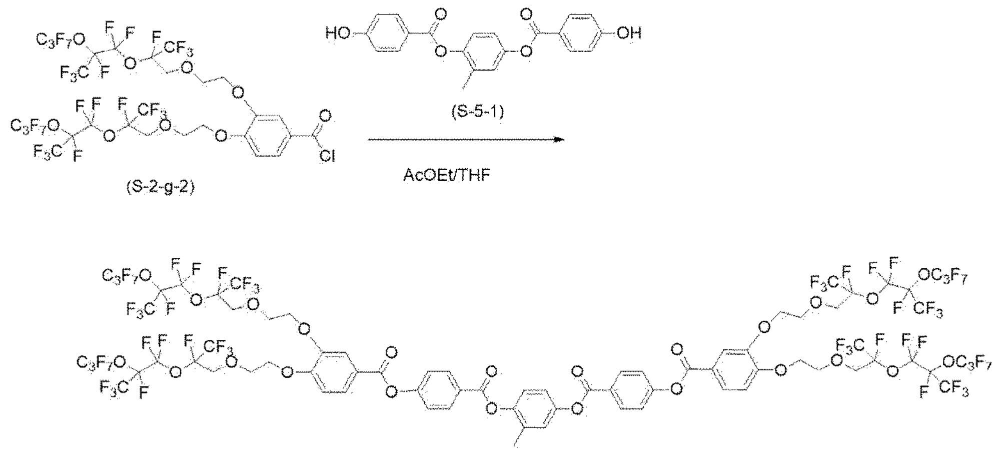


(S-2-g-2) (S-5-1)

(A-1139)

The synthesis was carried out in the same manner as in the compound (A-1102) except that hydroquinone as a raw material was changed to a compound (S-5-1), thereby obtaining 189 g (yield: 68%) of a colorless solid compound (A-1139).

The $^1H$-NMR result of the compound (A-1139) is shown below.

$^1H$-NMR (solvent: heavy acetone) δ (ppm): 8.36-8.31 (m, 4H), 7.89 (d, 2H), 7.77 (s, 2H), 7.55-7.52 (m, 4H), 7.49-7.22 (m, 5H), 4.48-4.36 (m, 18H), 4.14-4.10 (m, 8H)

[Synthesis of compound (A-1142)]

A compound (A-1142) was synthesized by the following route.

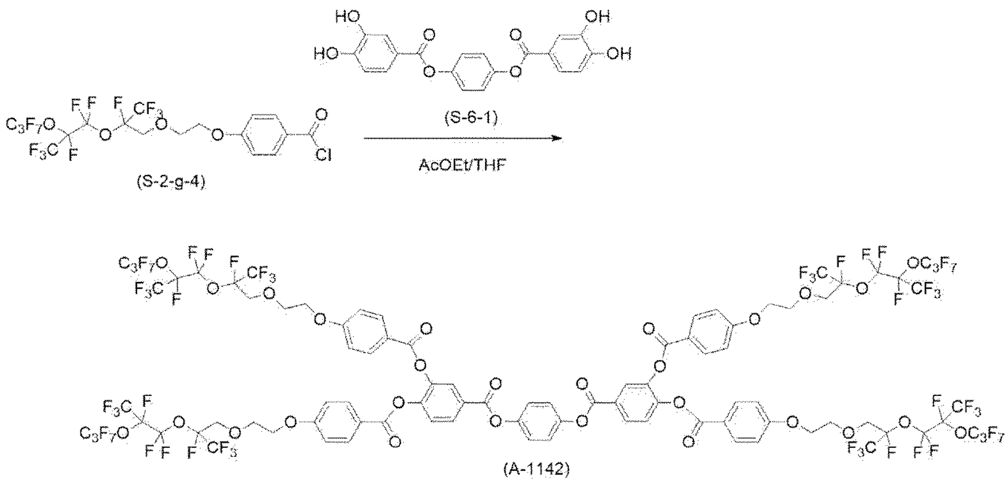


(S-2-g-4) (S-6-1)

(A-1142)

The synthesis was carried out in the same manner as in the compound (A-1101) except that hydroquinone as a raw material was changed to the compound (S-6-1) and the using amounts of the compound (S-2-g-4) and the triethylamine were changed to be 2 times, thereby obtaining 175 g (yield: 58%) of a colorless solid compound (A-1142).

The $^1H$-NMR result of the compound (A-1142) is shown below.

$^1H$-NMR (solvent: heavy acetone) δ (ppm): 8.28-8.25 (m, 4H), 8.07-8.03 (m, 8H), 7.73 (d, 2H), 7.50 (s, 4H), 7.05 (d, 8H), 4.42 (s, 4H), 4.39 (s, 4H), 4.31 (t, 8H), 4.08 (s, 8H)

[Synthesis of compound (A-1144)]

The synthesis was carried out in the same manner as in the compound (A-1102) except that hydroquinone as a raw material was changed to phloroglucinol and the using amounts of the compound (S-2-g-2) and the triethylamine were changed to be 1.5 times, thereby obtaining 195 g (yield: 52%) of a colorless oily compound (A-1144).

The $^1H$-NMR result of the compound (A-1144) is shown below.

$^1$H-NMR (solvent: heavy acetone) δ (ppm): 7.86 (d, 3H), 7.77 (s, 3H), 7.23 (s, 3H), 7.21 (d, 3H), 4.47-4.34 (m, 24H), 4.13-4.09 (m, 12H)

[Synthesis of compound (A-1145)]

The synthesis was carried out in the same manner as in the compound (A-1103) except that hydroquinone as a raw material was changed to phloroglucinol and the using amounts of the compound (S-2-g-1) and the triethylamine were changed to be 1.5 times, thereby obtaining 209 g (yield: 56%) of a colorless oily compound (A-1145).
The $^1$H-NMR result of the compound (A-1145) is shown below.
$^1$H-NMR (solvent: heavy acetone) δ (ppm): 7.48 (s, 6H), 7.29 (s, 3H), 6.88 (s, 3H), 4.44 (s, 6H), 4.40 (s, 6H), 4.31 (t, 12H), 4.10 (t, 12H)

[Synthesis of compound (A-1146)]

The synthesis was carried out in the same manner as in the compound (A-1104) except that hydroquinone as a raw material was changed to phloroglucinol and the using amounts of the compound (S-2-g-3) and the triethylamine were changed to be 1.5 times, thereby obtaining 275 g (yield: 51%) of a colorless oily compound (A-1146).
The $^1$H-NMR result of the compound (A-1146) is shown below.
$^1$H-NMR (solvent: heavy acetone) δ (ppm): 7.55 (s, 6H), 7.23 (s, 3H), 4.48-4.32 (m, 34H), 4.12 (t, 12H), 4.01 (t, 8H)

[Synthesis of compound (A-1153)]

The synthesis was carried out in the same manner as in the compound (A-1104) except that hydroquinone as a raw material was changed to 1,2-dihydroxyethane, thereby obtaining 178 g (yield: 50%) of a colorless oily compound (A-1153).
The $^1$H-NMR result of the compound (A-1153) is shown below.
$^1$H-NMR (solvent: heavy acetone) δ (ppm): 7.36 (s, 4H), 4.66 (s, 4H), 4.40 (d, 12H), 4.26 (t, 12H), 4.09 (t, 8H), 3.97 (t, 4H)

[Synthesis of compound (A-1156)]

A compound (A-1156) was synthesized by the following route.

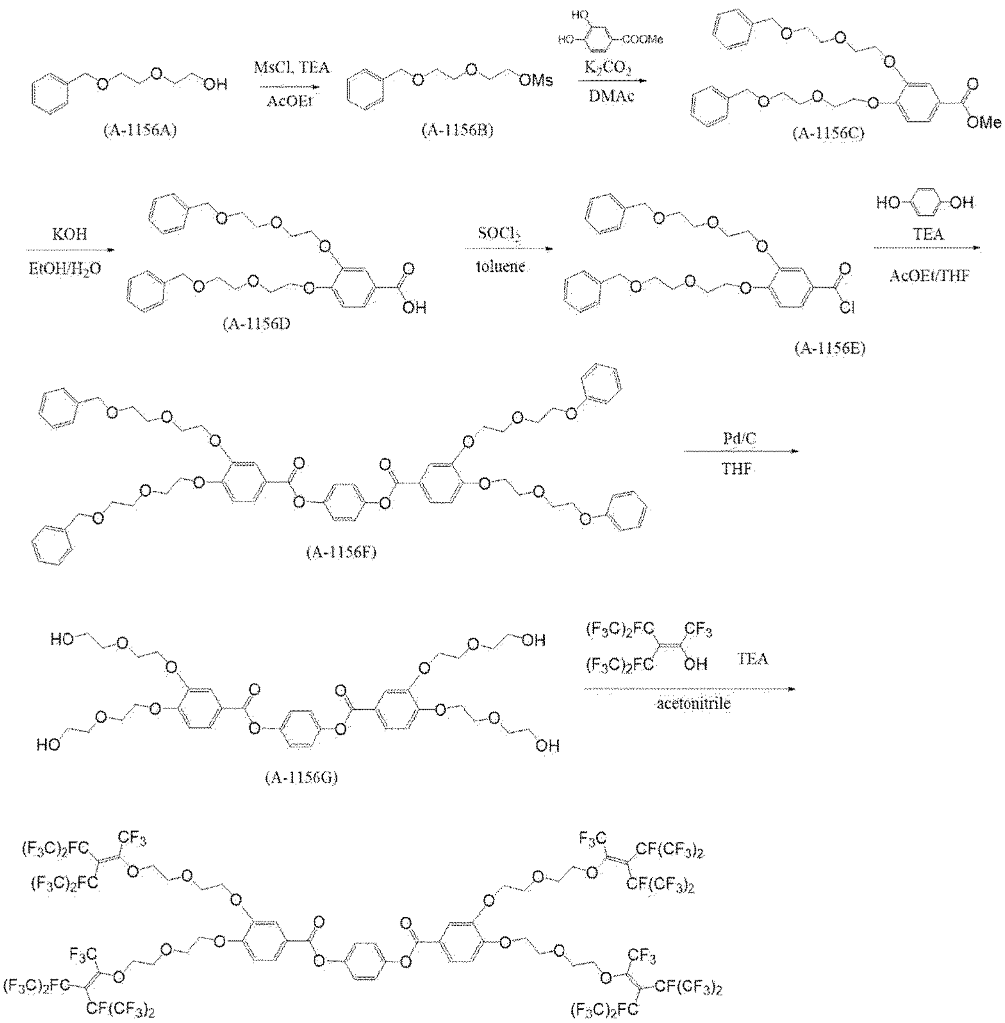


**(A-1156)**

<Synthesis of compound (A-1156B)>

20.0 g (0.1 mol) of (A-1156A) and 24 mL of ethyl acetate were placed in a 200 mL three-neck flask, and cooling was carried out to an internal temperature of 10°C by ice cooling. 14.0 g (0.12 mol) of methanesulfonic acid chloride and 11.3 g (0.11 mol) of triethylamine were added dropwise thereto. After completion of the dropwise addition, the internal temperature was raised to 30°C and stirring was carried out for 1 hour. 24 mL of ethyl acetate and 30 mL of water were added, and stirring was carried out for 5 minutes. The aqueous layer was removed, and 10 mL of 5% sodium bicarbonate solution and 24 mL of a 10% saline solution were added to the organic layer, followed by stirring. The aqueous layer was removed, the organic layer was dried with anhydrous magnesium sulfate, the desiccant was removed by filtration, and the filtrate was concentrated under reduced pressure. 25.8 g (yield: 92%) of a colorless oily compound (A-1156B) was obtained.

<Synthesis of compound (A-1156C)>

Under a nitrogen stream, 3.0 g (18 mmol) of methyl 3,4-dihydroxybenzoate and 74 mL of N-dimethylacetamide were placed in a 300 mL three-neck flask. The internal temperature was raised to 90°C, and 10.1 g (37 mmol) of the compound (A-1156B) was added dropwise. After completion of the dropwise addition, the reaction solution was stirred at an internal temperature of 90°C for 2 hours and cooled to room temperature. 98 mL of ethyl acetate, 49 mL of 1 N hydrochloric acid water, and 5 mL of concentrated hydrochloric acid were added dropwise thereto, and stirring was carried. The aqueous layer was removed, and the organic layer was washed with 49 mL of a 10% saline solution. The organic layer was dried with anhydrous magnesium sulfate, the desiccant was removed by filtration, and the filtrate was concentrated under reduced pressure. 8.9 g (yield: 94%) of a yellow oily compound (A-1156C) was obtained.

<Synthesis of compound (A-1156D)>

8.8 g (16 mmol) of the compound (A-1156C), 63 mL of ethanol, 3.8 g (67 mmol) of potassium hydroxide, and 23 mL of water were placed in a 300 mL three-neck flask, and stirring was carried out at an internal temperature of 70°C for 1 hour. The reaction solution was cooled to room temperature, and 106 mL of ethyl acetate, 6.9 mL of concentrated hydrochloric acid, and 106 mL of water were added thereto, followed by stirring. The aqueous layer was removed, the organic layer was dried with anhydrous magnesium sulfate, the desiccant was removed by filtration, and then, the filtrate was concentrated under reduced pressure. 5.6 g (yield: 65%) of a brown oily compound (A-1156D) was obtained.

<Synthesis of compound (A-1156F)>

5.3 g (10 mmol) of the compound (A-1156D), 0.16 mL of N-dimethylformamide, and 30 mL of toluene were placed in a 200 mL three-neck flask, and the temperature was raised to an internal temperature of 75°C. 1.5 mL (21 mmol) of thionyl chloride was added dropwise thereto, and stirring was carried out at 75°C for 1 hour. Toluene was distilled off under reduced pressure to quantitatively obtain a compound (A-1156E). 52 mL of tetrahydrofuran, 52 mL of ethyl acetate, and 0.5 g (5 mmol) of hydroquinone were added thereto, and the internal temperature was decreased to 10°C. 1.4 g (14 mmol) of triethylamine was added dropwise, and the internal temperature after the dropwise addition was raised to 25°C. After stirring at 25°C for 1 hour, 5 mL of water was added, followed by stirring for 5 minutes. 50 mL of ethyl acetate and 60 mL of 1 N hydrochloric acid water were added thereto, and stirring was carried. The aqueous layer was removed, and the organic layer was washed with 50 mL of a 10% saline solution. The organic layer was dried with anhydrous magnesium sulfate, the desiccant was removed by filtration, and the filtrate was concentrated under reduced pressure. 3.6 g (yield: 60%) of a brown oily compound (A-1156F) was obtained.

<Synthesis of compound (A-1156G)>

3.1 g (2.8 mmol) of the compound (A-1156F), 31 mL of tetrahydrofuran, and 0.3 g (0.3 mmol) of 10% palladium carbon were placed in a 100 mL three-neck flask, and a hydrogen balloon was attached thereto. The reaction solution was stirred at an internal temperature of 30°C for 2 hours and filtered through celite. The filter material was washed with tetrahydrofuran, and the filtrate was concentrated under reduced pressure. 2.1 g (yield: 70%) of a brown oily compound (A-1156G) was obtained.

<Synthesis of compound (A-1156)>

1.0 g (1.0 mmol) of the compound (A-1156G), 10 mL of acetonitrile, 0.7 g (7 mmol) of triethylamine, and 3.2 g (7 mmol) of hexafluoropropene were placed in a 100 mL three-neck flask, and stirring was at 25°C for 3 hours. 10 mL of 1 N hydrochloric acid water and 30 mL of ethyl acetate were added thereto, and stirring was carried. The aqueous layer was removed, and the organic layer was washed with 30 mL of a 10% saline solution. The organic layer was dried with anhydrous magnesium sulfate, the desiccant was removed by filtration, and the filtrate was concentrated under reduced pressure. The crude product was purified by silica gel column chromatography to obtain 3.3 g (yield: 70%) of a colorless oily compound (A-1156).

The $^1$H-NMR result of the compound (A-1156) is shown below.

$^1$H-NMR (solvent: heavy acetone) δ (ppm): 7.86 (d, 2H), 7.74 (s, 2H), 7.36 (s, 4H), 7.19 (d, 2H), 4.48-4.45 (m, 2H), 4.32-4.17 (m, 14H), 3.99-3.90 (m, 16H)

[Synthesis of compound (A-1301)]

The synthesis was carried out in the same manner as in the compound (A-1102) except that hydroquinone as a raw material was changed to 4-hydroxybiphenyl and the using amounts of the compound (S-2-g-2) and the triethylamine were changed to be half, thereby obtaining 97 g (yield: 70%) of a colorless solid compound (A-1301).

The $^1$H-NMR result of the compound (A-1301) is shown below.

$^1$H-NMR (solvent: heavy acetone) δ (ppm): 7.70 (dd, 1H), 7.75 (d, 3H), 7.70 (d, 2H), 7.50 (t, 2H), 7.40-7.34 (m, 3H), 7.20 (d, 1H), 4.47-4.32 (m, 8H), 4.11 (t, 4H)

[Synthesis of compound (A-1302)]

The synthesis was carried out in the same manner as in the compound (A-1103) except that hydroquinone as a raw material was changed to 4-hydroxybiphenyl and the using amounts of the compound (S-2-g-1) and the triethylamine were changed to be half, thereby obtaining 97 g (yield: 70%) of a colorless liquid compound (A-1302).

The $^1H$-NMR result of the compound (A-1302) is shown below.

$^1H$-NMR (solvent: heavy acetone) $\delta$ (ppm): 7.77 (d, 2H), 7.70 (d, 2H), 7.49 (t, 2H), 7.41-7.36 (m, 5H), 6.88 (t, 1H), 4.44 (s, 4H), 4.41 (s, 4H), 4.32 (t, 4H), 4.10 (t, 4H)

[Synthesis of compound (A-1303)]

The synthesis was carried out in the same manner as in the compound (A-1104) except that hydroquinone as a raw material was changed to 4-hydroxybiphenyl and the using amounts of the compound (S-2-g-3) and the triethylamine were changed to be half, thereby obtaining 94 g (yield: 68%) of a colorless liquid compound (A-1303).

The $^1H$-NMR result of the compound (A-1303) is shown below.

$^1H$-NMR (solvent: heavy acetone) $\delta$ (ppm): 7.77 (d, 2H), 7.71 (d, 2H), 7.54 (s, 2H), 7.49 (t, 2H), 7.41-7.34 (m, 3H), 4.46-4.33 (m, 12H), 4.13 (t, 4H), 4.02 (t, 2H)

[Examples 1 to 29 and Comparative Example 1]

As shown in Table 1 below, the compound according to the embodiment of the present invention was used as a liquid crystal alignment accelerator to form an optically anisotropic film, which was subsequently subjected to evaluation.

First, a coating liquid having the following composition was prepared. It is noted that the concentration of the liquid crystal alignment accelerator was adjusted to be 0.02 parts by mass, 0.03 parts by mass, 0.10 parts by mass, and 0.20 parts by mass with respect to 100 parts by mass of the total of the rod-like liquid crystal compound, thereby preparing respective coating liquids.

| (Coating liquid of liquid crystal composition) | |
|---|---|
| · Rod-like liquid crystal compound M1 described below | 84 parts by mass |
| · Rod-like liquid crystal compound M2 described below | 14 parts by mass |
| · Rod-like liquid crystal compound M3 described below | 2 parts by mass |
| · Chiral agent (12) described below | 5.0 parts by mass |
| · IRGACURE 819 (manufactured by BASF SE) | 3 parts by mass |
| · Liquid crystal alignment accelerator described in Table 1 below | amount described above |
| · Methyl ethyl ketone (MEK) | amount at which solute concentration is 25% by mass |

Polymerizable rod-like liquid crystal compound M1

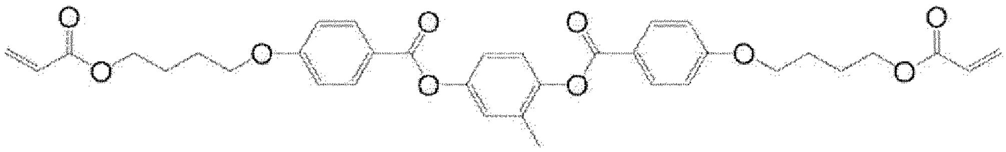

Polymerizable rod-like liquid crystal compound M2

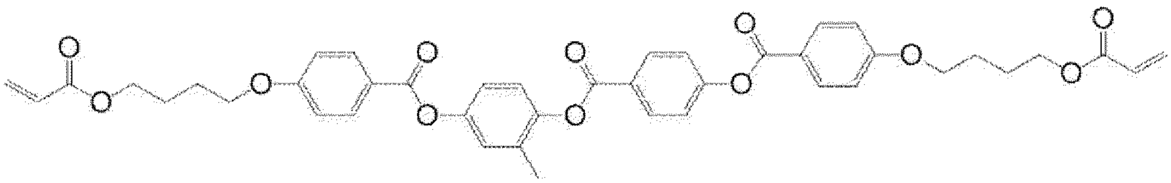

Polymerizable rod-like liquid crystal compound M3

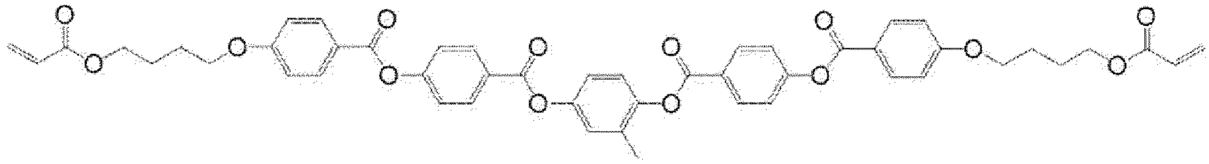

Chiral agent (12)

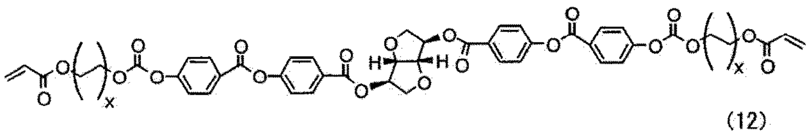

(12)

50 μl of the prepared coating liquid was weighed out using a micropipettor, dropped onto glass attached with an alignment film (SE-130), and subjected to spin coating.

Next, heating was carried out at 85°C for 2 minutes, cooling was carried out for 1 minute, and then irradiation with ultraviolet light was carried out in a nitrogen atmosphere (ultraviolet intensity: 300 $mJ/m^2$) to form an optically anisotropic film. The film thickness of the optically anisotropic film was about 4 μm.

In addition, the formed optically anisotropic film exhibited selective reflectivity of green color derived from a cholesteric liquid crystal phase. As a result of measuring the reflection wavelength with UVPC-3000 manufactured by Shimadzu Corporation, the central wavelength was 530 nm.

The aligning properties of each of the manufactured optically anisotropic films were evaluated visually and by haze. The haze was measured using a haze meter NDH2000 manufactured by NIPPON DENSHOKU INDUSTRIES Co., Ltd.

Further, a dissolution/alignment promotion test was carried out, and haze values were compared between the optically anisotropic films having concentrations of liquid crystal alignment accelerator of 0.02% by mass, 0.03% by mass, 0.10% by mass, and 0.20% by mass, respectively, and the dissolution/alignment promoting action was evaluated according to the following criteria. The results are shown in Table 1 below.

Here, it is indicated that in those having a high evaluation, the solubility is good and the alignment promoting action is also large. It is indicated that in those having a low evaluation, the solubility is low mainly.

A: Less than 0.15
B: 0.15 or more and less than 0.35
C: 0.35 or more and less than 0.90
D: 0.90 or more

[Table 1]

| | Liquid crystal alignment accelerator | Solvent | Dissolution/alignment promotion test | | | | |
|---|---|---|---|---|---|---|---|
| | | | Concentration 0.02% by mass | Concentration 0.03% by mass | Concentration 0.10% by mass | Concentration 0.20% by mass | Concentration 0.30% by mass |
| Comparative Example 1 | Compound (Y) | MEK | C | A | C | D | D |
| Example 1 | A-101 | MEK | B | A | A | A | B |
| Example 2 | A-410 | MEK | A | A | A | B | C |
| Example 3 | A-501 | MEK | A | A | A | B | C |
| Example 4 | A-1102 | MEK | A | A | A | A | B |
| Example 5 | A-1119 | MEK | A | A | A | A | B |
| Example 6 | A-1139 | MEK | A | A | A | A | B |
| Example 7 | A-1132 | MEK | A | A | A | A | A |
| Example 8 | A-1145 | MEK | A | A | A | A | A |
| Example 9 | A-1109 | MEK | A | A | A | A | B |
| Example 10 | A-1104 | MEK | B | A | A | A | A |
| Example 11 | A-1156 | MEK | B | A | A | A | A |
| Example 12 | A-1135 | MEK | B | A | A | A | B |
| Example 13 | A-1103 | MEK | A | A | A | B | C |
| Example 14 | A-1142 | MEK | B | A | A | A | B |
| Example 15 | A-1146 | MEK | A | A | B | C | C |
| Example 16 | A-1153 | MEK | B | A | A | B | C |
| Example 17 | A-1101 | MEK | B | A | A | A | B |
| Example 18 | A-1106 | MEK | B | A | A | A | A |
| Example 19 | A-1107 | MEK | A | A | A | B | B |
| Example 20 | A-1108 | MEK | B | A | A | A | B |
| Example 21 | A-1003 | MEK | A | A | A | A | B |
| Example 22 | A-1017 | MEK | A | A | A | A | A |

(continued)

| | Liquid crystal alignment accelerator | Solvent | Dissolution/alignment promotion test | | | | |
|---|---|---|---|---|---|---|---|
| | | | Concentration 0.02% by mass | Concentration 0.03% by mass | Concentration 0.10% by mass | Concentration 0.20% by mass | Concentration 0.30% by mass |
| Example 23 | A-1115 | MEK | A | A | A | A | B |
| Example 24 | A-1117 | MEK | A | A | A | B | C |
| Example 25 | A-1121 | MEK | A | A | A | B | C |
| Example 26 | A-1131 | MEK | A | A | A | A | B |
| Example 27 | A-1133 | MEK | A | A | A | B | C |
| Example 28 | A-1137 | MEK | A | A | A | B | C |
| Example 29 | A-1144 | MEK | A | A | A | B | C |

The structures of the liquid crystal alignment accelerators in Table 1 are shown below.

Compound (Y) [compound (30) of JP2002-129162A]

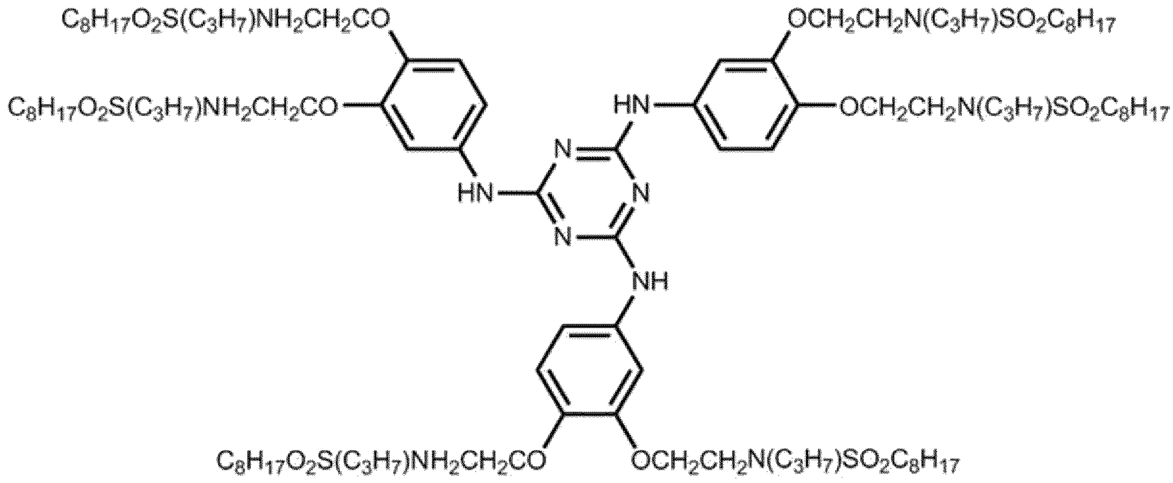

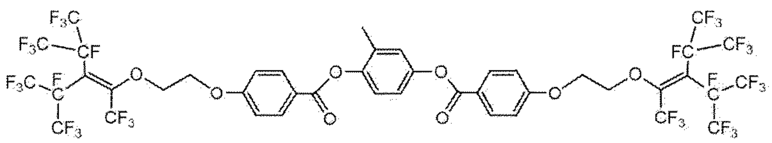

A-101

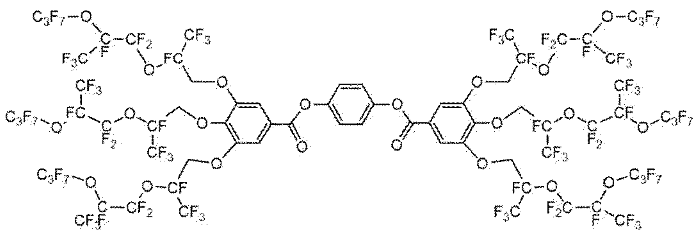

A-410

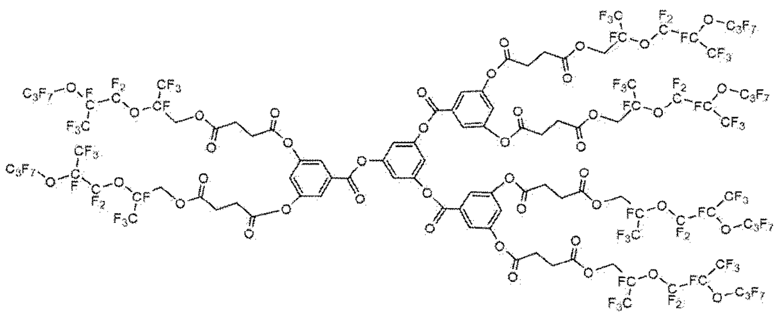

A-501

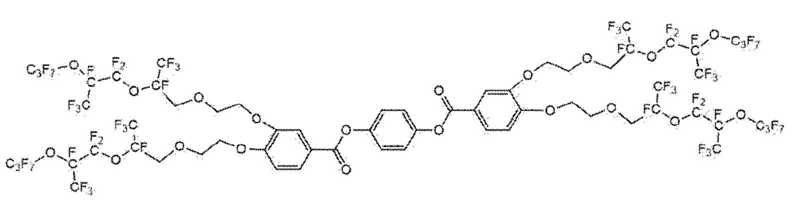

A-1102

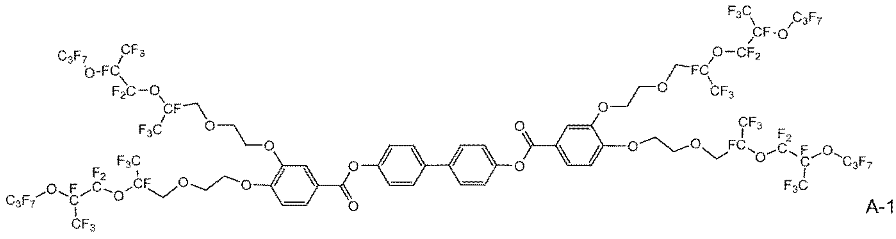

A-1119

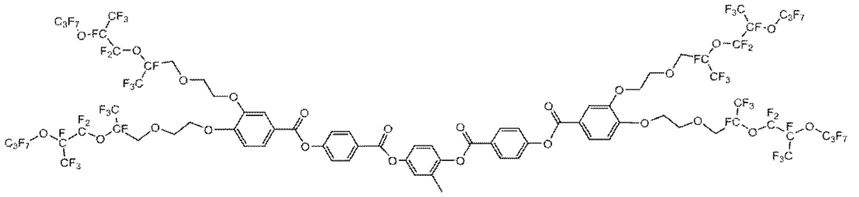

A-1139

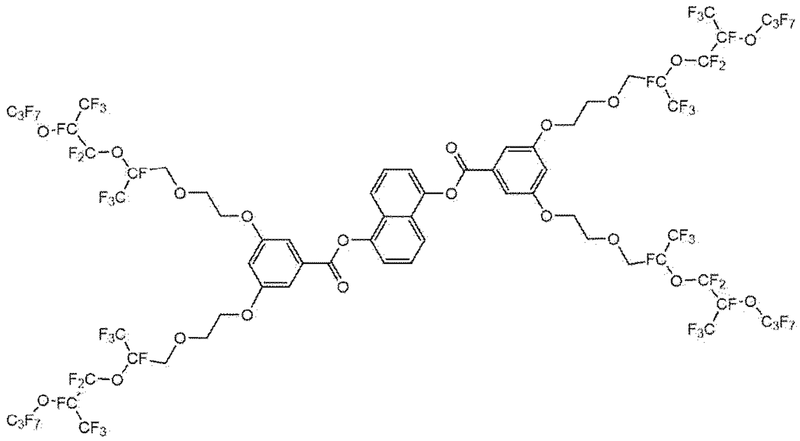

A-1132

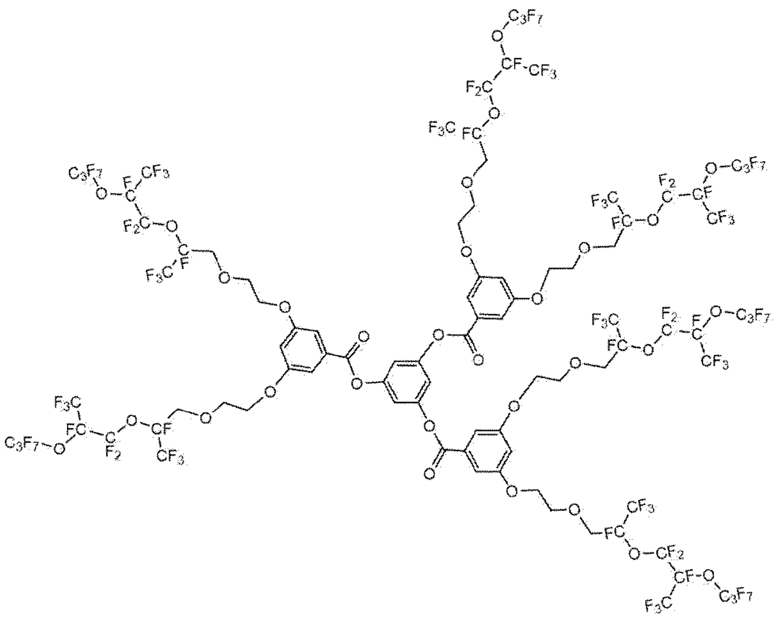

A-1145

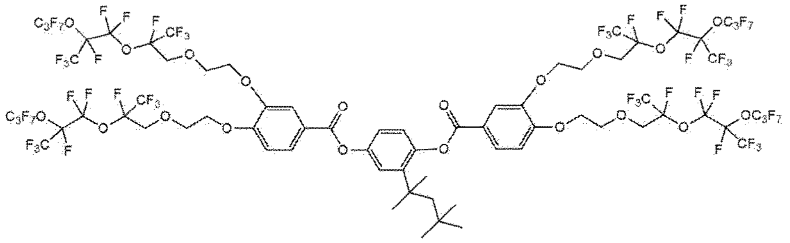

A-1109

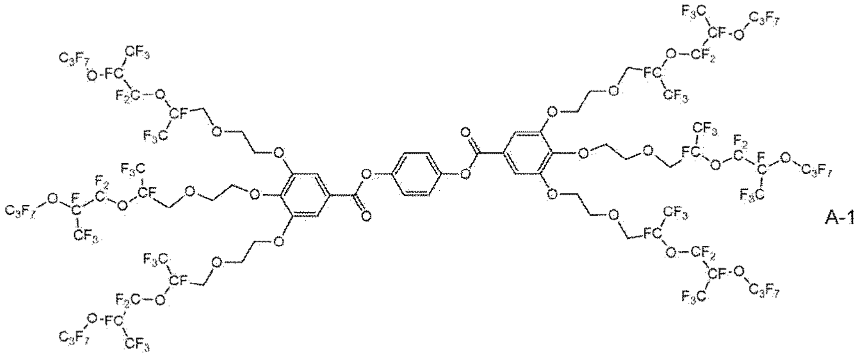

A-1104

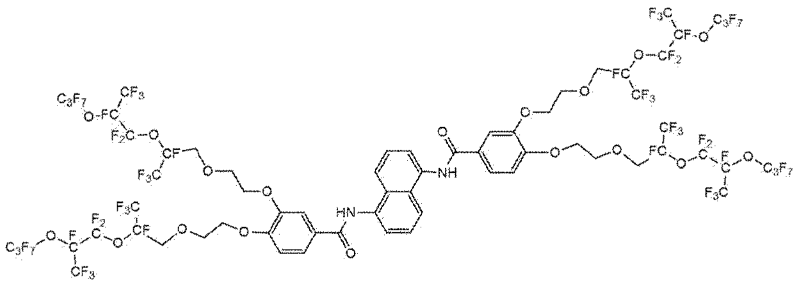

A-1135

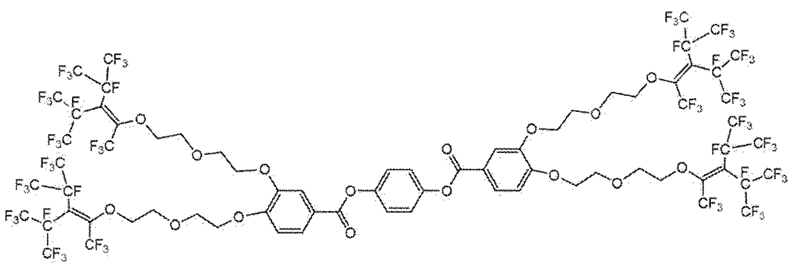

A-1156

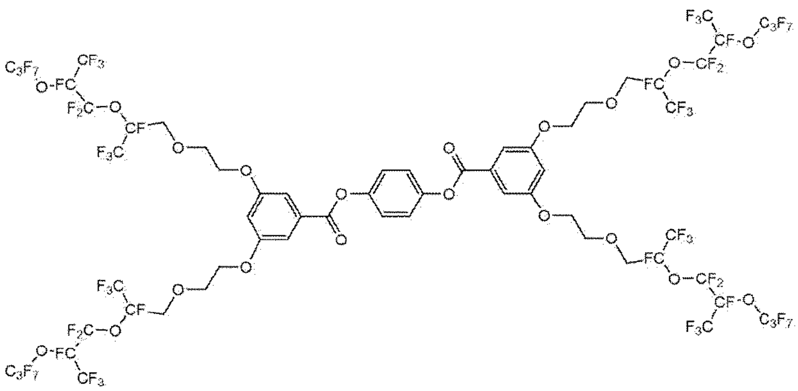

A-1103

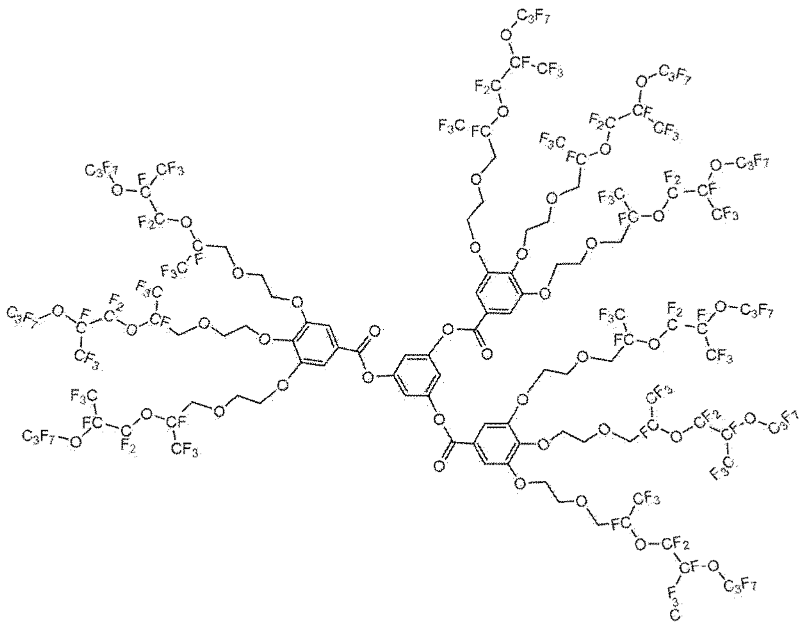

A-1146

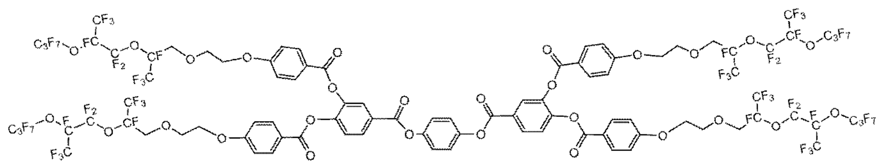

A-1142

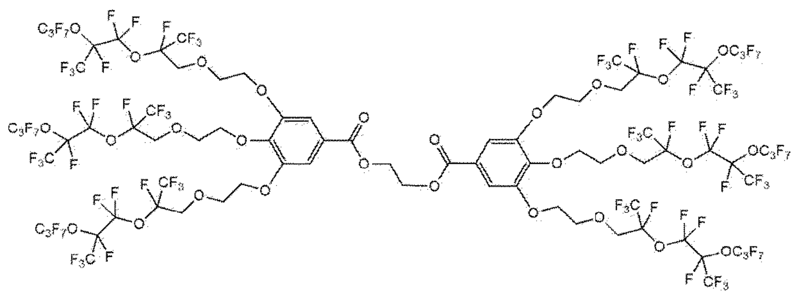

**A-1153**

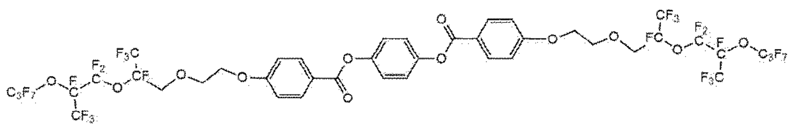

A-1101

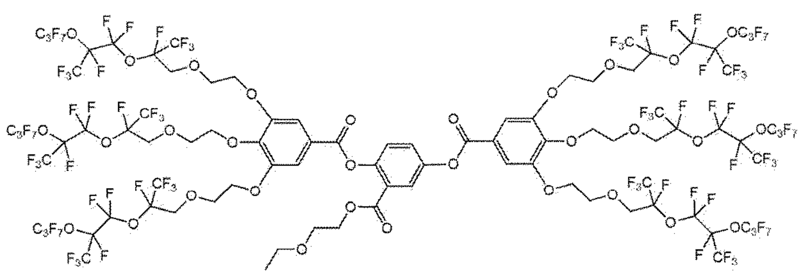

A-1106

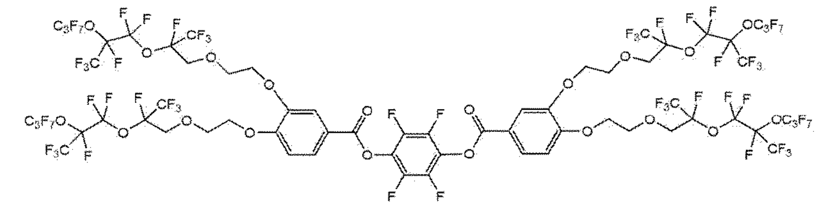

A-1107

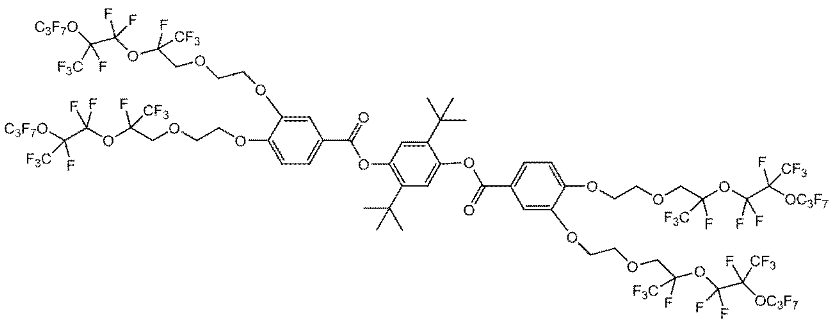

A-1108

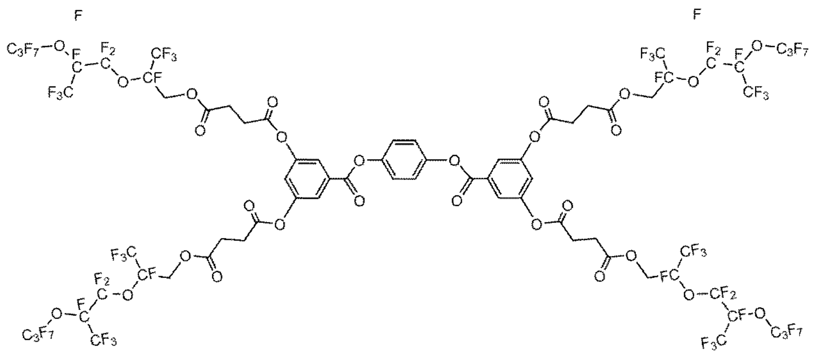

A-1003

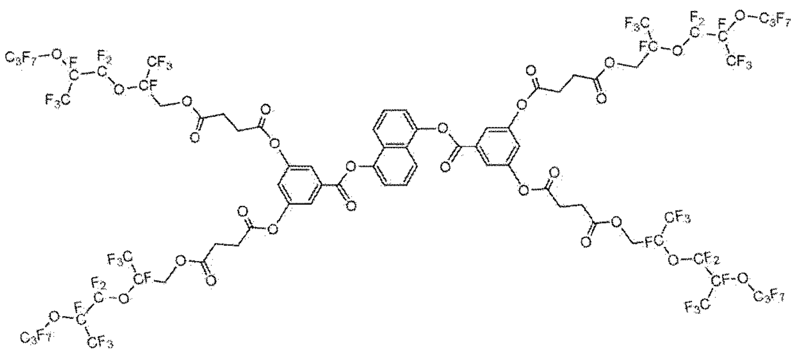

A-1017

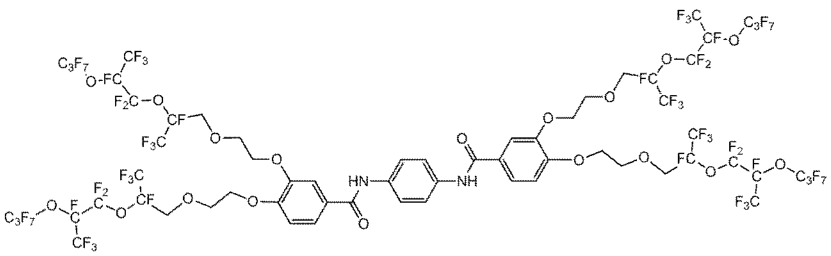

A-1115

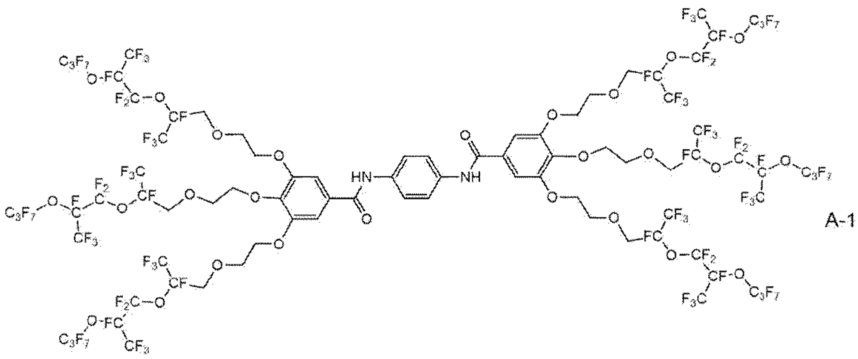

A-1117

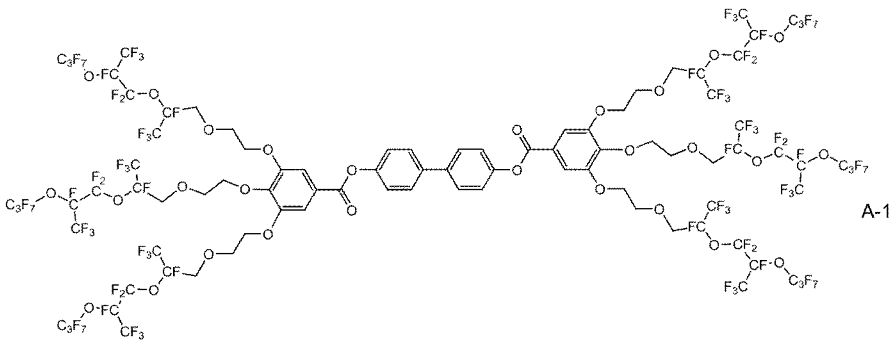

A-1121

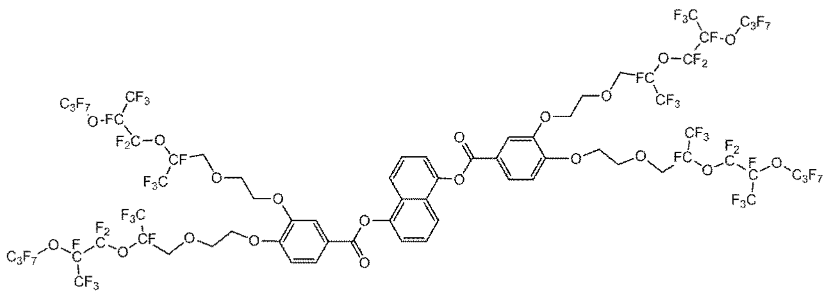

A-1131

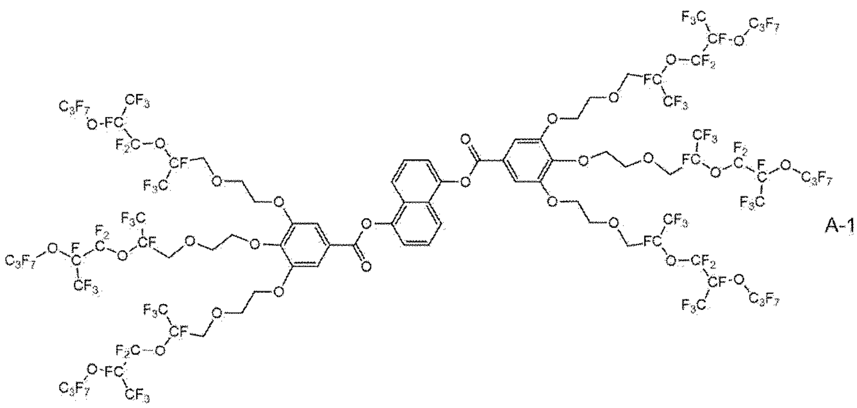

A-1133

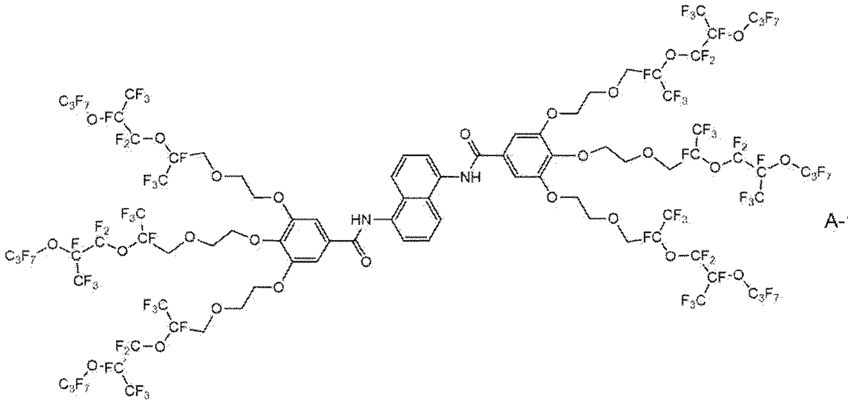

A-1137

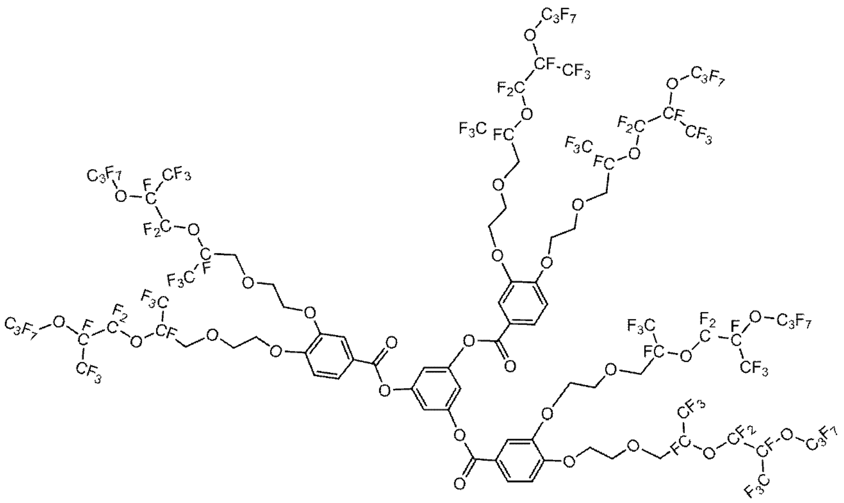

A-1144

As shown in Table 1 above, it has been confirmed that the compound according to the embodiment of the present invention has a large liquid crystal alignment action and has a high solubility in a solvent even at a high concentration. In addition, it has been confirmed that the compound according to the embodiment of the present invention has a wide range of application for a coating solvent and has high use suitability.

[Examples 30 to 32]

As shown in Table 2 below, the compound according to the embodiment of the present invention was used as a liquid crystal alignment accelerator to form an optically anisotropic film, which was subsequently subjected to evaluation.

First, a coating liquid having the following composition was prepared. It is noted that the concentration of the liquid crystal alignment accelerator was adjusted to be 0.02 parts by mass, 0.03 parts by mass, 0.10 parts by mass, 0.20 parts by mass, and 0.30 parts by mass with respect to 100 parts by mass of the total of the disk-like liquid crystal compound, thereby preparing respective coating liquids.

| (Coating liquid of liquid crystal composition) | |
|---|---|
| · Disk-like liquid crystal compound M4 described below | 80 parts by mass |
| · Disk-like liquid crystal compound M5 described below | 20 parts by mass |
| · Alignment film interface aligning agent 1 described below | 0.55 parts by mass |
| · IRGACURE 819 (manufactured by BASF SE) | 3 parts by mass |
| · Liquid crystal alignment accelerator described in Table 2 below | amount described above |
| · Methyl ethyl ketone (MEK) | amount at which solute concentration is 33% by mass |

Polymerizable disk-like liquid crystal compound M4

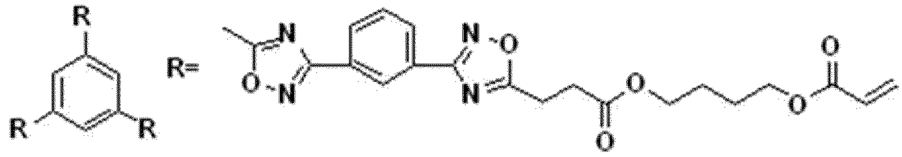

Polymerizable disk-like liquid crystal compound M5

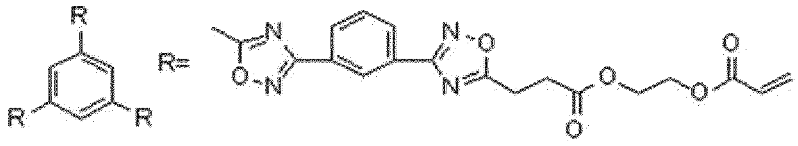

Alignment film interface aligning agent 1

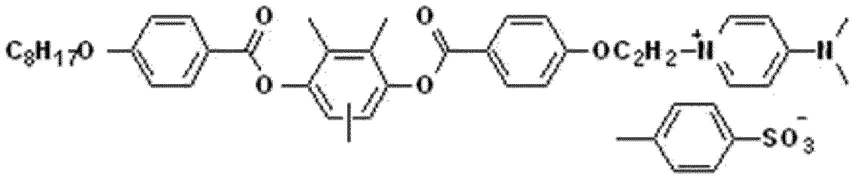

50 μl of the prepared coating liquid was weighed out using a micropipettor, dropped onto glass attached with an alignment film (PVA-103), and subjected to spin coating. Heating was carried out at 110°C for 2 minutes, cooling was carried out for 1 minute, and then irradiating with ultraviolet light (ultraviolet intensity: 500 $mJ/cm^2$) was carried out in a nitrogen atmosphere to obtain an optically anisotropic film. The film thickness of the optically anisotropic film was about 1.1 μm.
The aligning properties of each of the manufactured optically anisotropic films were evaluated visually and by haze. The haze was measured using a haze meter NDH2000 manufactured by NIPPON DENSHOKU INDUSTRIES Co., Ltd.
Further, a dissolution/alignment promotion test was carried out, and haze values were compared between the optically anisotropic films having concentrations of liquid crystal alignment accelerator of 0.10% by mass, 0.20% by mass, 0.30% by mass, and 0.50% by mass, respectively, and the dissolution/alignment promoting action was evaluated according to the following criteria. The results are shown in Table 2 below.
Here, it is indicated that in those having a high evaluation, the solubility is good and the alignment promoting action is also large. It is indicated that in those having a low evaluation, the solubility is low mainly.

A: Less than 0.15

B: 0.15 or more and less than 0.35

C: 0.35 or more and less than 0.90

D: 0.90 or more

[Table 2]

| | Liquid crystal alignment accelerator | Solvent | Dissolution/alignment promotion test | | | |
|---|---|---|---|---|---|---|
| | | | Concentration 0.10% by mass | Concentration 0.20% by mass | Concentration 0.30% by mass | Concentration 0.50% by mass |
| Example 30 | A-1301 | MEK | B | A | A | B |
| Example 31 | A-1302 | MEK | B | A | A | B |
| Example 32 | A-1303 | MEK | A | A | B | B |

The structures of the liquid crystal alignment accelerators in Table 2 are shown below.

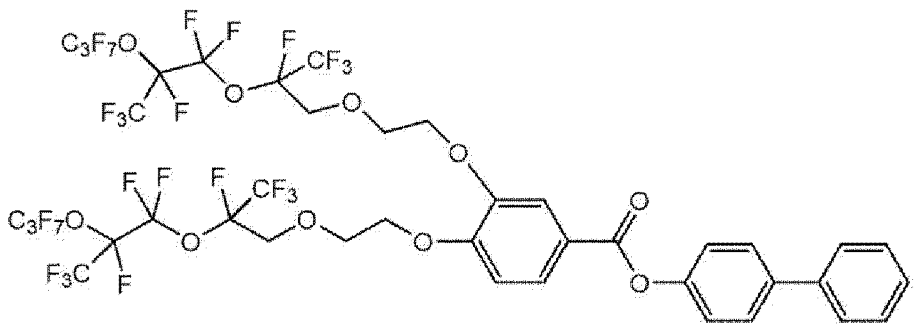

**A-1301**

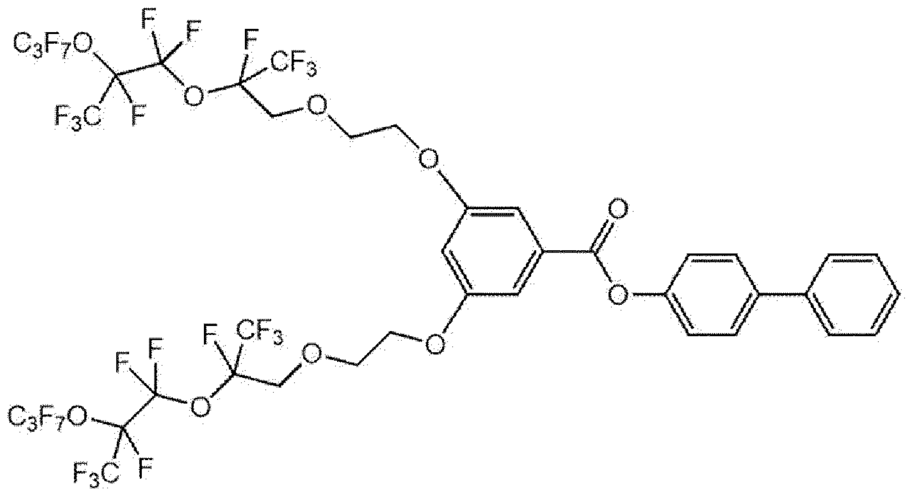

**A-1302**

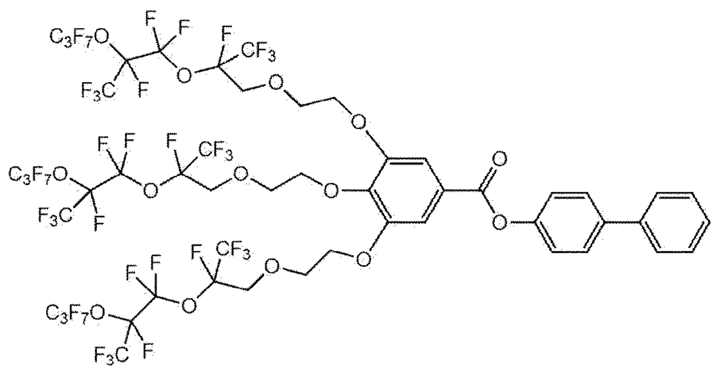

**A-1303**

As shown in Table 2 above, even in a case where a disk-like liquid crystal compound is used as the polymerizable liquid crystal compound contained in the liquid crystal composition, it has been confirmed that the compound according to the embodiment of the present invention has a large liquid crystal alignment action and has a high solubility in a solvent even at a high concentration. As a result, it has been confirmed that the compound according to the embodiment of the present invention has a wide range of application for a coating solvent and has high use suitability.

## Claims

**1.** A compound comprising, in a molecule:

a structure A represented by the following A; and
a structure B represented by the following B,
wherein the compound includes two or more of the structures B in the molecule,

A: a structure in which at least one or more aromatic ring structures are included and a total number of $\pi$ electrons of the aromatic ring structure in the molecule is 8 or more, provided that aryl amine structures represented by Formulae (I) to (IV) are excluded,
B: a fluorine-containing terminal structure represented by any one of Formulae (B-1), ..., or (B-5),

(I) 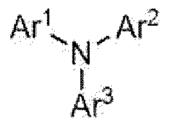

(II) 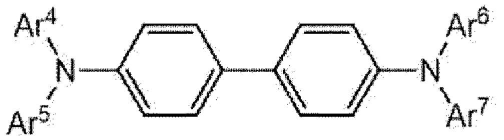

(III) 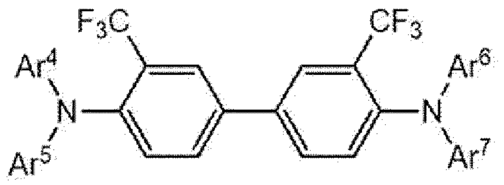

(IV) 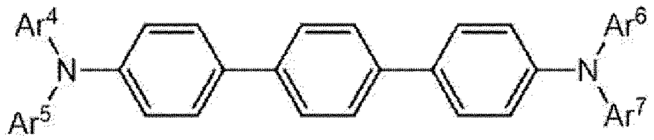

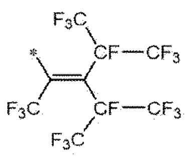
(B-1)

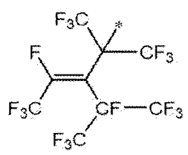
(B-2)

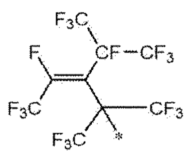
(B-3)

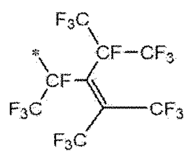
(B-4)

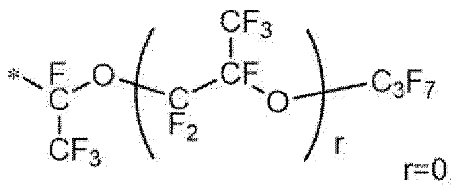

r=0,1,2

(B-5)

here, in Formulae (I) to (IV), $Ar^1$, $Ar^2$, $Ar^3$, $Ar^4$, $Ar^5$, $Ar^6$, and $Ar^7$ each independently represent a substituted or unsubstituted aryl group having 6 to 20 carbon atoms, and
in Formulae (B-1) to (B-5), * represents a bonding position.

**2.** The compound according to claim 1,
wherein the structure A is a structure represented by Formula (1) or (2),

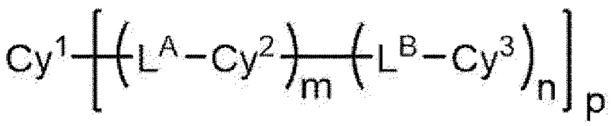
(1)

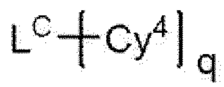
(2)

here, in Formula (1),

m represents 0 or 1, n represents 1 or 2, and p represents an integer of 0 to 3,
in a case where p is 2 or 3, a plurality of $L^A$'s, $Cy^2$'s, m's, $L^B$'s, $Cy^3$'s, and n's may be respectively the same or different from each other,
in a case where n is 2, a plurality of $L^B$'s and $Cy^3$'s may be respectively the same or different from each other,
$L^A$ and $L^B$ each independently represent a single bond or a divalent linking group, and
$Cy^1$, $Cy^2$, and $Cy^3$ each independently represent a ring structure represented by any one of Formulae (CY-1), ..., or (CY-23), provided that at least one of $Cy^1$, $Cy^2$, or $Cy^3$ has a ring structure having aromaticity, and among $Cy^1$, $Cy^2$, and $Cy^3$, a total number of $\pi$ electrons of the ring structure exhibiting aromaticity is 8 or more, and

in Formula (2),

q represents 3 or 4, where a plurality of $Cy^4$'s may be the same or different from each other,
$L^C$ represents a q-valent linking group, and
$Cy^4$ represents a ring structure represented by any one of Formulae (CY-1), ..., or (CY-23), provided that at least one of a plurality of $Cy^4$'s has a ring structure having aromaticity, and among the plurality of $Cy^4$'s, a total number of $\pi$ electrons of the ring structure exhibiting aromaticity is 8 or more,

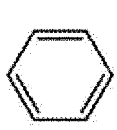
(CY-1)

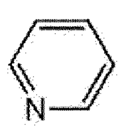
(CY-2)

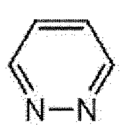
(CY-3)

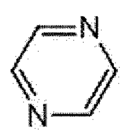
(CY-4)

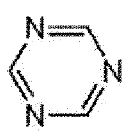
(CY-5)

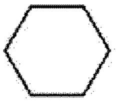
(CY-6)
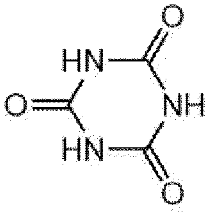
(CY-7)
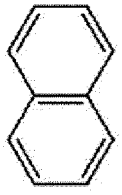
(CY-8)
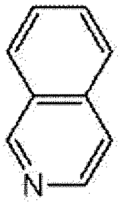
(CY-9)
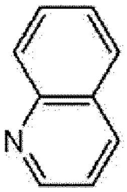
(CY-10)

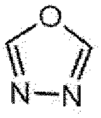
(CY-11)
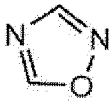
(CY-12)
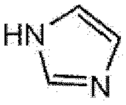
(CY-13)
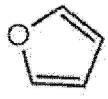
(CY-14)
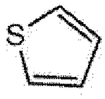
(CY-15)
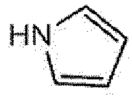
(CY-16)

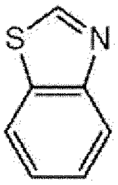
(CY-17)
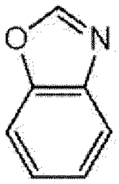
(CY-18)
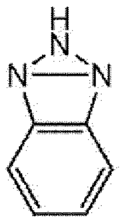
(CY-19)
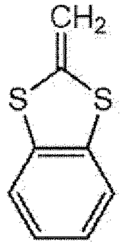
(CY-20)

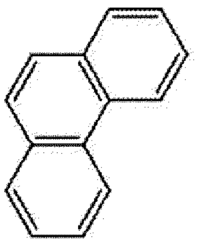
(CY-21)
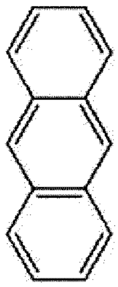
(CY-22)
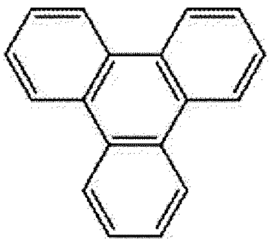
(CY-23)

here, a hydrogen atom in Formulae (CY-1) to (CY-23) may be substituted with a substituent or a group including the structure B.

**3.** The compound according to claim 1 or 2,
wherein the structure A is a structure represented by any one of Formulae (1-1), ..., or (1-10),

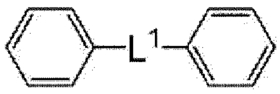
(1-1)

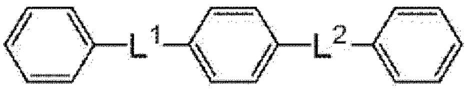
(1-2)
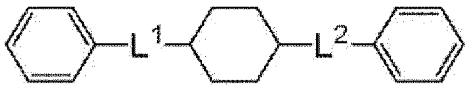
(1-3)

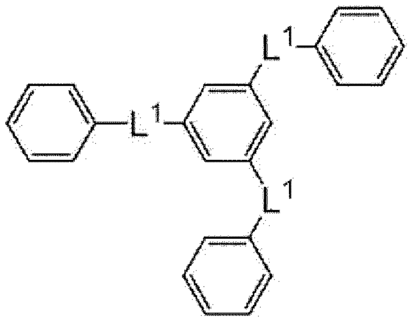

(1-4)

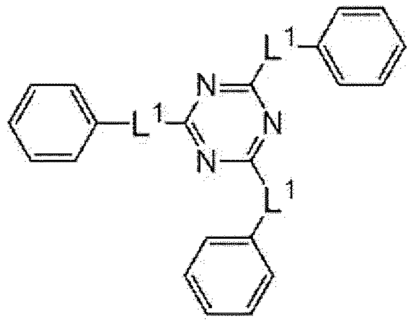

(1-5)

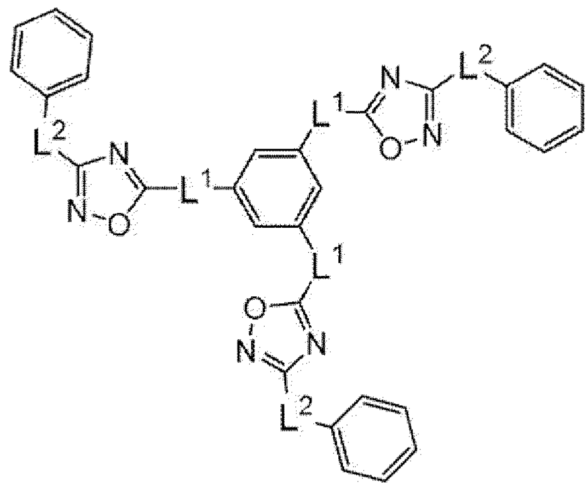

(1-6)

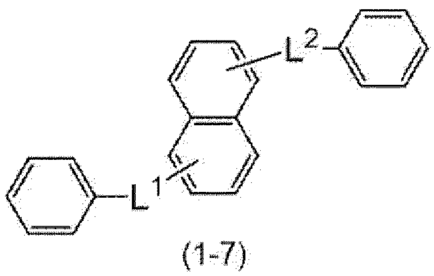

(1-7)

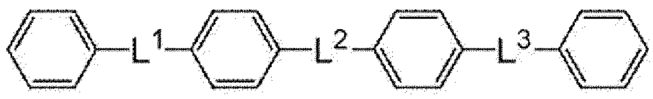

(1-8)

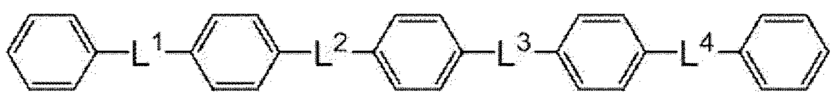

(1-9)

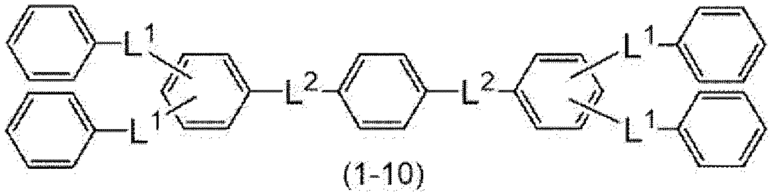

(1-10)

in Formulae (1-1) to (1-10),

a hydrogen atom possessed by a carbon atom constituting a ring structure may be substituted with a substituent

or a group including the structure B, and
$L^1$, $L^2$, $L^3$, and $L^4$ each independently represent a single bond or a divalent linking group.

**4.** The compound according to claim 3,
wherein in Formulae (1-1) to (1-10), $L^1$, $L^2$, $L^3$, and $L^4$ are each independently a single bond or a divalent linking group of any one of -C(=O)O-, -OC(=O)-, -O-, -NH-C(=O)-, or -NH-.

**5.** The compound according to claim 1 or 2,
wherein the structure A is a structure represented by Formula (2-1) or (2-2),

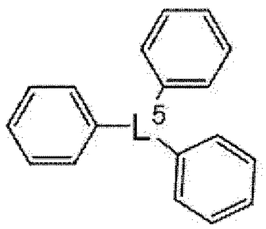

(2-1)

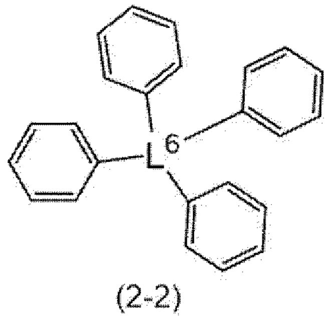

(2-2)

in Formulae (2-1) to (2 to 2),

a hydrogen atom possessed by a carbon atom constituting a ring structure may be substituted with a substituent or a group including the structure B,
$L^5$ represents a trivalent linking group, and
$L^6$ represents a tetravalent linking group.

**6.** The compound according to any one of claims 1 to 5,
wherein the compound is used in a liquid crystal alignment accelerator.

**7.** A liquid crystal composition comprising:

the compound according to any one of claims 1 to 6; and
a polymerizable liquid crystal compound.

**8.** The liquid crystal composition according to claim 7,
wherein the polymerizable liquid crystal compound is a rod-like liquid crystal compound or a disk-like liquid crystal compound.

**9.** The liquid crystal composition according to claim 7 or 8, further comprising at least one chiral compound.

**10.** A cured substance obtained by polymerizing the liquid crystal composition according to any one of claims 7 to 9.

**11.** A film comprising at least one kind of the cured substance according to claim 10.

**12.** A film obtained by immobilizing a cholesteric liquid crystalline phase of the liquid crystal composition according to any one of claims 7 to 9.

**13.** The film according to claim 11 or 12,
wherein the film exhibits optical anisotropy.

**14.** The film according to any one of claims 11 to 13,
wherein the film exhibits selective reflection characteristics.

**15.** The film according to claim 14,
wherein the film exhibits selective reflection characteristics in an infrared wavelength range.

**16.** The film according to claim 14,
wherein the film exhibits selective reflection characteristics in a visible light wavelength range.

## INTERNATIONAL SEARCH REPORT

International application No.
**PCT/JP2021/015240**

**A. CLASSIFICATION OF SUBJECT MATTER**

***C09K 19/12***(2006.01)i; ***C09K 19/14***(2006.01)i; ***C09K 19/16***(2006.01)i; ***C09K 19/18***(2006.01)i; ***C09K 19/20***(2006.01)i; ***C09K 19/22***(2006.01)i; ***C09K 19/24***(2006.01)i; ***C09K 19/28***(2006.01)i; ***C09K 19/30***(2006.01)i; ***C09K 19/32***(2006.01)i; ***C09K 19/34***(2006.01)i; ***C09K 19/38***(2006.01)i; ***C09K 19/42***(2006.01)i; ***C09K 19/54***(2006.01)i; ***G02B 5/26***(2006.01)i; ***G02B 5/30***(2006.01)i; ***C07C 69/92***(2006.01)i
FI: C07C69/92 CSP; C09K19/38; C09K19/42; C09K19/34; C09K19/32; C09K19/30; C09K19/24; C09K19/28; C09K19/22; C09K19/20; C09K19/18; C09K19/16; C09K19/14; C09K19/12; C09K19/54 B; G02B5/30; G02B5/26

According to International Patent Classification (IPC) or to both national classification and IPC

**B. FIELDS SEARCHED**

Minimum documentation searched (classification system followed by classification symbols)

C09K19/12; C09K19/14; C09K19/16; C09K19/18; C09K19/20; C09K19/22; C09K19/24; C09K19/28; C09K19/30; C09K19/32; C09K19/34; C09K19/38; C09K19/42; C09K19/54; G02B5/26; G02B5/30; C07C69/92

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | JP 2020-500992 A (MERCK PATENT GMBH) 16 January 2020 (2020-01-16) claims, paragraphs [0155]-[0176], [0198], [0230]-[0233] | 1-16 |
| X | WO 2019/233978 A1 (MERCK PATENT GMBH) 12 December 2019 (2019-12-12) claims, pages 90-110, 120, 131 | 1-16 |
| X | JP 4-325566 A (NEOS CO., LTD.) 13 November 1992 (1992-11-13) example 6 | 1-6 |
| A | | 7-16 |
| X | JP 60-139751 A (TORAY INDUSTRIES, INC.) 24 July 1985 (1985-07-24) 2nd table | 1-6 |
| A | | 7-16 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **10 June 2021** | **06 July 2021** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2021/015240**

**C. DOCUMENTS CONSIDERED TO BE RELEVANT**

| Category* | Citation of document, with indication, where appropriate, of the relevant passages | Relevant to claim No. |
|---|---|---|
| X<br><br>A | JP 2006-117928 A (FUJI PHOTO FILM CO., LTD.) 11 May 2006 (2006-05-11)<br>paragraph [0078] | 1-6<br><br>7-16 |
| X<br><br>A | JP 7-118554 A (MITSUBISHI CHEM CORP.) 09 May 1995 (1995-05-09)<br>table 1 | 1-6<br><br>7-16 |
| A | JP 2012-211306 A (FUJIFILM CORP.) 01 November 2012 (2012-11-01)<br>entire text, all drawings | 1-16 |
| A | JP 2002-129162 A (FUJI PHOTO FILM CO., LTD.) 09 May 2002 (2002-05-09)<br>entire text, all drawings | 1-16 |
| A | JP 2013-047204 A (FUJIFILM CORP.) 07 March 2013 (2013-03-07)<br>entire text, all drawings | 1-16 |

Form PCT/ISA/210 (second sheet) (January 2015)

**INTERNATIONAL SEARCH REPORT**
**Information on patent family members**

International application No.
**PCT/JP2021/015240**

| Patent document cited in search report | Publication date (day/month/year) | Patent family member(s) | Publication date (day/month/year) |
|---|---|---|---|
| JP 2020-500992 A | 16 January 2020 | US 2019/0300791 A1<br>claims, paragraphs [0230], [0272], [0297]-[0302]<br>WO 2018/104285 A2<br>EP 3551723 A2<br>CN 110062801 A<br>KR 10-2019-0092463 A | |
| WO 2019/233978 A1 | 12 December 2019 | (Family: none) | |
| JP 4-325566 A | 13 November 1992 | (Family: none) | |
| JP 60-139751 A | 24 July 1985 | (Family: none) | |
| JP 2006-117928 A | 11 May 2006 | US 2006/0068998 A1<br>page 24 | |
| JP 7-118554 A | 09 May 1995 | (Family: none) | |
| JP 2012-211306 A | 01 November 2012 | US 2014/0014877 A1<br>entire text, all drawings<br>WO 2012/128306 A1<br>EP 2690155 A1<br>CN 103443246 A | |
| JP 2002-129162 A | 09 May 2002 | US 2002/0039627 A1<br>entire text, all drawings<br>EP 1170353 A2 | |
| JP 2013-047204 A | 07 March 2013 | US 2014/0138580 A1<br>entire text, all drawings<br>WO 2013/015077 A1<br>EP 2738155 A1<br>CN 103702971 A<br>KR 10-2014-0049022 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2002129162 A
- JP 2013047204 A
- JP 2007108732 A
- JP 2010244038 A
- US 4683327 A
- US 5622648 A
- US 5770107 A
- WO 9522586 A
- WO 9524455 A
- WO 9700600 A
- WO 9823580 A
- WO 9852905 A
- JP 1989272551 A
- JP H1272551 A
- JP 6016616 A
- JP H616616 A
- JP 7110469 A
- JP H7110469 A
- JP 11080081 A
- JP H1180081 A
- JP 1999513019 A
- JP H11513019 A
- JP 2001328973 A
- JP 2005289980 A
- JP 2014198815 A
- JP 2014198814 A
- JP 2002080478 A
- JP 2002080851 A
- JP 2002179668 A
- JP 2002179669 A
- JP 2002179670 A
- JP 2002179681 A
- JP 2002179682 A
- JP 2002338575 A
- JP 2002338668 A
- JP 2003313189 A
- JP 2003313292 A
- US 2367661 A
- US 2367670 A
- US 2448828 A
- US 2722512 A
- US 3046127 A
- US 2951758 A
- US 3549367 A
- JP 60105667 A
- JP S60105667 A
- US 4239850 A
- US 4212970 A
- JP 63040799 B
- JP S6340799 B
- JP 5029234 B
- JP H529234 B
- JP 10095788 B
- JP H1095788 B
- JP 10029997 B
- JP H1029997 B
- JP 9152509 A
- JP H9152509 A
- EP 0911656 A2
- JP 7333433 A
- JP H7333433 A


### Non-patent literature cited in the description


- **IWANAMI SHOTEN.** *Polymer Physics-Phase Transition Dynamics, Masao Doi,* 1992, 2
- *Makromol. Chem.,* 1989, vol. 190, 2255
- *Advanced Materials,* 1993, vol. 5, 107
- Chiral Agent for TN and STN. Handbook of Liquid Crystal Device. Japan Society for the Promotion of Science, 1989, 199
- Easy Steps in Liquid Crystal Chemistry Experiment. Sigma Publishing, 2007, 46
- Liquid Crystal Handbook. Maruzen, 196